(19) Europäisches Patentamt
European Patent Office
Office européen des brevets

(11) EP 4 023 754 A1

(12) EUROPEAN PATENT APPLICATION
published in accordance with Art. 153(4) EPC

(43) Date of publication:
06.07.2022 Bulletin 2022/27

(21) Application number: 20857558.9

(22) Date of filing: 21.08.2020

(51) International Patent Classification (IPC):
*C12N 15/53* (2006.01) *C12Q 1/26* (2006.01)
*C12N 9/04* (2006.01)

(52) Cooperative Patent Classification (CPC):
C12Q 1/26

(86) International application number:
PCT/JP2020/031553

(87) International publication number:
WO 2021/039611 (04.03.2021 Gazette 2021/09)

(84) Designated Contracting States:
AL AT BE BG CH CY CZ DE DK EE ES FI FR GB GR HR HU IE IS IT LI LT LU LV MC MK MT NL NO PL PT RO RS SE SI SK SM TR
Designated Extension States:
BA ME
Designated Validation States:
KH MA MD TN

(30) Priority: 27.08.2019 JP 2019154965

(71) Applicant: Kikkoman Corporation
Noda-shi, Chiba 278-8601 (JP)

(72) Inventors:
- MARUSHIMA, Kazuya
SINGAPORE R&D LABORATORY PTE. LTD., 11 Biopolis Way, 05-01 Helios, Singapore 138667 (SG)
- CHEN, Yi Hsin
SINGAPORE R&D LABORATORY PTE. LTD., 11 Biopolis Way, 05-01 Helios, Singapore 138667 (SG)
- LIANTO, Dian Kartikasari
SINGAPORE R&D LABORATORY PTE. LTD., 11 Biopolis Way, 05-01 Helios, Singapore 138667 (SG)
- SATO, Takuya
SINGAPORE R&D LABORATORY PTE. LTD., 11 Biopolis Way, 05-01 Helios, Singapore 138667 (SG)
- ICHIYANAGI, Atsushi
Noda-shi, Chiba 278-8601 (JP)
- ARAKI, Yasuko
Noda-shi, Chiba 278-8601 (JP)

(74) Representative: Zacco Sweden AB
P.O. Box 5581
Löjtnantsgatan 21
114 85 Stockholm (SE)

# (54) METHOD FOR MEASURING PENTOSIDINE AND MEASUREMENT KIT

(57) Provided is a method for measuring pentosidine in a specimen, the measurement method comprising the steps of: degrading the specimen with an amino acid degrading enzyme; contacting the specimen after the degradation step with a protein having activity that oxidatively degrades pentosidine; and detecting change resulting from the contact, wherein the amino acid degrading enzyme and the protein having activity that oxidatively degrades pentosidine are different from each other.

EP 4 023 754 A1

SEQUENCE LISTING

<110> Kikkoman Corporation

<120> Method and kit for measuring pentosidine

<130> K0770ALP0004

<160> 23

<170> PatentIn version 3.5

<210> 1
<211> 2487
<212> DNA
<213> Sarocladium sp.

<400> 1
atgaagtctc ccagtctggc cgtggccggc cttcttcttg gctcgagttc cttaagccat 60

gctacccagc ttcgtattga gacgaggaaa tcgctcaact ctcgtatcgc caacgtccac 120

attgatgttg acgctccagt cgctcaccaa gttgtcttca catatggccc ttgtgattcg 180

gagagccaag agaacgctca ccatgtcatt gctcaaagcc aaaagctaga gggcaggaaa 240

ccccatcgat tgatctggac tatgcccaag gatcttcacc cggatgactg catctctgcg 300

tggggtgaat caggagacct cctcggtcgc agtgtccctc aaaaggtcgc gcacaaggag 360

atgaggagac gcaagagaga tgatagcgac tactccatcc ctatgaacag ctcgagtggc 420

atcgacgttt atggcccttg gttcgatggt gtagctctcc tcgagaagag cgacaatcac 480

aatgtggacg tcgaagccgc caaggccaag gagattgcta tcgtcggggc tgggatggct 540

ggtctgacca cgtacttcat cctcagtgaa gctggactca gcaacctgac gatcctggag 600

gcaagtggtc gtcttggagg ccgcgtacgc accgaatatc tctccggagg acccagggac 660

cattcctatg ccgagatggg ccctatgcgg atcccatacc aggctcgttt tggagacaag 720

gcatacaaca tctcggatca agcaatattc ttccgacttg tggagaaggt gaacgagcgg 780

aacaagaagc ttggaaacac caaggacctc atcaacctga tcccgttcat ccagtccagt 840

ccgaatgggc tcgcttatta tcaaggtaac aagctggaga atgggctgcc gccaacgcag 900

gcagatgttg ctgcagatcc agcactgggg aatgaatccc ctgagatccc tgaatctgca 960

caagaattgg ctgctcagct tcagagagca ttgcctaacg ctgaattcct tgagctaatg 1020

gccacaaact tctggcaagc ccatgccgaa ttcctcggta tgttgcttca gctcaccacc 1080

ccgtatagcc agggctctca aaccctcata gaggatgcca ctaaaatgaa atgcgaatgt 1140

agagaatcag ggtccagctg gcctgcctgg cgatcagtgg tctgagtttg ctttcctggt 1200

gaactacctc aacgcgaccg tcttcgatgc caacgcagtc actggtggtt atgactggca 1260

cagcagcctt gacagggtac gtgacgctga agaagatctc gtggttgcat cattaatccc 1320

acggtctccc tagttgtact acactatgct cttcggaggc gggggcagtt tcaagacgat 1380

tgatggaggc aagtcaaaat gagcgttgcg ggcacctgga cgagaactga cagagctcgc 1440

tcgtaggcat gaacttgctc cccaacgctt tccatcccct agttgacgat atcacgaagt 1500

tcaatgcaaa agtagagaag gttcagctcg atgagaagac ttcccgcctg aagctgcatt 1560

ggcgcgcgaa ctacactgat ccggagctcg agtcgcagtc atttgactac gccattctgt 1620

cacctaccat gccagcagta cagaagttgc gccttccagg taggtcctgc gcaatctctc 1680

gttgctttgc ggaccatagc tttcaatgtc acaggtctac ccttcgctat gcgcaacgcc 1740

gtcgactcca tgccttacgc ctctgcgtgc aaagtcgccc tcgaataccg cactcggttc 1800

tgggaaaagt tcgacaaccc catctacggc tcctgttcca ccagcaccga cattcccggt 1860

attggatccg tgtgctaccc atcttccaac atcaacggca gcggcccagc ttccctcctt 1920

gcgagctacg agattggcag gccttacggc gcagagtggg ctggcattcc tgaggagcag 1980

catgtgcagt atgtgattga cgcaatgatt gatatccacg gcgaggttgc acgacgagag 2040

tttaccggga aatgcaagag aaagtgttgg gctctggacg agttctcaaa cggggggttgg 2100

gcttcaccga ccgtgggtaa tcatgagacg tatctgccat cgtttttcga gacccacagc 2160

catgtgagtg ctccgacatt tgacctcttg tgcgaggaat gagggacttg ttgaccttgt 2220

tccagatgat attcgtgggt gagcatacct cctatacaca cgcatggatc gcctctgcga 2280

tcgaatctgc agtccgaggc agcgtgcagc tgctcttggg tatgtttcgc gttcttgcaa 2340

atcgtgcaga tgtgcttctg acattacttt gccacagagc ttggccttat agacgaggcg 2400

aaggatgtcg taaacacgtg gatggcacga tggatcagtg ttgtaagtga cctaagcaaa 2460

gccgtggtgt ctggagcgtc agcgtag 2487

<210> 2
<211> 689
<212> PRT
<213> Sarocladium sp.

<400> 2

Met Lys Ser Pro Ser Leu Ala Val Ala Gly Leu Leu Leu Gly Ser Ser
1 5 10 15

Ser Leu Ser His Ala Thr Gln Leu Arg Ile Glu Thr Arg Lys Ser Leu
20 25 30

Asn Ser Arg Ile Ala Asn Val His Ile Asp Val Asp Ala Pro Val Ala
35 40 45

His Gln Val Val Phe Thr Tyr Gly Pro Cys Asp Ser Glu Ser Gln Glu
50 55 60

```
Asn Ala His His Val Ile Ala Gln Ser Gln Lys Leu Glu Gly Arg Lys
65                  70                  75                  80

Pro His Arg Leu Ile Trp Thr Met Pro Lys Asp Leu His Pro Asp Asp
                85                  90                  95

Cys Ile Ser Ala Trp Gly Glu Ser Gly Asp Leu Leu Gly Arg Ser Val
            100                 105                 110

Pro Gln Lys Val Ala His Lys Glu Met Arg Arg Arg Lys Arg Asp Asp
        115                 120                 125

Ser Asp Tyr Ser Ile Pro Met Asn Ser Ser Ser Gly Ile Asp Val Tyr
    130                 135                 140

Gly Pro Trp Phe Asp Gly Val Ala Leu Leu Glu Lys Ser Asp Asn His
145                 150                 155                 160

Asn Val Asp Val Glu Ala Ala Lys Ala Lys Glu Ile Ala Ile Val Gly
                165                 170                 175

Ala Gly Met Ala Gly Leu Thr Thr Tyr Phe Ile Leu Ser Glu Ala Gly
            180                 185                 190

Leu Ser Asn Leu Thr Ile Leu Glu Ala Ser Gly Arg Leu Gly Gly Arg
        195                 200                 205

Val Arg Thr Glu Tyr Leu Ser Gly Gly Pro Arg Asp His Ser Tyr Ala
    210                 215                 220

Glu Met Gly Pro Met Arg Ile Pro Tyr Gln Ala Arg Phe Gly Asp Lys
225                 230                 235                 240

Ala Tyr Asn Ile Ser Asp Gln Ala Ile Phe Phe Arg Leu Val Glu Lys
                245                 250                 255

Val Asn Glu Arg Asn Lys Lys Leu Gly Asn Thr Lys Asp Leu Ile Asn
            260                 265                 270

Leu Ile Pro Phe Ile Gln Ser Ser Pro Asn Gly Leu Ala Tyr Tyr Gln
        275                 280                 285

Gly Asn Lys Leu Glu Asn Gly Leu Pro Pro Thr Gln Ala Asp Val Ala
    290                 295                 300

Ala Asp Pro Ala Leu Gly Asn Glu Ser Pro Glu Ile Pro Glu Ser Ala
305                 310                 315                 320
```

```
Gln Glu Leu Ala Ala Gln Leu Gln Arg Ala Leu Pro Asn Ala Glu Phe
                325                 330                 335

Leu Glu Leu Met Ala Thr Asn Phe Trp Gln Ala His Ala Glu Phe Leu
            340                 345                 350

Glu Asn Gln Gly Pro Ala Gly Leu Pro Gly Asp Gln Trp Ser Glu Phe
        355                 360                 365

Ala Phe Leu Val Asn Tyr Leu Asn Ala Thr Val Phe Asp Ala Asn Ala
    370                 375                 380

Val Thr Gly Gly Tyr Asp Trp His Ser Ser Leu Asp Arg Ser Ser Leu
385                 390                 395                 400

Val Gly Met Asn Leu Leu Pro Asn Ala Phe His Pro Leu Val Asp Asp
                405                 410                 415

Ile Thr Lys Phe Asn Ala Lys Val Glu Lys Val Gln Leu Asp Glu Lys
            420                 425                 430

Thr Ser Arg Leu Lys Leu His Trp Arg Ala Asn Tyr Thr Asp Pro Glu
        435                 440                 445

Leu Glu Ser Gln Ser Phe Asp Tyr Ala Ile Leu Ser Pro Thr Met Pro
    450                 455                 460

Ala Val Gln Lys Leu Arg Leu Pro Gly Leu Pro Phe Ala Met Arg Asn
465                 470                 475                 480

Ala Val Asp Ser Met Pro Tyr Ala Ser Ala Cys Lys Val Ala Leu Glu
                485                 490                 495

Tyr Arg Thr Arg Phe Trp Glu Lys Phe Asp Asn Pro Ile Tyr Gly Ser
            500                 505                 510

Cys Ser Thr Ser Thr Asp Ile Pro Gly Ile Gly Ser Val Cys Tyr Pro
        515                 520                 525

Ser Ser Asn Ile Asn Gly Ser Gly Pro Ala Ser Leu Leu Ala Ser Tyr
    530                 535                 540

Glu Ile Gly Arg Pro Tyr Gly Ala Glu Trp Ala Gly Ile Pro Glu Glu
545                 550                 555                 560

Gln His Val Gln Tyr Val Ile Asp Ala Met Ile Asp Ile His Gly Glu
                565                 570                 575
```

Val Ala Arg Arg Glu Phe Thr Gly Lys Cys Lys Arg Lys Cys Trp Ala
580 585 590

Leu Asp Glu Phe Ser Asn Gly Gly Trp Ala Ser Pro Thr Val Gly Asn
595 600 605

His Glu Thr Tyr Leu Pro Ser Phe Phe Glu Thr His Ser His Met Ile
610 615 620

Phe Val Gly Glu His Thr Ser Tyr Thr His Ala Trp Ile Ala Ser Ala
625 630 635 640

Ile Glu Ser Ala Val Arg Gly Ser Val Gln Leu Leu Leu Glu Leu Gly
645 650 655

Leu Ile Asp Glu Ala Lys Asp Val Val Asn Thr Trp Met Ala Arg Trp
660 665 670

Ile Ser Val Val Ser Asp Leu Ser Lys Ala Val Val Ser Gly Ala Ser
675 680 685

Ala

<210> 3
<211> 2086
<212> DNA
<213> Sarocladium sp.

<400> 3
atgttcactc ccaaagcttg gatccctctg ttggccttga gccgcgaggt cttctccaat 60

cctacttctg catcccattc catctctttc cacggtctcc ttggcgtctc ttcggagtca 120

gtccacaaca tccacctcac ttatggcgat gccttcccac atggagactt ccgtgttgtc 180

tttggagact gcggtatgac cagtgaggat gaacttcatc acgaggttgc atctctctcc 240

acgaagatgg agtctgctcc tgatcgtttg gtctggcttg tgccaaagga tgtccgcgag 300

aatggttgtc ttcacgcttt ctcggagggc gtcctcctcg gtcggtctga gcctgtcgct 360

ttcacagagc cactccggaa gcgcgagtct ctctctgaga ttggtgactc cgatggcctc 420

tggttcagcg gaatccgcta tctgcagtca agcaacctga catccgtcaa ggccgccgaa 480

gctaaggaaa agaagattgg catcgttggc ggtggcattt ccggtctgat gacgagtctg 540

ctcctgactt ccgtcggcat gaccaactgg cacatcattg aggcgactga gcgtgtcgga 600

ggccgcatcc gcaccaagta tatgaatgga actagtcccg atgactacca gtaccaggag 660

atggggccca tgaggttccc cgttgaggtc aaatacaacg acaccaacga gacgctgccc 720

attcaggatc acaagatggt cttccagctg gctgaagtct tgaacgagat gaacggcaac 780

gacacagatc tcgctgtcaa gttcattccc tggacccaga acaatccgaa cactcccgcc 840

aactctcgag gataccgcct ccctgatggc cgcatcccta ccgcggccca aatcgcccag 900

aaccccagca tcttgccggc agctgccaac gcgtcagacc cccaagatgc ggaacttggc 960

aaggagtcct tggagcgtct cagtgacctt acccccgagc ggatgcgcaa catctccgcc 1020

aacatcttca aggcgcaccg agatgccatt gaccgcggct tgtttcactg gtctgaggca 1080

gcctatctcc ggtaccagct gggccttgac gacgatactg tggacttcgt ggctgcctcc 1140

gacaactatc ccatgttccc tgactggtgg cacgcggtct acttcgcggc cacgaagtgg 1200

ctcacgatcg acaagggcct cgattcgctg tccagggctt ttgtacccca cgtcaaggac 1260

aagatcacgt acggtcgcaa gattgaagcc atgcaatgga atgagtcaac atccaagatc 1320

tcactgtcat ggagggagag ccctcttgcg gcggcgaagt ccgatgagta cgactatgct 1380

gtcgtcgcgg tgccgttctc caaggttcgt ctgtggaagc ggccggctta ctccaacctc 1440

ttgacgaggg ctattggcaa gctgaactac gagcaggcct gcaaagtgag cacccccctca 1500

tccgcccgaa gagtgactag aatactaatc cctccaaaag gttgctctgc tctacgagac 1560

acgcttctgg gagcaccagg agatccccat ctttggaggc tgtggctcgg ttgatatccc 1620

gggtattggt ggcgtgtgct acccatcata cgagatcaac tccacgaggc ctggcgtgat 1680

tctctcttcc tacattaccg gcacagaagc cagatccgtg gtagccctga gcgaggaaga 1740

tcacgtcgcc atggtgcagc gtgccatggt cgaagtccac ggccctatgg cggatgagca 1800

gtggaccggg atttacgacc gtctgtgctg ggaggtggat gagaacgcgg ccggaggctg 1860

ggcttcgccg acagttggcc agcaggagct gttcatcccg gcgtaccaca agacggagct 1920

caacaccatc ttcatcggag agcacacgag catcacgcac gggtggatct tctcggccct 1980

ggaatcgtcg gtcaggggca cgacgcagtt gctgctcgat cttggtctag tggatgaggc 2040

gaagcagatt gttgagactt ggatggcgag gtggatcacc gtttga 2086

<210> 4
<211> 676
<212> PRT
<213> Sarocladium sp.

<400> 4

Met Phe Thr Pro Lys Ala Trp Ile Pro Leu Leu Ala Leu Ser Arg Glu
1 5 10 15

Val Phe Ser Asn Pro Thr Ser Ala Ser His Ser Ile Ser Phe His Gly
20 25 30

```
Leu Leu Gly Val Ser Ser Glu Ser Val His Asn Ile His Leu Thr Tyr
        35                  40                  45

Gly Asp Ala Phe Pro His Gly Asp Phe Arg Val Val Phe Gly Asp Cys
    50                  55                  60

Gly Met Thr Ser Glu Asp Glu Leu His His Glu Val Ala Ser Leu Ser
65                  70                  75                  80

Thr Lys Met Glu Ser Ala Pro Asp Arg Leu Val Trp Leu Val Pro Lys
                85                  90                  95

Asp Val Arg Glu Asn Gly Cys Leu His Ala Phe Ser Glu Gly Val Leu
            100                 105                 110

Leu Gly Arg Ser Glu Pro Val Ala Phe Thr Glu Pro Leu Arg Lys Arg
        115                 120                 125

Glu Ser Leu Ser Glu Ile Gly Asp Ser Asp Gly Leu Trp Phe Ser Gly
    130                 135                 140

Ile Arg Tyr Leu Gln Ser Ser Asn Leu Thr Ser Val Lys Ala Ala Glu
145                 150                 155                 160

Ala Lys Glu Lys Lys Ile Gly Ile Val Gly Gly Gly Ile Ser Gly Leu
                165                 170                 175

Met Thr Ser Leu Leu Leu Thr Ser Val Gly Met Thr Asn Trp His Ile
            180                 185                 190

Ile Glu Ala Thr Glu Arg Val Gly Gly Arg Ile Arg Thr Lys Tyr Met
        195                 200                 205

Asn Gly Thr Ser Pro Asp Asp Tyr Gln Tyr Gln Glu Met Gly Pro Met
    210                 215                 220

Arg Phe Pro Val Glu Val Lys Tyr Asn Asp Thr Asn Glu Thr Leu Pro
225                 230                 235                 240

Ile Gln Asp His Lys Met Val Phe Gln Leu Ala Glu Val Leu Asn Glu
                245                 250                 255

Met Asn Gly Asn Asp Thr Asp Leu Ala Val Lys Phe Ile Pro Trp Thr
            260                 265                 270

Gln Asn Asn Pro Asn Thr Pro Ala Asn Ser Arg Gly Tyr Arg Leu Pro
        275                 280                 285
```

Asp Gly Arg Ile Pro Thr Ala Ala Gln Ile Ala Gln Asn Pro Ser Ile
290 295 300

Leu Pro Ala Ala Ala Asn Ala Ser Asp Pro Gln Asp Ala Glu Leu Gly
305 310 315 320

Lys Glu Ser Leu Glu Arg Leu Ser Asp Leu Thr Pro Glu Arg Met Arg
325 330 335

Asn Ile Ser Ala Asn Ile Phe Lys Ala His Arg Asp Ala Ile Asp Arg
340 345 350

Gly Leu Phe His Trp Ser Glu Ala Ala Tyr Leu Arg Tyr Gln Leu Gly
355 360 365

Leu Asp Asp Asp Thr Val Asp Phe Val Ala Ala Ser Asp Asn Tyr Pro
370 375 380

Met Phe Pro Asp Trp Trp His Ala Val Tyr Phe Ala Ala Thr Lys Trp
385 390 395 400

Leu Thr Ile Asp Lys Gly Leu Asp Ser Leu Ser Arg Ala Phe Val Pro
405 410 415

His Val Lys Asp Lys Ile Thr Tyr Gly Arg Lys Ile Glu Ala Met Gln
420 425 430

Trp Asn Glu Ser Thr Ser Lys Ile Ser Leu Ser Trp Arg Glu Ser Pro
435 440 445

Leu Ala Ala Ala Lys Ser Asp Glu Tyr Asp Tyr Ala Val Val Ala Val
450 455 460

Pro Phe Ser Lys Val Arg Leu Trp Lys Arg Pro Ala Tyr Ser Asn Leu
465 470 475 480

Leu Thr Arg Ala Ile Gly Lys Leu Asn Tyr Glu Gln Ala Cys Lys Val
485 490 495

Ala Leu Leu Tyr Glu Thr Arg Phe Trp Glu His Gln Glu Ile Pro Ile
500 505 510

Phe Gly Gly Cys Gly Ser Val Asp Ile Pro Gly Ile Gly Gly Val Cys
515 520 525

Tyr Pro Ser Tyr Glu Ile Asn Ser Thr Arg Pro Gly Val Ile Leu Ser
530 535 540

```
Ser Tyr Ile Thr Gly Thr Glu Ala Arg Ser Val Val Ala Leu Ser Glu
545                 550                 555                 560

Glu Asp His Val Ala Met Val Gln Arg Ala Met Val Glu Val His Gly
                565                 570                 575

Pro Met Ala Asp Glu Gln Trp Thr Gly Ile Tyr Asp Arg Leu Cys Trp
            580                 585                 590

Glu Val Asp Glu Asn Ala Ala Gly Gly Trp Ala Ser Pro Thr Val Gly
        595                 600                 605

Gln Gln Glu Leu Phe Ile Pro Ala Tyr His Lys Thr Glu Leu Asn Thr
    610                 615                 620

Ile Phe Ile Gly Glu His Thr Ser Ile Thr His Gly Trp Ile Phe Ser
625                 630                 635                 640

Ala Leu Glu Ser Ser Val Arg Gly Thr Thr Gln Leu Leu Leu Asp Leu
                645                 650                 655

Gly Leu Val Asp Glu Ala Lys Gln Ile Val Glu Thr Trp Met Ala Arg
            660                 665                 670

Trp Ile Thr Val
        675


<210>  5
<211>  2070
<212>  DNA
<213>  Sarocladium sp.

<400>  5
atgaagtctc catctctggc tgtggctggt ctcctgcttg gatcctctag cctctcgcat        60

gccacccagc tgcgcatcga aactcgtaag tcgctcaact cccgcatcgc taatgtgcat       120

atcgatgttg acgcccctgt cgctcaccag gtcgtgttca cctacggtcc ctgcgattcc       180

gagtctcagg aaaacgccca tcacgttatc gctcagtcgc agaagctcga gggacggaag       240

cctcatcgcc tgatctggac tatgcctaag gatcttcacc ccgatgactg tatctctgcc       300

tggggagaat ctggtgactt gctcggccgg tccgtgccac agaaggttgc tcataaggag       360

atgcgccgtc ggaagcgcga tgactctgac tacagcatcc caatgaactc gtcctctgga       420

atcgatgtgt atggcccgtg gttcgacggt gttgccctgc ttgagaagtc cgataaccac       480

aatgttgacg tcgaagccgc taaggccaag gagatcgcta tcgtcggagc cggcatggct       540

ggccttacca cttatttcat cttgtcggaa gccggtcttt ccaacttgac catcctcgaa       600
```

gcttctggac gcctgggcgg tcgtgtgcgc actgaatacc tttctggagg cccccgtgat 660

cacagctatg ccgagatggg ccctatgcgt atcccctacc aggcccggtt cggtgataag 720

gcttataata tcagcgacca ggctatcttc ttccgcctcg tcgagaaggt taacgagcgc 780

aataagaagc ttggaaacac caaggacctg atcaatctta tccctttcat ccagagctcg 840

cccaacggac tggcctacta tcagggcaac aagttggaaa atggtctccc tcccactcaa 900

gctgatgtgg ctgctgaccc agctctcggt aatgagtcgc cagaaattcc tgagtctgct 960

caagaattgg ctgctcagct tcagcgtgct ttgccgaacg ctgaattcct tgagttgatg 1020

gccaccaatt tctggcaggc ccatgctgaa ttcttggaga accagggtcc agctggactc 1080

ccgggcgatc agtggtctga gttcgccttc ctcgtcaact acctgaatgc taccgttttc 1140

gacgccaatg ctgtcactgg tggatatgat tggcattcct ctcttgaccg cagctcgttg 1200

gttggcatga acttgctccc aaatgccttc cacccgttgg tcgatgacat caccaagttc 1260

aacgctaagg tcgaaaaggt gcagctcgat gagaagacct ctcgcctcaa gctgcactgg 1320

cgtgccaatt acactgatcc agagttggaa agccagtcgt tcgactatgc catcctcagc 1380

cctaccatgc ccgctgtcca gaagcttcgt ttgccaggct tgccgttcgc catgcggaac 1440

gctgtcgact ctatgcctta cgccagcgct tgcaaggtgg ctcttgaata tcgtactcgg 1500

ttctgggaga agttcgataa tcccatctac ggttcctgct ctaccagcac tgacatccca 1560

ggtatcggat ccgtttgtta tccgtcctct aacatcaatg gctccggtcc ggcctctctg 1620

cttgctagct acgaaattgg acgtccttat ggtgctgagt gggctggcat ccccgaggaa 1680

cagcatgttc agtacgtcat cgatgccatg atcgacatcc acggagaagt cgctcgccgt 1740

gagttcaccg gcaagtgcaa gcgtaagtgt tgggccctgg atgagttctc taacggcggt 1800

tgggcctctc caaccgtggg aaatcatgaa acttaccttc cgtcgttctt cgagactcat 1860

tcccacatga tcttcgttgg cgaacatacc tcgtatactc acgcctggat cgcctcggct 1920

atcgagtccg ctgtccgtgg ttccgtgcag ttgctcctgg aactcggact gatcgatgag 1980

gccaaggacg ttgtcaacac ctggatggct cggtggatct cggtggtttc cgatctgtct 2040

aaggccgtcg tgagcggtgc ctcggcttga 2070

<210> 6
<211> 2031
<212> DNA
<213> Artificial Sequence

<220>
<223> codon-modified g4462 (penox1)

<400> 6
atgttcactc ctaaggcttg gattccactc ctggctctga gccgcgaggt cttctcgaac 60

cctacctctg cctctcatag catctcgttc cacggacttt tgggcgtctc ctctgaatct 120
gtgcataata tccaccttac ctacggtgac gctttccccc atggagattt ccgtgtcgtg 180
ttcggtgact gcggaatgac ttctgaggat gaactgcatc acgaggtcgc ctccctttct 240
accaagatgg aaagcgctcc agaccgcctc gtgtggctgg ttccgaagga tgtgcgtgag 300
aacggatgtt tgcacgcctt ctctgaaggc gttctcctgg gtcggagcga gcctgtcgct 360
ttcactgaac ccttgcgcaa gcgtgagagc ctctcggaaa tcggagactc tgatggcctg 420
tggttcagcg gcatccgcta tctgcagagc tcgaatctta cctctgtcaa ggccgctgag 480
gccaaggaaa agaagatcgg tatcgtgggc ggtggaatct ccggacttat gacctctctt 540
ttgctcacta gcgtgggtat gaccaactgg catatcatcg aggctaccga acgcgttggc 600
ggtcggatcc gcactaagta catgaatgga accagccctg atgactacca gtatcaggag 660
atgggcccta tgcgtttccc cgtcgaggtg aagtataacg acaccaatga aactctcccc 720
atccaggatc acaagatggt gttccagttg gccgaggttc tcaacgaaat gaacggcaat 780
gacactgatt tggctgtcaa gttcatccca tggactcaga acaatcctaa cacccctgcc 840
aatagccgcg gctaccgtct cccagacggt cgtatcccga ccgccgctca gatcgctcag 900
aacccatcta ttttgcctgc tgctgctaat gcttctgacc cacaggatgc tgagcttggc 960
aaggagtcgc ttgaacggtt gtccgatctc actccggaac gtatgcggaa catctcggcc 1020
aatatcttca aggcccatcg tgacgctatc gatcggggtc tgttccactg gtccgaggct 1080
gcctaccttc gctatcagct cggactggat gacgataccg tcgacttcgt ggctgcctcc 1140
gataactacc caatgttccc ggactggtgg catgctgtgt atttcgctgc caccaagtgg 1200
ctgactatcg acaagggcct ggattccctt tctcgggcct tcgttccaca cgtcaaggat 1260
aagatcactt acggtcgcaa gatcgaggct atgcagtgga atgaaagcac ctcgaagatc 1320
tccctttcct ggcgtgagtc gccgctcgct gctgctaagt ccgacgaata cgattatgcc 1380
gttgtcgctg ttccattctc taaggtccgt ctgtggaagc gtcctgccta ttccaacctg 1440
cttactcgcg ctatcggcaa gcttaattac gagcaggcct gcaaggtggc tttgctctat 1500
gaaacccgtt tctgggagca tcaggaaatc ccaatcttcg gaggctgcgg atcggtggat 1560
atccctggca tcggtggagt ttgttacccc tcttatgaga tcaacagcac ccgtccggga 1620
gttatcttgt cctcttatat caccggcact gaagcccggt cggtggttgc tctctccgag 1680
gaagaccatg tggccatggt tcagcgggct atggttgagg tccacggtcc catggccgat 1740
gaacagtgga ctggaatcta cgaccgcctc tgttgggaag tcgatgaaaa tgctgctggc 1800
ggttgggctt ctcctactgt tggacagcag gagttgttca tccccgctta tcacaagacc 1860
gagctcaata ctatcttcat cggcgaacat acctcgatca ctcacggttg gatcttctcc 1920
gccctggaga gctcggttcg tggcaccact cagctgcttt tggaccttgg tttggtcgat 1980

gaggccaagc agatcgtgga aacttggatg gctcggtgga tcaccgtctg a 2031

<210> 7
<211> 748
<212> DNA
<213> Artificial Sequence

<220>
<223> Ptef

<400> 7
tgtggaccag acaggcgcca ctcggccggg ccacaactgc ttgggttttg accgggagcg 60

gaccaattaa ggactcgaac gaccgcgggg ttcaaatgca aacaagtaca acacgcagca 120

aacgaagcag cccaccactg cgttgatgcc cagtttgtct gtccgaaatc caccggaaag 180

gtggaaacat actatgtaac aatcagaggg aagaaaaatt ttttatcgac gaggcaggat 240

agtgactgat ggtggggtca tggtcgggtc tccgagcgaa agagaaccaa ggaaacaaga 300

tcaacgaggt tggtgtaccc aaaaggccgc agcaacaaga gtcatcgccc aaaagtcaac 360

agtctggaag agactccgcc gtgcagattc tgcgtcggtc ccgcacatgc gtggtggggg 420

cattacccct ccatgtccaa tgataagggc ggcggtcgag ggcttaagcc cgcccactaa 480

ttcgccttct cgcttgcccc tccatataag gattcccctc cttcccctcc cacaactttt 540

ttcctctttc tctcttcgtc cgcatcagta cgtatatctt tccccccctac ctctttctca 600

ctcttcctcg attcattcca ctcttctcct tactgacatc tgttttgctc agtacctcta 660

cgcgatcagc cgtagtatct gagcaagctt ttttacagaa tctttctagt atcttacaaa 720

gaactacaaa gttcgcacca ccttcaaa 748

<210> 8
<211> 800
<212> DNA
<213> Artificial Sequence

<220>
<223> Talp

<400> 8
gtaccaggag tacattggag agttctacca ttgttgctgg aatacaatga tgattagaaa 60

ccgaagagtg ttatgattcg gacggatata cgcatggcac gcatacagcg tgatacatag 120

gctgtttgct caagaattag gattttatct gaatccatgt acagagttta cttatgttag 180

tagtcaatga aatcttggct ttctaatttt gtccgatcta caaggggtag tcgatcacag 240

aacgaactag atgtgcaggg aacgatgatc acccgctctt agcaagacct ctagtagttt 300

tcgaccatag ctttaacgcg aatcatgacc ctactatttt ctagattgca gaccaagtca 360

catgacaatg tcctctttga agtaggatca gtagctgatt agattccggg aaatgaatta 420

gggctggcgt tccaactact ggggagtgcc gatgttgctg tatgaaagat agtaagatta 480

ctagtgcaca gctgtagtaa ttatttactc tagattatat attccaaata ataagtaatc 540

taagatagta gacagtccta tgatatagct ccgggttcga agtcggcaaa agatatgcaa 600

tcacctgtcg ggatgatata tgtatatctg aaataccgac atcaaccatc cagtcggatc 660

agctaaacga agtatcactt ctttcgccac tgccaatcac tacttctatt aaagttcatg 720

ttacagtata agccacaaga cttatctcca gaactaactt gtgcatagga gctctgccga 780

tagccgggtg gttggatcgg 800

<210> 9
<211> 1487
<212> DNA
<213> Artificial Sequence

<220>
<223> pyrG3

<400> 9
taagtactca tttatacaat agttgcagaa ccccgcgcta cccctccatt gccaacatgt 60

cttccaagtc gcaattgacc tacagcgcac gcgctagcaa gcaccccaat gcgctcgtga 120

agaagctctt cgaggttgcc gaggccaaga aaaccaatgt caccgtttcc gccgacgtga 180

caaccaccaa agagctgctg gatttggctg accgtatgcg caccggggat gccacttaca 240

tatgatctag taatggttaa tggtggaata tataacagga ctcggtccgt acattgccgt 300

gatcaaaact cacatcgata tcctctccga tttcagcgaa gagaccatca tcggtctgaa 360

ggcccttgca gagaagcaca atttcctcat cttcgaagat cgcaagttca tcgatatcgg 420

aaacacagtc caaaagcagt accatggcgg cactctgcgc atctctgagt gggcccacat 480

catcaactgc agtattctgc ccggtgaggg tatcgtcgag gctctggccc agactgcttc 540

ggccgaggac ttcccctatg gctctgagag ggcccttttg atccttgcgg agatgacatc 600

caagggatct ttggctaccg gtcaatatac tacttcttct gttgactatg cccggaagta 660

taagaagttt gtgatgggat tcgtctcgac gcgtcacctg ggcgaggttc agtctgaagt 720

tagctcgcct tcggaggagg aggatttcgt cgtcttcacg acaggtgtca acctctcctc 780

gaagggagac aaactgggac agcaatacca gactcctgag tctgctgttg gacgcggtgc 840

cgactttatc attgctggtc gtggaattta tgctgctcct gatcccgtgg aggcagcgaa 900

gcggtaccag aaagagggat gggatgcata ccagaagcgt gttggtgcgc aataagtagt 960

ggtgaatacg tgctcttttt atggcagtat atcgcaagta tgatgcgatt cataaattca 1020

gcagtcgaat tctacgagag aacgatgcta agagataccc tctctatatg aataatatgc 1080

ctgcctcgag atatggacat attcaagatc agagttaagg gtcatgtttc aaaatcacac 1140

caatctccaa catagacgag aatttttacc ggattgtctg aaggtgcagc tggagattgg 1200

tctattttct aagagtgggg tatcactaat gtacagtcgg tcactatcgt acaaacaatc 1260

acaattatat acaagatttc ccatcacccc ttactctaac atggcacttt tatccatcga 1320

gtccgagcct agccaccatt tggtgctttc gtagagacca aagtataacc ctgatccgac 1380

agcggccata aacgtgttga tagcacaccc tcggaatagt cctctcgggc catctgttcg 1440

tataatctcc cgtacggtat tgatcatcct tttcttctga ggtgcgg 1487

<210> 10
<211> 40
<212> DNA
<213> Artificial Sequence

<220>
<223> Ptef reverse primer

<400> 10
agatggagac ttcattttga aggtggtgcg aactttgtag 40

<210> 11
<211> 38
<212> DNA
<213> Artificial Sequence

<220>
<223> Talp forward primer

<400> 11
ggtgcctcgg cttgagtacc aggagtacat tggagagt 38

<210> 12
<211> 498
<212> PRT
<213> Crotalus adamanteus

<400> 12

Ala His Asp Arg Asn Pro Leu Glu Glu Cys Phe Arg Glu Thr Asp Tyr
1 5 10 15

Glu Glu Phe Leu Glu Ile Ala Lys Asn Gly Leu Thr Ala Thr Ser Asn
20 25 30

Pro Lys Arg Val Val Ile Val Gly Ala Gly Met Ala Gly Leu Ser Ala
35 40 45

Ala Tyr Val Leu Ala Gly Ala Gly His Gln Val Thr Val Leu Glu Ala
50 55 60

Ser Glu Arg Val Gly Gly Arg Val Arg Thr Tyr Arg Lys Lys Asp Trp
65 70 75 80

```
Tyr Ala Asn Leu Gly Pro Met Arg Leu Pro Thr Lys His Arg Ile Val
                85                  90                  95

Arg Glu Tyr Ile Lys Lys Phe Asp Leu Lys Leu Asn Glu Phe Ser Gln
            100                 105                 110

Glu Asn Glu Asn Ala Trp Tyr Phe Ile Lys Asn Ile Arg Lys Arg Val
        115                 120                 125

Arg Glu Val Lys Asn Asn Pro Gly Leu Leu Glu Tyr Pro Val Lys Pro
    130                 135                 140

Ser Glu Glu Gly Lys Ser Ala Ala Gln Leu Tyr Val Glu Ser Leu Arg
145                 150                 155                 160

Lys Val Val Glu Glu Leu Arg Ser Thr Asn Cys Lys Tyr Ile Leu Asp
                165                 170                 175

Lys Tyr Asp Thr Tyr Ser Thr Lys Glu Tyr Leu Leu Lys Glu Gly Asn
            180                 185                 190

Leu Ser Pro Gly Ala Val Asp Met Ile Gly Asp Leu Leu Asn Glu Asp
        195                 200                 205

Ser Gly Tyr Tyr Val Ser Phe Ile Glu Ser Leu Lys His Asp Asp Ile
    210                 215                 220

Phe Gly Tyr Glu Lys Arg Phe Asp Glu Ile Val Gly Gly Met Asp Gln
225                 230                 235                 240

Leu Pro Thr Ser Met Tyr Glu Ala Ile Lys Glu Lys Val Gln Val His
                245                 250                 255

Phe Asn Ala Arg Val Ile Glu Ile Gln Gln Asn Asp Arg Glu Ala Thr
            260                 265                 270

Val Thr Tyr Gln Thr Ser Ala Asn Glu Met Ser Ser Val Thr Ala Asp
        275                 280                 285

Tyr Val Ile Val Cys Thr Thr Ser Arg Ala Ala Arg Arg Ile Lys Phe
    290                 295                 300

Glu Pro Pro Leu Pro Pro Lys Lys Ala His Ala Leu Arg Ser Val His
305                 310                 315                 320

Tyr Arg Ser Gly Thr Lys Ile Phe Leu Thr Cys Thr Lys Lys Phe Trp
                325                 330                 335
```

Glu Asp Asp Gly Ile His Gly Gly Lys Ser Thr Thr Asp Leu Pro Ser
340 345 350

Arg Phe Ile Tyr Tyr Pro Asn His Asn Phe Thr Ser Gly Val Gly Val
355 360 365

Ile Ile Ala Tyr Gly Ile Gly Asp Asp Ala Asn Phe Phe Gln Ala Leu
370 375 380

Asp Phe Lys Asp Cys Ala Asp Ile Val Ile Asn Asp Leu Ser Leu Ile
385 390 395 400

His Glu Leu Pro Lys Glu Asp Ile Gln Thr Phe Cys His Pro Ser Met
405 410 415

Ile Gln Arg Trp Ser Leu Asp Lys Tyr Ala Met Gly Gly Ile Thr Thr
420 425 430

Phe Thr Pro Tyr Gln Phe Gln His Phe Ser Glu Ala Leu Thr Ala Pro
435 440 445

Phe Lys Arg Ile Tyr Phe Ala Gly Glu Tyr Thr Ala Gln Phe His Gly
450 455 460

Trp Ile Asp Ser Thr Ile Lys Ser Gly Leu Thr Ala Ala Arg Asp Val
465 470 475 480

Asn Arg Ala Ser Glu Asn Pro Ser Gly Ile His Leu Ser Asn Asp Asn
485 490 495

Glu Phe

<210> 13
<211> 498
<212> PRT
<213> Crotalus atrox

<400> 13

Ala His Asp Arg Asn Pro Leu Glu Glu Cys Phe Arg Glu Thr Asp Tyr
1 5 10 15

Glu Glu Phe Leu Glu Ile Ala Lys Asn Gly Leu Thr Ala Thr Ser Asn
20 25 30

Pro Lys Arg Val Val Ile Val Gly Ala Gly Met Ala Gly Leu Ser Ala
35 40 45

```
Ala Tyr Val Leu Ala Gly Ala Gly His Gln Val Thr Val Leu Glu Ala
    50                  55                  60

Ser Glu Arg Val Gly Gly Arg Val Arg Thr Tyr Arg Lys Lys Asp Trp
65                  70                  75                  80

Tyr Ala Asn Leu Gly Pro Met Arg Leu Pro Thr Lys His Arg Ile Val
                85                  90                  95

Arg Glu Tyr Ile Lys Lys Phe Asp Leu Lys Leu Asn Glu Phe Ser Gln
            100                 105                 110

Glu Asn Glu Asn Ala Trp Tyr Phe Ile Lys Asn Ile Arg Lys Arg Val
        115                 120                 125

Arg Glu Val Lys Asn Asn Pro Gly Leu Leu Glu Tyr Pro Val Lys Pro
    130                 135                 140

Ser Glu Glu Gly Lys Ser Ala Ala Gln Leu Tyr Val Glu Ser Leu Arg
145                 150                 155                 160

Lys Val Val Glu Glu Leu Arg Ser Thr Asn Cys Lys Tyr Ile Leu Asp
                165                 170                 175

Lys Tyr Asp Thr Tyr Ser Thr Lys Glu Tyr Leu Leu Lys Glu Gly Asn
            180                 185                 190

Leu Ser Pro Gly Ala Val Asp Met Ile Gly Asp Leu Leu Asn Glu Asp
        195                 200                 205

Ser Gly Tyr Tyr Val Ser Phe Ile Glu Ser Leu Lys His Asp Asp Ile
    210                 215                 220

Phe Gly Tyr Glu Lys Arg Phe Asp Glu Ile Val Gly Gly Met Asp Gln
225                 230                 235                 240

Leu Pro Thr Ser Met Tyr Glu Ala Ile Lys Glu Lys Val Gln Val His
                245                 250                 255

Phe Asn Ala Arg Val Ile Glu Ile Gln Gln Asn Asp Arg Glu Ala Thr
            260                 265                 270

Val Thr Tyr Gln Thr Ser Ala Asn Glu Met Ser Ser Val Thr Ala Asp
        275                 280                 285

Tyr Val Ile Val Cys Thr Thr Ser Arg Ala Ala Arg Arg Ile Lys Phe
    290                 295                 300
```

Glu Pro Pro Leu Pro Pro Lys Lys Ala His Ala Leu Arg Ser Val His
305                 310                 315                 320

Tyr Arg Ser Gly Thr Lys Ile Phe Leu Thr Cys Thr Lys Lys Phe Trp
                325                 330                 335

Glu Asp Asp Gly Ile His Gly Gly Lys Ser Thr Thr Asp Leu Pro Ser
            340                 345                 350

Arg Phe Ile Tyr Tyr Pro Asn His Asn Phe Thr Ser Gly Val Gly Val
        355                 360                 365

Ile Ile Ala Tyr Gly Ile Gly Asp Asp Ala Asn Phe Phe Gln Ala Leu
    370                 375                 380

Asp Phe Lys Asp Cys Ala Asp Ile Val Ile Asn Asp Leu Ser Leu Ile
385                 390                 395                 400

His Glu Leu Pro Lys Glu Asp Ile Gln Thr Phe Cys His Pro Ser Met
                405                 410                 415

Ile Gln Arg Trp Ser Leu Asp Lys Tyr Ala Met Gly Gly Ile Thr Thr
            420                 425                 430

Phe Thr Pro Tyr Gln Phe Gln His Phe Ser Glu Ala Leu Thr Ala Pro
        435                 440                 445

Phe Lys Arg Ile Tyr Phe Ala Gly Glu Tyr Thr Ala Gln Phe His Gly
    450                 455                 460

Trp Ile Asp Ser Thr Ile Lys Ser Gly Leu Thr Ala Ala Arg Asp Val
465                 470                 475                 480

Asn Arg Ala Ser Glu Asn Pro Ser Gly Ile His Leu Ser Asn Asp Asn
                485                 490                 495

Glu Phe

<210>  14
<211>  618
<212>  PRT
<213>  Trichoderma viride

<400>  14

Met Asp Asn Val Asp Phe Ala Glu Ser Val Arg Thr Arg Trp Ala Arg
1               5                   10                  15

Arg Leu Ile Arg Glu Lys Val Ala Lys Glu Leu Asn Ile Leu Thr Glu
20 25 30

Arg Leu Gly Glu Val Pro Gly Ile Pro Pro Pro Arg Glu Gly Arg Phe
35 40 45

Leu Gly Gly Gly Tyr Ser His Asp Asn Leu Pro Ser Asp Pro Leu Tyr
50 55 60

Ser Ser Ile Lys Pro Ala Leu Leu Lys Glu Ala Pro Arg Ala Glu Glu
65 70 75 80

Glu Leu Pro Pro Arg Lys Val Cys Ile Val Gly Ala Gly Val Ser Gly
85 90 95

Leu Tyr Ile Ala Met Ile Leu Asp Asp Leu Lys Ile Pro Asn Leu Thr
100 105 110

Tyr Asp Ile Phe Glu Ser Ser Ser Arg Thr Gly Gly Arg Leu Tyr Thr
115 120 125

His His Phe Thr Asp Ala Lys His Asp Tyr Tyr Asp Ile Gly Ala Met
130 135 140

Arg Tyr Pro Asp Ile Pro Ser Met Lys Arg Thr Phe Asn Leu Phe Lys
145 150 155 160

Arg Thr Gly Met Pro Leu Ile Lys Tyr Tyr Leu Asp Gly Glu Asn Thr
165 170 175

Pro Gln Leu Tyr Asn Asn His Phe Phe Ala Lys Gly Val Val Asp Pro
180 185 190

Tyr Met Val Ser Val Ala Asn Gly Gly Thr Val Pro Asp Asp Val Val
195 200 205

Asp Ser Val Gly Glu Lys Leu Gln Gln Ala Phe Gly Tyr Tyr Lys Glu
210 215 220

Lys Leu Ala Glu Asp Phe Asp Lys Gly Phe Asp Glu Leu Met Leu Val
225 230 235 240

Asp Asp Met Thr Thr Arg Glu Tyr Leu Lys Arg Gly Gly Pro Lys Gly
245 250 255

Glu Ala Pro Lys Tyr Asp Phe Phe Ala Ile Gln Trp Met Glu Thr Gln

260 265 270

Asn Thr Gly Thr Asn Leu Phe Asp Gln Ala Phe Ser Glu Ser Val Ile
275 280 285

Asp Ser Phe Asp Phe Asp Asn Pro Thr Lys Pro Glu Trp Tyr Cys Ile
290 295 300

Glu Gly Gly Thr Ser Leu Leu Val Asp Ala Met Lys Glu Thr Leu Val
305 310 315 320

His Lys Val Gln Asn Asn Lys Arg Val Glu Ala Ile Ser Ile Asp Leu
325 330 335

Asp Ala Pro Asp Asp Gly Asn Met Ser Val Lys Ile Gly Gly Lys Asp
340 345 350

Tyr Ser Gly Tyr Ser Thr Val Phe Asn Thr Thr Ala Leu Gly Cys Leu
355 360 365

Asp Arg Met Asp Leu Arg Gly Leu Asn Leu His Pro Thr Gln Ala Asp
370 375 380

Ala Ile Arg Cys Leu His Tyr Asp Asn Ser Thr Lys Val Ala Leu Lys
385 390 395 400

Phe Ser Tyr Pro Trp Trp Ile Lys Asp Cys Gly Ile Thr Cys Gly Gly
405 410 415

Ala Ala Ser Thr Asp Leu Pro Leu Arg Thr Cys Val Tyr Pro Ser Tyr
420 425 430

Asn Leu Gly Asp Thr Gly Glu Ala Val Leu Leu Ala Ser Tyr Thr Trp
435 440 445

Ser Gln Asp Ala Thr Arg Ile Gly Ser Leu Val Lys Asp Ala Pro Pro
450 455 460

Gln Pro Pro Lys Glu Asp Glu Leu Val Glu Leu Ile Leu Gln Asn Leu
465 470 475 480

Ala Arg Leu His Ala Glu His Met Thr Tyr Glu Lys Ile Lys Glu Ala
485 490 495

Tyr Thr Gly Val Tyr His Ala Tyr Cys Trp Ala Asn Asp Pro Asn Val
500 505 510

Gly Gly Ala Phe Ala Leu Phe Gly Pro Gly Gln Phe Ser Asn Leu Tyr
515 520 525

Pro Tyr Leu Met Arg Pro Ala Ala Gly Gly Lys Phe His Ile Val Gly
530 535 540

Glu Ala Ser Ser Val His His Ala Trp Ile Ile Gly Ser Leu Glu Ser
545 550 555 560

Ala Tyr Thr Ala Val Tyr Gln Phe Leu Tyr Lys Tyr Lys Met Trp Asp
565 570 575

Tyr Leu Arg Leu Leu Leu Glu Arg Trp Gln Tyr Gly Leu Gln Glu Leu
580 585 590

Glu Thr Gly Lys His Gly Thr Ala His Leu Gln Phe Ile Leu Gly Ser
595 600 605

Leu Pro Lys Glu Tyr Gln Val Lys Ile Val
610 615

<210> 15
<211> 517
<212> PRT
<213> Aplysia californica

<400> 15

Asp Gly Val Cys Arg Asn Arg Arg Gln Cys Asn Lys Glu Val Cys Gly
1 5 10 15

Ser Ser Tyr Asp Val Ala Ile Val Gly Ala Gly Pro Gly Gly Ala Asn
20 25 30

Ser Ala Tyr Met Leu Arg Glu Ser Gly Leu Asp Ile Ala Val Phe Glu
35 40 45

Tyr Ser Asp Arg Val Gly Gly Arg Leu Phe Thr Tyr Gln Leu Pro Asn
50 55 60

Thr Pro Asp Val Asn Leu Glu Ile Gly Gly Met Arg Phe Ile Glu Gly
65 70 75 80

Ala Met His Arg Leu Trp Lys Val Ile Ser Glu Leu Gly Leu Thr Pro
85 90 95

Lys Val Phe Lys Glu Gly Phe Gly Lys Glu Gly Arg Gln Arg Phe Tyr
100 105 110

```
Leu Arg Gly Gln Ser Leu Thr Lys Lys Gln Val Lys Ser Gly Asp Val
        115                 120                 125

Pro Tyr Asp Leu Ser Pro Glu Glu Lys Ala Asn Gln Gly Arg Leu Val
    130                 135                 140

Glu Tyr Tyr Leu Glu Lys Leu Thr Gly Leu Gln Leu Asn Gly Gly Pro
145                 150                 155                 160

Leu Lys Arg Glu Val Ala Leu Lys Leu Thr Val Pro Asp Gly Arg Phe
                165                 170                 175

Leu Tyr Asp Leu Thr Phe Asp Glu Ala Leu Asp Leu Val Ala Ser Pro
            180                 185                 190

Glu Gly Lys Glu Phe Ala Arg Asp Thr His Val Phe Thr Ser Glu Val
        195                 200                 205

Thr Leu Asp Ala Ser Ala Ile Ser Ile Phe Asp Asp His Leu Gly Glu
    210                 215                 220

Asp Tyr Tyr Gly Ser Glu Ile Tyr Thr Leu Glu Glu Gly Met Ser Ser
225                 230                 235                 240

Val Pro Gln Gly Leu Leu Gln Thr Phe Leu Asp Ala Ala Lys Ser Asn
                245                 250                 255

Glu Phe Phe Pro Asn Asn His Leu Lys Ala Leu Arg Arg Arg Thr Asn
            260                 265                 270

Gly Gln Tyr Val Leu Tyr Phe Glu Pro Thr Thr Ser Lys Asp Gly Gln
        275                 280                 285

Thr Thr Ile Asn Tyr Leu Glu Pro Leu Lys Val Val Cys Ala Gln Arg
    290                 295                 300

Val Ile Leu Ala Met Pro Val Tyr Ala Leu Arg Gln Leu Asp Trp Ser
305                 310                 315                 320

Gln Leu Arg Asn Asp Arg Ala Thr Gln Ala Tyr Arg Ala Val Arg Pro
                325                 330                 335

Met Pro Ala Ser Lys Val Phe Met Thr Phe Asp Gln Pro Trp Trp Leu
            340                 345                 350

Gln Asn Glu Arg Lys Ser Trp Val Thr Lys Ser Asp Ala Leu Phe Ser
        355                 360                 365
```

Gln Met Tyr Asp Trp Gln Lys Ser Glu Ala Ser Gly Asp Tyr Ile Leu
370 375 380

Ile Ala Ser Tyr Ala Asp Gly Leu Lys Ala Gln Tyr Leu Arg Glu Leu
385 390 395 400

Lys Asn Gln Gly Glu Asp Ile Pro Gly Ser Asp Pro Gly Tyr Asn Gln
405 410 415

Val Thr Val Pro Leu Lys Asp Ala Ile Leu Glu His Leu Thr Glu Ala
420 425 430

Tyr Gly Val Glu Arg Asp Ser Ile Pro Glu Pro Val Thr Ala Ala Ser
435 440 445

Gln Phe Trp Thr Asp Tyr Pro Phe Gly Cys Gly Trp Ile Thr Trp Arg
450 455 460

Ala Gly Tyr His Phe Asp Asp Val Ile Ser Thr Met Arg Arg Pro Ser
465 470 475 480

Leu Lys Asp Glu Val Tyr Val Val Gly Ser Asp Tyr Ser Trp Gly Leu
485 490 495

Ile Ser Ser Trp Ile Glu Gly Ala Leu Glu Thr Ser Glu Asn Val Ile
500 505 510

Asn Asp Tyr Phe Leu
515

<210> 16
<211> 69
<212> DNA
<213> Aspergillus oryzae

<220>
<223> AoCDHss

<400> 16
atgaaacttg tcaaccgtct gctggcttcg ttcttgtccg catccaccgt tctccagtcg 60

tgctgggct 69

<210> 17
<211> 1554
<212> DNA
<213> Aplysia californica

<400> 17
gacggcgtct gcagaaacag gcgtcagtgt aacaaagagg tgtgcggttc ctcttatgac 60

gtggccatcg tgggggcggg gccaggggga gctaactccg cctacatgct gagggagtct 120

ggcctggaca tcgctgtgtt cgagtactct gaccgggtgg gtggccgtct gttcacctac 180

cagctgccca acacacccga cgtgaacctg gagattggcg gcatgaggtt catcgagggc 240

gccatgcaca gactctggaa agtcatttca gaactcggac tgacccccaa ggtgttcaag 300

gaaggtttcg gcaaggaggg cagacagagg ttctacctac gtggacagag cctgaccaag 360

aaacaggtca agagcgggga cgtaccctat gacctcagcc cggaggagaa ggccaaccag 420

ggacgtctgg tcgaatacta cctggagaaa ctgaccggat tacagctcaa cggcggacca 480

ctcaaacggg aggtggcgct caaactgacc gtgccggacg gcagattcct ctatgacctc 540

acgtttgacg aagccctgga cctggtggcc tcccctgagg gcaaagagtt cgcccgagac 600

acgcacgtgt ttacctcaga ggtcacactg gacgcgtcgg ctatctccat cttcgatgac 660

cacctgggag aggactacta tggcagtgag atctacaccc tggaggaagg aatgtcttct 720

gtaccacaag gactgctaca gacttttctg gacgccgcta aatccaacga gttcttcccc 780

aacaaccacc tgaaggctct gagacggagg accaatggtc agtatgtcct gtactttgag 840

cccaccacgt ccaaggatgg acaaaccacc atcaactacc tggaacccct gaaggttgtg 900

tgtgctcaga gagtcatcct ggccatgccg gtctacgctc tcagacaact ggactggtca 960

caactcagaa atgaccgcgc cacccaagcg tacagagccg tgcgtcctat gcctgccagt 1020

aaagtcttta tgacctttga ccagccctgg tggttgcaga atgagaggaa atcttgggta 1080

accaaatcag acgcgctttt cagccaaatg tatgactggc agaagtctga ggcgtctgga 1140

gattacatcc tgatcgccag ctacgccgac ggcctcaaag cccagtactt gcgggagctg 1200

aagaaccagg gagaggacat cccaggctct gaccccggct acaaccaggt caccgtaccc 1260

ctcaaggacg ccattcttga acacctcacc gaggcttacg gcgtggagcg agactcgatc 1320

ccagaacccg tgactgctgc ctcccagttt tggacagact acccgtttgg ctgcggctgg 1380

atcacctgga gggccggtta ccattttgat gacgtcatca gcaccatgcg tcgcccgtca 1440

ctgaaagatg aagtctacgt ggtaggatcg gattactcct ggggacttat ctcctcctgg 1500

atagagggcg ctcttgagac atcggaaaac gtcatcaacg actacttcct ctag 1554

<210> 18
<211> 1554
<212> DNA
<213> Aplysia californica

<400> 18
gatggggttt gtcggaatcg ccgccaatgc aacaaggagg tttgcggttc ttcctacgac 60

gtggctatcg tgggagccgg ccctggcggc gctaactctg cttacatgct ccgcgaatct 120

gggttggaca tagctgtatt cgaatattcc gaccgagtcg gcggtcgact cttcacctat 180

cagctcccta acaccccga cgtcaatctc gaaattggag gtatgcgctt catcgaaggg 240

gccatgcacc gtctttggaa agtaatatcc gaactcggcc ttacgcctaa ggtcttcaag 300

gaggggttcg gtaaggaagg tcgacagcgt ttttatctgc gcggccaatc gctcaccaaa 360

aagcaagtga aaagtggtga tgtgccctac gacctgtctc ctgaggaaaa ggccaatcag 420

ggccgcctgg tcgagtacta tctggagaag ctcaccggac tccagctcaa tggcggtccc 480

ctcaaacgag aagtggcgct gaagctcacc gtccccgatg gccgttttct ctatgatctc 540

accttcgacg aggctcttga cctggtcgcc tccccagagg gtaaagaatt cgcacgcgat 600

acccacgttt tcaccagcga agtcactctg gacgcatctg ccatcagtat tttcgacgac 660

catcttggtg aggattatta tggcagcgaa atctatacct tggaggaagg catgtcctcc 720

gtcccccaag gccttctgca aactttcctt gatgccgcca agagcaacga atttttcccc 780

aataatcacc tgaaggccct tcgacggcgg acgaacggcc agtacgtctt gtattttgag 840

cctactacgt ctaaggacgg acaaacaacg atcaattacc tggagcctct gaaggttgtc 900

tgcgcgcaac gggtgatcct ggctatgcca gtatacgccc tgcgtcaatt ggattggtct 960

cagctacgta atgatcgggc cactcaagca tatagggcag tcaggcctat gccggcttca 1020

aaagtattta tgacgttcga tcagccgtgg tggcttcaga acgagaggaa gagctgggtg 1080

actaagagtg atgcactatt tagtcagatg tacgattggc agaaaagcga agcgagtggt 1140

gactacattc ttattgcttc gtatgcggac ggtcttaaag ctcaatactt gagagagcta 1200

aaaaaccagg gagaggatat cccggggagc gatccggggt acaaccaggt gacagtacca 1260

ttgaaggacg cgatcctaga gcatttgact gaagcatacg gtgtggaaag agacagcatt 1320

ccggagccag ttactgcggc atcgcagttc tggacagact atccatttgg atgtgggtgg 1380

atcacatgga gagcaggata ccattttgat gatgtgattt cgacaatgag gagaccatca 1440

ttgaaggatg aggtttacgt tgttggatca gattactcgt ggggactaat ctcatcatgg 1500

attgagggag cactagagac atcggagaac gttattaacg attatttttt gtag 1554

<210> 19
<211> 498
<212> PRT
<213> Crotalus adamanteus

<400> 19

Ala His Asp Arg Asn Pro Leu Glu Glu Cys Phe Arg Glu Thr Asp Tyr
1 5 10 15

Glu Glu Phe Leu Glu Ile Ala Lys Asn Gly Leu Thr Ala Thr Ser Asn
20 25 30

Pro Lys Arg Val Val Ile Val Gly Ala Gly Met Ala Gly Leu Ser Ala
35 40 45

Ala Tyr Val Leu Ala Gly Ala Gly His Gln Val Thr Val Leu Glu Ala
50 55 60

Ser Glu Arg Val Gly Gly Arg Val Arg Thr Tyr Arg Lys Lys Asp Trp
65 70 75 80

Tyr Ala Asn Leu Gly Pro Met Arg Leu Pro Thr Lys His Arg Ile Val
85 90 95

Arg Glu Tyr Ile Lys Lys Phe Asp Leu Lys Leu Asn Glu Phe Ser Gln
100 105 110

Glu Asn Glu Asn Ala Trp Tyr Phe Ile Lys Asn Ile Arg Lys Arg Val
115 120 125

Arg Glu Val Lys Asn Asn Pro Gly Leu Leu Glu Tyr Pro Val Lys Pro
130 135 140

Ser Glu Glu Gly Lys Ser Ala Ala Gln Leu Tyr Val Glu Ser Leu Arg
145 150 155 160

Lys Val Val Glu Glu Leu Arg Ser Thr Asn Cys Lys Tyr Ile Leu Asp
165 170 175

Lys Tyr Asp Thr Tyr Ser Thr Lys Glu Tyr Leu Leu Lys Glu Gly Asn
180 185 190

Leu Ser Pro Gly Ala Val Asp Met Ile Gly Asp Leu Leu Asn Glu Asp
195 200 205

Ser Gly Tyr Tyr Val Ser Phe Ile Glu Ser Leu Lys His Asp Asp Ile
210 215 220

Phe Gly Tyr Glu Lys Arg Phe Asp Glu Ile Val Gly Gly Met Asp Gln
225 230 235 240

Leu Pro Thr Ser Met Tyr Glu Ala Ile Lys Glu Lys Val Gln Val His
245 250 255

Phe Asn Ala Arg Val Ile Glu Ile Gln Gln Asn Asp Arg Glu Ala Thr
260 265 270

Val Thr Tyr Gln Thr Ser Ala Asn Glu Met Ser Ser Val Thr Ala Asp
275 280 285

```
Tyr Val Ile Val Cys Thr Thr Ser Arg Ala Ala Arg Arg Ile Lys Phe
    290                 295                 300

Glu Pro Pro Leu Pro Pro Lys Lys Ala His Ala Leu Arg Ser Val His
305                 310                 315                 320

Tyr Arg Ser Gly Thr Lys Ile Phe Leu Thr Cys Thr Lys Lys Phe Trp
                325                 330                 335

Glu Asp Asp Gly Ile His Gly Gly Lys Ser Thr Thr Asp Leu Pro Ser
            340                 345                 350

Arg Phe Ile Tyr Tyr Pro Asn His Asn Phe Thr Ser Gly Val Gly Val
        355                 360                 365

Ile Ile Ala Tyr Gly Ile Gly Asp Asp Ala Asn Phe Phe Gln Ala Leu
    370                 375                 380

Asp Phe Lys Asp Cys Ala Asp Ile Val Ile Asn Asp Leu Ser Leu Ile
385                 390                 395                 400

His Glu Leu Pro Lys Glu Asp Ile Gln Thr Phe Cys His Pro Ser Met
                405                 410                 415

Ile Gln Arg Trp Ser Leu Asp Lys Tyr Ala Met Gly Gly Ile Thr Thr
            420                 425                 430

Phe Thr Pro Tyr Gln Phe Gln His Phe Ser Glu Ala Leu Thr Ala Pro
        435                 440                 445

Phe Lys Arg Ile Tyr Phe Ala Gly Glu Tyr Thr Ala Gln Phe His Gly
    450                 455                 460

Trp Ile Asp Ser Thr Ile Lys Ser Gly Leu Thr Ala Ala Arg Asp Val
465                 470                 475                 480

Asn Arg Ala Ser Glu Asn Pro Ser Gly Ile His Leu Ser Asn Asp Asn
                485                 490                 495

Glu Phe


<210>  20
<211>  12
<212>  DNA
<213>  Artificial Sequence

<220>
```

```
<223>  Synthetic gene (partial)

<400>  20
accaccttca aa                                                          12


<210>  21
<211>  12
<212>  DNA
<213>  Artificial sequence

<220>
<223>  Synthetic gene (partial)

<400>  21
gtaccaggag ta                                                          12


<210>  22
<211>  28
<212>  DNA
<213>  Artificial sequence

<220>
<223>  reverse primer

<400>  22
cattttgaag gtggtgcgaa ctttgtag                                         28


<210>  23
<211>  28
<212>  DNA
<213>  Artificial sequence

<220>
<223>  forward primer

<400>  23
tgagtaccag gagtacattg gagagttc                                         28
```

## Description

Technical Field

The present invention relates to a method for measuring pentosidine, etc. More specifically, the present invention relates to a method for measuring pentosidine, etc., which reduces a measurement error to obtain an accurate measurement value.

Background Art

Pentosidine ((2S)-2-amino-6-[2-[[(4S)-4-amino-4-carboxybutyl]amino]imidazo[4,5-b]pyridin-4-yl]hexanoic acid) has a structure where pentose, and equimolar lysine and arginine are cross-linked, and is known to accumulate in the human skin in correlation with aging or the development of diabetes mellitus and to increase, particularly, during the development of diabetes mellitus or in end-stage nephropathy.

It is known that pentosidine can be quantified by HPLC with its fluorescence (Ex: 335 nm, Em: 385 nm) as an index after acid hydrolysis, and can also be quantified by use of an immunochemical method (e.g., ELISA) using a monoclonal antibody against pentosidine.

Pentosidine is known to be associated with schizophrenia in addition to aging or diabetes mellitus. For example, a method for testing schizophrenia, comprising the step of measuring an amount of pentosidine with the intended use for biological samples is disclosed (see e.g., Patent Literature 1).

Citation List

Patent Literature

Patent Literature 1: Japanese Patent No. 5738346

Summary of Invention

Technical Problem

The quantification of pentosidine by an immunochemical method or an instrumental analytical approach may be complicated and expensive. An object of the present invention is to provide an inexpensive and simple method for measuring pentosidine using a novel enzyme, as compared with an immunochemical method or an instrumental analytical approach. Particularly, an object of the present invention is to provide a method for measuring pentosidine which reduces a measurement error to obtain an accurate measurement value.

Solution to Problem

The present inventors have completed the present invention by finding that a novel enzyme identified from a filamentous fungus is useful in the quantification of pentosidine and finding that use of this enzyme in combination with an amino acid degrading enzyme more reduces a measurement error.

The present invention is summarized as follows.

[1] A method for measuring pentosidine in a specimen, the measurement method comprising the steps of:

degrading the specimen with an amino acid degrading enzyme;
contacting the specimen after the degradation step with a protein having activity that oxidatively degrades pentosidine; and
detecting change resulting from the contact, wherein the amino acid degrading enzyme and the protein having activity that oxidatively degrades pentosidine are different from each other.

[2] The measurement method according to [1], wherein in the detection step, change in an amount of oxygen, hydrogen peroxide or ammonia is detected.
[3] The measurement method according to [1] or [2], wherein the protein having activity that oxidatively degrades pentosidine has the following physicochemical properties:

(1) action: activity that oxidatively degrades pentosidine; and

(2) molecular weight based on SDS-PAGE: 75,000 to 85,000.

[4] The measurement method according to any of [1] to [3], wherein the protein having activity that oxidatively degrades pentosidine is any protein selected from the group consisting of the following (a) to (f):

(a) a protein consisting of the amino acid sequence as set forth in SEQ ID NO: 2 or SEQ ID NO: 4;
(b) a protein encoded by a gene consisting of the nucleotide sequence as set forth in SEQ ID NO: 1, SEQ ID NO: 3, SEQ ID NO: 5 or SEQ ID NO: 6;
(c) a protein consisting of an amino acid sequence having 75% or higher identity to the amino acid sequence as set forth in SEQ ID NO: 2 or SEQ ID NO: 4;
(d) a protein encoded by a gene consisting of a nucleotide sequence having 75% or higher identity to the nucleotide sequence as set forth in SEQ ID NO: 1, SEQ ID NO: 3, SEQ ID NO: 5 or SEQ ID NO: 6;
(e) a protein consisting of an amino acid sequence derived from the amino acid sequence as set forth in SEQ ID NO: 2 or SEQ ID NO: 4 by the deletion, substitution and/or addition of one or more amino acids; and
(f) a protein encoded by a nucleotide sequence that hybridizes under stringent conditions to the nucleotide sequence as set forth in SEQ ID NO: 1, SEQ ID NO: 3, SEQ ID NO: 5 or SEQ ID NO: 6.

[5] The measurement method according to any of [1] to [4], wherein the protein having activity that oxidatively degrades pentosidine is derived from a filamentous fungus.
[6] The measurement method according to any of [1] to [5], wherein

the protein having activity that oxidatively degrades pentosidine is pentosidine oxidase, and
the amino acid degrading enzyme degrades an amino acid contained in the specimen, wherein the amino acid is selected from arginine, leucine, methionine, phenylalanine, tryptophan and tyrosine.

[6'] The measurement method according to any of [1] to [5], wherein the amino acid degrading enzyme degrades an amino acid selected from arginine, leucine, methionine, phenylalanine, tryptophan and tyrosine.
[7] The measurement method according to any of [1] to [6], wherein the amino acid to be degraded by the amino acid degrading enzyme is an amino acid against which the protein having activity that oxidatively degrades pentosidine has 40% or higher relative activity when the activity of the protein having activity that oxidatively degrades pentosidine against pentosidine is defined as 100%.
[8] The measurement method according to any of [1] to [7], wherein the amino acid degrading enzyme is selected from the group consisting of amino acid oxidase, amino acid dehydrogenase, amino acid aminotransferase, amino acid decarboxylase, amino acid ammonia lyase, amino acid oxygenase and amino acid hydrolase.
[9] A kit for measuring pentosidine in a specimen, comprising:

(i) an amino acid degrading enzyme; and
(ii) a protein having activity that oxidatively degrades pentosidine.

[10] The kit according to [9], wherein the protein having activity that oxidatively degrades pentosidine is any protein selected from the group consisting of the following (a) to (f):

(a) a protein consisting of the amino acid sequence as set forth in SEQ ID NO: 2 or SEQ ID NO: 4;
(b) a protein encoded by a gene consisting of the nucleotide sequence as set forth in SEQ ID NO: 1, SEQ ID NO: 3, SEQ ID NO: 5 or SEQ ID NO: 6;
(c) a protein consisting of an amino acid sequence having 75% or higher identity to the amino acid sequence as set forth in SEQ ID NO: 2 or SEQ ID NO: 4;
(d) a protein encoded by a gene consisting of a nucleotide sequence having 75% or higher identity to the nucleotide sequence as set forth in SEQ ID NO: 1, SEQ ID NO: 3, SEQ ID NO: 5 or SEQ ID NO: 6;
(e) a protein consisting of an amino acid sequence derived from the amino acid sequence as set forth in SEQ ID NO: 2 or SEQ ID NO: 4 by the deletion, substitution and/or addition of one or more amino acids; and
(f) a protein encoded by a nucleotide sequence that hybridizes under stringent conditions to the nucleotide sequence as set forth in SEQ ID NO: 1, SEQ ID NO: 3, SEQ ID NO: 5 or SEQ ID NO: 6.

[11] The kit according to [9] or [10], wherein the amino acid degrading enzyme is an enzyme that degrades an amino acid selected from arginine, leucine, methionine, phenylalanine, tryptophan and tyrosine.
[12] A method for producing a reaction product of pentosidine derived from a specimen, the method comprising the steps of:

degrading the specimen with an amino acid degrading enzyme; and
contacting the specimen after the degradation step with a protein having activity that oxidatively degrades pentosidine, wherein

the amino acid degrading enzyme and the protein having activity that oxidatively degrades pentosidine are different from each other.

[13] The measurement method according to any of [1] to [8], wherein the amino acid degrading enzyme comprises *Aplysia californica*-derived escapin and/or *Crotalus* adamanteus-derived amino acid oxidase.

[14] The measurement method according to any of [1] to [8], wherein the amino acid degrading enzyme degrades an amino acid contained in the specimen, wherein the amino acid is selected from asparagine, glutamine and histidine.

[14'] The measurement method according to any of [1] to [7], wherein the amino acid degrading enzyme degrades an amino acid selected from asparagine, glutamine and histidine.

[15] The kit according to any of [9] to [11], further comprising (iii) at least one member selected from a reagent for hydrogen peroxide detection, a reagent for ammonia detection, a reagent for pentosidine deamination product detection and a reagent for oxygen detection.

Advantageous Effects of Invention

The present invention enables pentosidine to be conveniently and rapidly detected and quantified by an enzymatic method. In this respect, a measurement error is reduced to obtain an accurate measurement value.

Brief Description of Drawings

[Figure 1] Figure 1 shows results of a substrate concentration dependence test on a partially purified enzyme liquid (Elution 1) fractionated by use of anion-exchange chromatography from *Sarocladium* sp. ΔOD (ordinate) was plotted against a final pentosidine concentration (abscissa). Data after 20 minutes from the start of reaction was used.

[Figure 2] Figure 2 shows results of a thermal deactivation test on the partially purified enzyme liquid (Elution 1). The results were of analyzing the deactivation of enzymatic activity by heat treatment and corresponded to data after 20 minutes from the start of reaction.

[Figure 3] Figure 3 shows results of measuring the concentration of hydrogen peroxide produced through the reaction of pentosidine with pentosidine oxidase. The concentration of hydrogen peroxide was measured at absorbance of 658 nm.

[Figure 4] Figure 4 shows the relationship between the final concentration of pentosidine and the amount of elevation in $A_{658}$ (ΔA) caused by the oxidation of pentosidine.

[Figure 5] Figure 5 shows a putative mechanism of reaction through which pentosidine oxidase degrades pentosidine. The drawing illustrates a manner in which the respective amino groups of lysine and arginine constituting pentosidine are oxidatively deaminated to form hydrogen peroxide and ammonia.

[Figure 6A] Figure 6A shows the sequences of SEQ ID NO: 1 and SEQ ID NO: 2.

[Figure 6B] Figure 6B shows the sequences of SEQ ID NO: 3 and SEQ ID NO: 4.

[Figure 6C] Figure 6C shows the sequences of SEQ ID NO: 5 and SEQ ID NO: 6.

[Figure 6D] Figure 6D shows the sequences of SEQ ID NO: 7 to SEQ ID NO: 11.

[Figure 6E] Figure 6E shows the sequences of SEQ ID NO: 12 to SEQ ID NO: 14.

[Figure 7] Figure 7 shows the range of the optimum pH of PenOX2.

[Figure 8] Figure 8 shows the range of the optimum temperature of PenOX2.

[Figure 9] Figure 9 shows the range of the heat stability of PenOX2.

[Figure 10] Figure 10 shows the range of the stable pH of PenOX2.

[Figure 11] Figure 11 shows the Km value of PenOX2 for pentosidine.

[Figure 12] Figure 12 shows the molecular weight of PenOX2.

[Figure 13] Figure 13 shows the substrate specificity of amino acid degrading enzyme 1 measured in Example 13.

[Figure 14] Figure 14 shows the substrate specificity of amino acid degrading enzyme 2 measured in Example 14.

[Figure 15] Figure 15 shows the substrate specificity of pentosidine oxidase measured in Example 15.

Description of Embodiments

Hereinafter, the method for measuring pentosidine, etc. according to one aspect of the present invention (hereinafter, also referred to as the "present embodiment") will be described in detail. However, the technical scope of the present invention is not limited by the items of the present embodiment. The present invention can assume various forms as long as the object of the present invention is attained.

(Protein having activity that oxidatively degrades pentosidine)

In one aspect, the present embodiment relates to a method for measuring pentosidine in a specimen.

Pentosidine has a structure where pentose, and equimolar lysine and arginine are cross-linked, as described above. The "protein having activity that oxidatively degrades pentosidine" for use in the measurement method of the present embodiment is not limited as long as the protein has such degrading activity. The protein includes pentosidine oxidase having pentosidine oxidase activity and pentosidine dehydrogenase having pentosidine dehydrogenase activity.

The protein having activity that oxidatively degrades pentosidine described in Examples mentioned later is a novel enzyme and has at least pentosidine oxidase activity. As used herein, the "pentosidine oxidase activity" means activity that oxidatively degrades pentosidine, more specifically, activity that oxidizes pentosidine to produce its deamination product, hydrogen peroxide, and ammonia, or activity that consumes oxygen.

As used herein, the "pentosidine dehydrogenase activity" means activity that oxidatively degrades pentosidine, more specifically, activity that oxidizes pentosidine to produce its deamination product, a reduced coenzyme, and ammonia, or activity that consumes an oxidized coenzyme. In this context, examples of the coenzyme include flavin adenine dinucleotide (FAD), flavin mononucleotide (FMN), nicotinamide adenine dinucleotide (NAD), and nicotinamide adenine dinucleotide phosphate (NADP). The reduced coenzyme may further reduce a mediator. The mediator is not particularly limited as long as the mediator can transfer or accept electrons to or from a coenzyme contained in the pentosidine dehydrogenase of the present invention. Examples of the mediator include, but are not limited to, quinones, phenazines, ferricyanides, osmium salts or complexes, ruthenium salts or complexes, nitrosoanilines, aminoanilines, viologens, cytochromes, phenoxazines, phenothiazines, ferredoxins, ferrocenes, and derivatives thereof. Examples of the quinones include, but are not limited to, naphthoquinone and derivatives thereof (e.g., naphthoquinone-4-sulfonate), phenanthrolinequinone and derivatives thereof, and phenanthrenequinone and derivatives thereof. Examples of the phenazines include, but are not limited to, phenazine methosulfate (PMS) and derivatives thereof (e.g., 1-methoxy PMS and 1-ethoxy PMS). Examples of the ferricyanides include, but are not limited to, potassium ferricyanide. Examples of the osmium salts or complexes include, but are not limited to, osmium chloride and hexaammineosmium. Examples of the ruthenium salts or complexes include, but are not limited to, ruthenium chloride and hexaammineruthenium. Examples of the nitrosoanilines include, but are not limited to, N,N-dimethyl-4-nitrosoaniline and N,N-bis-hydroxyethyl-4-nitrosoaniline and their derivatives. Other examples of the mediator also include mediators known to those skilled in the art. In the specification of the present application, the term "mediator" includes neither oxygen nor hydrogen peroxide unless otherwise specified.

It should be understood that every protein and a gene encoding the protein are included, without being limited by a particular sequence, in the scope of the present embodiment as long as the protein has the enzymatic activity as described above. The nucleotide sequence and the amino acid sequence of the pentosidine oxidase as the protein having activity that oxidatively degrades pentosidine will be described below by taking an enzyme derived from a filamentous fungus of the genus *Sarocladium* as an example.

(Amino acid sequence of pentosidine oxidase)

The pentosidine oxidase is not particularly limited by its amino acid sequence as long as the pentosidine oxidase has the enzymatic activity described above. Examples of one form of the enzyme having the pentosidine oxidase activity described above include a protein having the amino acid sequence represented by SEQ ID NO: 2 or SEQ ID NO: 4. Hereinafter, the proteins having the amino acid sequences represented by SEQ ID NO: 2 and SEQ ID NO: 4 are also referred to as pentosidine oxidase 1 (or PenOX1) and pentosidine oxidase 2 (or PenOX2), respectively. A gene (g4462) encoding the pentosidine oxidase 1 is presumably constituted by six exons and five introns, whereas a gene (g10122) encoding the pentosidine oxidase 2 is presumably constituted by two exons and one intron.

The pentosidine oxidase having the amino acid sequence represented by SEQ ID NO: 2 or SEQ ID NO: 4 is derived from a filamentous fungus of the genus *Sarocladium*. The nucleotide sequences of the genes encoding these enzymes are the nucleotide sequences represented by SEQ ID NO: 1 and SEQ ID NO: 3, respectively. Figure 6 shows the amino acid sequences and the nucleotide sequences of the enzymes.

The amino acid sequence of the pentosidine oxidase may consist of an amino acid sequence derived from the amino acid sequence of the wild-type enzyme, such as SEQ ID NO: 2 or SEQ ID NO: 4, by the deletion, substitution, addition, or the like of one or more amino acids, for example, 1, 2, 3, 4, 5, 6, 7, 8, 9, 10, 11, 12, 13, 14, 15, 16, 17, 18, 19, 20, 21, 22, 23, 24 or 25, preferably several amino acids, per unit (one unit involves 100 amino acids in the amino acid sequence) as long as the resulting pentosidine oxidase has the enzymatic activity of the pentosidine oxidase described above. In this context, the range of "one to several" in the "deletion, substitution, or addition of one to several amino acids" in the amino acid sequence is not particularly limited and means preferably approximately 1, 2, 3, 4, 5, 6, 7, 8, 9 or 10, more preferably approximately 1, 2, 3, 4 or 5, per unit described above. The "deletion of an amino acid" means the elimination or disappearance of an amino acid residue in a sequence. The "substitution of an amino acid" means the replacement of an amino acid residue in a sequence with another amino acid residue. The "addition of an amino acid" means the insertion of an additional amino acid residue into a sequence.

A specific form of the "deletion, substitution, or addition of an amino acid" is a form in which an amino acid is replaced with another amino acid chemically similar thereto to an extent that the pentosidine oxidase activity is maintained. Examples thereof can include the case of substituting a hydrophobic amino acid with another hydrophobic amino acid and the case of substituting a polar amino acid with another polar amino acid having the same charge thereas. Such chemically similar amino acids are known in the art on an amino acid basis.

Specifically, examples of the nonpolar (hydrophobic) amino acid include alanine, valine, isoleucine, leucine, proline, tryptophan, phenylalanine, and methionine. Examples of the polar (neutral) amino acid include glycine, serine, threonine, tyrosine, glutamine, asparagine, and cysteine. Examples of the basic amino acid having positive charge include arginine, histidine, and lysine. Examples of the acidic amino acid having negative charge include aspartic acid and glutamic acid.

Examples of the amino acid sequence of the pentosidine oxidase also include an amino acid sequence having sequence identity above a certain level to the amino acid sequence of the wild-type enzyme, such as SEQ ID NO: 2 or SEQ ID NO: 4, and include an amino acid sequence having 75% or higher, preferably 80% or higher, more preferably 85% or higher, more preferably 90% or higher, most preferably 95% or higher identity, to the amino acid sequence of the pentosidine oxidase.

(Gene encoding pentosidine oxidase)

The gene encoding the pentosidine oxidase (hereinafter, also referred to as the "pentosidine oxidase gene") is not particularly limited as long as the gene comprises a nucleotide sequence encoding the amino acid sequence of the enzyme having the pentosidine oxidase activity described above. In some aspects, the pentosidine oxidase gene is expressed in a transformant which thereby produces pentosidine oxidase.

As used herein, the "expression of a gene" means that an enzyme encoded by the gene is produced in a form having original catalytic activity via transcription, translation, etc. The "expression of a gene" also encompasses the high expression of the gene, i.e., the production of an enzyme encoded by the gene in an amount exceeding an original expression level from a host organism by the insertion of the gene.

The pentosidine oxidase gene may be a gene capable of producing the pentosidine oxidase via splicing after transcription of the gene or may be a gene capable of producing the pentosidine oxidase without the mediation of splicing after transcription of the gene, when transferred to a host organism.

The pentosidine oxidase gene may not be completely identical to a gene originally carried (i.e., a wild-type gene) by a source organism such as a filamentous fungus of the genus *Sarocladium,* and may be DNA having a nucleotide sequence that hybridizes under stringent conditions to a nucleotide sequence complementary to the nucleotide sequence of the wild-type gene as long as the gene encodes the enzyme having the pentosidine oxidase activity described above.

As used herein, the "nucleotide sequence that hybridizes under stringent conditions" means the nucleotide sequence of DNA that is obtained by use of colony hybridization, plaque hybridization, Southern blot hybridization, or the like using, as a probe, DNA corresponding to a portion of the nucleotide sequence of the wild-type gene, such as SEQ ID NO: 1 or SEQ ID NO: 3.

As used herein, the "stringent conditions" are conditions under which the signal of a specific hybrid is clearly discriminated from the signal of a nonspecific hybrid, and differ depending on the hybridization system used and the type, sequence and length of the probe. Such conditions can be determined by changing a hybridization temperature or changing a washing temperature and a salt concentration.

For example, if the signal of a nonspecific hybrid is strongly detected, the specificity can be enhanced by elevating the hybridization and washing temperatures while lowering, if necessary, the salt concentration for washing. If even the signal of a specific hybrid is not detected, the hybrid can be stabilized by lowering the hybridization and washing temperatures while elevating, if necessary, the salt concentration for washing.

In some aspects, specific examples of the stringent conditions include the following: a DNA probe is used as the probe, and the hybridization is performed overnight (approximately 8 to 16 hours) using 5 × SSC, 1.0% (w/v) blocking reagent for nucleic acid hybridization (manufactured by Boehringer Mannheim GmbH), 0.1% (w/v) N-lauroylsarcosine, and 0.02% (w/v) SDS. The washing is performed twice for 15 minutes each using 0.1 to 0.5 × SSC and 0.1% (w/v) SDS, preferably 0.1 × SSC and 0.1% (w/v) SDS. The temperatures at which the hybridization and the washing are performed are 65°C or higher, preferably 68°C or higher.

Examples of the DNA having a nucleotide sequence that hybridizes under stringent conditions can include DNA having the nucleotide sequence of the wild-type gene derived from a colony or a plaque, DNA that is obtained by hybridization under the stringent conditions described above using a filter on which a fragment of the DNA is immobilized, and DNA that can be identified by carrying out hybridization at 40 to 75°C in the presence of 0.5 to 2.0 M NaCl, then carrying out hybridization at 65°C, preferably in the presence of 0.7 to 1.0 M NaCl, and then washing the filter under a condition of 65°C using a 0.1 to 1 × SSC solution (the 1 × SSC solution consists of 150 mM sodium chloride and 15 mM sodium citrate). Methods for probe preparation or hybridization can be carried out in accordance with methods described in Molecular Cloning: A laboratory Manual, 2nd-Ed., Cold Spring Harbor Laboratory, Cold Spring Harbor, NY., 1989, and Current Protocols in Molecular Biology, Supplement 1-38, John Wiley & Sons, 1987-1997 (hereinafter, these literatures are also referred to as "technical references").

Those skilled in the art can appropriately set conditions for obtaining the DNA having a nucleotide sequence that hybridizes under stringent conditions to a nucleotide sequence complementary to the nucleotide sequence of the wild-type gene, by taking into consideration such conditions such as salt concentrations of buffers and temperatures as well as other conditions such as a probe concentration, a probe length, and a reaction time.

Examples of the DNA comprising a nucleotide sequence that hybridizes under stringent conditions include DNA having sequence identity above a certain level to the nucleotide sequence of DNA having the nucleotide sequence of the wild-type gene for use as a probe, and include DNA having 75% or higher, preferably 80% or higher, more preferably 85% or higher, more preferably 90% or higher, further preferably 95% or higher identity, to the nucleotide sequence of the wild-type gene.

The nucleotide sequence that hybridizes under stringent conditions to a nucleotide sequence complementary to the nucleotide sequence of the wild-type gene includes, for example, a nucleotide sequence derived from the nucleotide sequence of the wild-type gene by the deletion, substitution, addition, or the like of one or more bases, for example, 1 to 125, 1 to 100, 1 to 75, 1 to 50, 1 to 30 or 1 to 20, preferably 1 to several, for example, 1, 2, 3, 4, 5, 6, 7, 8, 9 or 10 bases, per unit (one unit involves 500 bases in the nucleotide sequence).

In this context, the "deletion of a base" means the elimination or disappearance of a base in a sequence. The "substitution of a base" means the replacement of a base in a sequence with another base. The "addition of a base" means the insertion of an additional base.

An enzyme encoded by the nucleotide sequence that hybridizes under stringent conditions to a nucleotide sequence complementary to the nucleotide sequence of the wild-type gene has the probability of being an enzyme having an amino acid sequence derived from the amino acid sequence of the enzyme encoded by the nucleotide sequence of the wild-type gene, by the deletion, substitution, addition, or the like of one or more, preferably several amino acids, but has the same enzymatic activity as that of the enzyme encoded by the nucleotide sequence of the wild-type gene.

The gene encoding the enzyme is a nucleotide sequence encoding an amino acid sequence identical or analogous to the amino acid sequence of the enzyme encoded by the wild-type gene, and may comprise a nucleotide sequence different from that of the wild-type gene, by exploiting several types of codons corresponding to one amino acid. Examples of such a codon-modified nucleotide sequence of the nucleotide sequence of the wild-type gene include the nucleotide sequence as set forth in SEQ ID NO: 5 (penox1) (codon-modified form of g4462) and SEQ ID NO: 6 (penox2) (codon-modified form of g10122) (Figure 6C). The codon-modified nucleotide sequence is preferably, for example, a nucleotide sequence that has undergone codon modification so as to facilitate expression in a host organism.

(Approach for calculating sequence identity)

The method for determining the sequence identity of a nucleotide sequence or an amino acid sequence is not particularly limited. The sequence identity is determined, for example, by using a program for aligning the wild-type gene or the amino acid sequence of the enzyme encoded by the wild-type gene with a targeted nucleotide sequence or amino acid sequence, and calculating the percent match between the sequences, usually by use of a known method.

For example, the algorithm of Karlin and Altschul (Proc. Natl. Acad. Sci. USA 87: 2264-2268, 1990; and Proc. Natl. Acad. Sci. USA 90: 5873-5877, 1993) is known as a program for calculating the percent match between two amino acid sequences or nucleotide sequences, and BLAST program using this algorithm has been developed by Altschul et al. (J. Mol. Biol. 215: 403-410, 1990). Further, gapped BLAST is also known as a program for determining sequence identity with higher sensitivity than that of BLAST (Nucleic Acids Res. 25: 3389-3402, 1997). Thus, those skilled in the art can utilize, for example, any of the programs described above to search a database for a sequence that exhibits high sequence identity to a given sequence. These are available in the internet website of the National Center for Biotechnology Information, U.S.
(http://blast.ncbi.nlm.nih.gov/Blast.cgi) .

Each of the methods described above can usually be used for searching a database for a sequence that exhibits sequence identity. Homology analysis of Genetyx network version 12.0.1 (manufactured by Genetyx Corp.) may be used as an approach of determining the sequence identity of an individual sequence. This method is based on the Lipman-Pearson method (Science 227: 1435-1441, 1985). A region (CDS or ORF) encoding a protein is used, if possible, in analyzing the sequence identity of a nucleotide sequence.

(Origin of gene encoding enzyme)

The gene encoding the enzyme is derived from an organism species having the ability to produce pentosidine oxidase. Examples of the source organism of the gene encoding the enzyme include microbes such as filamentous fungi. Specific examples of the microbe having the ability to produce pentosidine oxidase include the genus *Sarocladium.*

As described above, the source organism of the gene encoding the enzyme is not particularly limited, and an enzyme expressed in a transformant preferably exhibits activity without being inactivated under growing conditions of a host organism. Accordingly, the source organism of the gene encoding the enzyme is preferably a microbe similar in growing conditions to the host organism to be transformed by the insertion of the gene encoding the enzyme.

Examples of the distinctive physicochemical characteristics or properties of the enzyme having the pentosidine oxidase activity include the following:

- Molecular weight based on SDS-PAGE: 75,000 to 85,000
- Optimum pH: pH of approximately 6.5 to 8.0
  The optimum pH is pH at which the enzyme acts most suitably, and the pentosidine oxidase is also capable of acting at pH other than the range described above.
- Optimum temperature: Approximately 37 to 50°C
  The optimum temperature is a temperature at which the enzyme acts most suitably, and the pentosidine oxidase is also capable of acting at temperature other than the temperature range described above.
- Temperature stability: 90% or more of the pentosidine oxidase activity is maintained when the enzyme is preserved at 30°C for 10 minutes. 50% or more of the pentosidine oxidase activity is maintained when the enzyme is preserved at 40°C for 10 minutes.
- pH stability: 60% or more of the pentosidine oxidase activity is maintained in the range of pH 4.0 to 9.0.
- Km value: The Km value for pentosidine is 1 mM or less.

The Km value is a Michaelis constant. A specific calculation method therefor is not particularly limited, and the Km value can be calculated by an arbitrarily selected known method. The Km value can be calculated, for example, according to an Michaelis-Menten equation drawn by a method based on the Lineweaver-Burk plot, as in a method described in Example 9 mentioned later.

(Cloning of gene encoding enzyme by genetic engineering approach)

The gene encoding the enzyme can be inserted into any of appropriate various known vectors. Further, this vector can be transferred to an appropriate known host organism to prepare a transformant harboring a recombinant vector (recombinant DNA) containing the gene encoding the enzyme. Those skilled in the art can appropriately select a method for obtaining the gene encoding the enzyme, a method for obtaining information on the nucleotide sequence of the gene encoding the enzyme and the amino acid sequence of the enzyme, a method for producing various vectors, a method for preparing the transformant, etc. As used herein, the transformation and the transformant encompass transduction and a transductant, respectively. One nonlimiting example of the cloning of the gene encoding the enzyme will be mentioned later.

A usual gene cloning method generally used can be appropriately used for cloning the gene encoding the enzyme. For example, chromosomal DNA or mRNA can be extracted from a microbe or various cells having the ability to produce the enzyme by a routine method, for example, a method described in the technical references (supra). cDNA can be synthesized with the extracted mRNA as a template. A chromosomal DNA or cDNA library can be prepared using the chromosomal DNA or the cDNA thus obtained.

In some aspects, the gene encoding the enzyme can be obtained by cloning with chromosomal DNA or cDNA of a source organism having the gene as a template. Examples of the source organism of the gene encoding the enzyme can include, but are not particularly limited to, *Sarocladium* sp. described above. For example, *Sarocladium* sp. is cultured, and water is removed from the obtained fungus body, which is then physically ground into fungus body pieces in a fine powder form using a mortar or the like while cooled in liquid nitrogen. A chromosomal DNA fraction is extracted from the fungus body pieces by a usual method. A commercially available chromosomal DNA extraction kit such as DNeasy Plant Mini Kit (manufactured by Qiagen N.V.) can be used in chromosomal DNA extraction operation.

Subsequently, DNA is amplified by polymerase chain reaction (hereinafter, referred to as "PCR") using the chromosomal DNA as a template and primers complementary to a 5'-terminal sequence and a 3'-terminal sequence. The primers are not particularly limited as long as the primers are capable of amplifying a DNA fragment containing the gene. In another method, DNA containing a fragment of the gene of interest is amplified by appropriate PCR such as 5' RACE or 3' RACE and such DNAs can be linked to obtain DNA containing the full-length gene of interest.

The method for obtaining the gene encoding the enzyme is not particularly limited, and the gene encoding the enzyme can be constructed by use of, for example, a chemical synthesis method, instead of a genetic engineering approach.

The nucleotide sequence of the amplification product obtained by PCR amplification or the chemically synthesized gene can be confirmed, for example, as follows: DNA to be sequenced is inserted into an appropriate vector in accordance with a usual method to prepare recombinant DNA. A commercially available kit such as TA Cloning Kit (manufactured by Invitrogen Corp.); commercially available plasmid vector DNA such as pUC19 (manufactured by Takara Bio Inc.), pUC18 (manufactured by Takara Bio Inc.), pBR322 (manufactured by Takara Bio Inc.), pBluescript SK+ (manufactured by Stratagene California), or pYES2/CT (manufactured by Invitrogen Corp.); or commercially available bacteriophage vector DNA such as λEMBL3 (manufactured by Stratagene California) can be used in cloning into the vector. In some aspects, a host organism, for example, *Escherichia coli,* preferably *Escherichia coli* JM109 strain (manufactured by Takara Bio Inc.) or *Escherichia coli* DH5α strain (manufactured by Takara Bio Inc.), is transformed with the recombinant DNA. The recombinant DNA contained in the obtained transformant may be purified using QIAGEN Plasmid Mini Kit (manufactured by Qiagen N.V.) or the like.

The nucleotide sequence of each gene inserted in the recombinant DNA can be determined by a dideoxy method (Methods in Enzymology, 101, 20-78, 1983) or the like. Examples of the sequence analysis apparatus for use in determining the nucleotide sequence include, but are not particularly limited to, Li-COR MODEL 4200L sequencer (manufactured by Aloka Co., Ltd.), 370DNA sequencing system (manufactured by PerkinElmer, Inc.), and CEQ2000XL DNA analysis system (manufactured by Beckman Coulter Inc.). Then, the amino acid sequence of the protein to be obtained by translation, i.e., the enzyme, can be known on the basis of the determined nucleotide sequence.

(Construction of recombinant vector containing gene encoding enzyme)

The recombinant vector (recombinant DNA) containing the gene encoding the enzyme can be constructed by ligating a PCR amplification product containing any gene encoding the enzyme with any of various vectors in a form that permits expression of the gene encoding the enzyme. The recombinant vector can be constructed, for example, by excising a DNA fragment containing any gene encoding the enzyme with an appropriate restriction enzyme, and ligating the DNA fragment with a plasmid cleaved with the appropriate restriction enzyme. Alternatively, the recombinant vector can be obtained by ligating a DNA fragment containing the gene having both ends added to sequences homologous to a plasmid with a DNA fragment derived from the plasmid amplified by inverse PCR, using a commercially available recombinant vector preparation kit such as In-Fusion HD Cloning Kit (manufactured by Clontech Laboratories, Inc.) or the like.

(Method for preparing transformant)

Examples of the method for preparing the transformant include, but are not particularly limited to, a method of inserting the gene encoding the enzyme in an expressible form into a host organism according to a routine method. In some aspects, any gene encoding the enzyme is inserted to between an expression inducible promoter and a terminator to prepare a DNA construct. Subsequently, a host organism is transformed with the DNA construct containing the gene encoding the enzyme to obtain a transformant overexpressing the gene encoding the enzyme. In the present specification, a DNA fragment consisting of expression inducible promoter-gene encoding the enzyme-terminator prepared in order to transform a host organism, and a recombinant vector containing the DNA fragment are collectively referred to as a DNA construct.

Examples of the method for inserting the gene encoding the enzyme in an expressible form into a host organism include, but are not particularly limited to: an approach of directly inserting the gene onto a chromosome of the host organism through the use of homologous recombination or nonhomologous recombination; and an approach of ligating the gene with a plasmid vector, which is then transferred into the host organism.

In the method using homologous recombination, the DNA construct can be placed between and linked to sequences homologous to an upstream region and a downstream region of a recombination site on a chromosome, and thereby inserted into the genome of the host organism. In the method using nonhomologous recombination, the DNA construct can be inserted, even without being linked to the homologous sequences, into the genome of the host organism. Examples of the high-expression promoter include, but are not particularly limited to, a promoter region of translation elongation factor TEF1 gene (tef1), a promoter region of α-amylase gene (amy), a promoter region of alkaline protease gene (alp), and a promoter region of glyceraldehyde-3-phosphate dehydrogenase (gpd).

In the method using a vector, the DNA construct is integrated into a plasmid vector for use in the transformation of a host organism by a routine method, and the corresponding host organism can be transformed therewith by a routine method.

Such a suitable vector-host system is not particularly limited as long as the system is capable of producing the enzyme in the host organism. Examples thereof include a system of pUC19 and a filamentous fungus and a system of pSTA14 (Mol. Gen. Genet. 218, 99-104, 1989) and a filamentous fungus.

The DNA construct is preferably used by transfer into a chromosome of the host organism. In other methods, the DNA construct may be integrated into an autonomous replicating vector (Ozeki et al., Biosci. Biotechnol. Biochem. 59, 1133 (1995)) and thereby used without being transferred to a chromosome.

The DNA construct may contain a marker gene for rendering transformed cells selectable. Examples of the marker gene include, but are not particularly limited to: genes, such as pyrG, niaD, and adeA, which complement the auxotrophy of the host organism; and drug resistance genes against drugs such as pyrithiamine, hygromycin B, and oligomycin. Also, the DNA construct preferably contains a promoter that permits overexpression of the gene encoding the enzyme in the host organism, a terminator, and other control sequences (e.g., an enhancer and a polyadenylation sequence). Examples of the promoter include, but are not particularly limited to, appropriate expression inducible promoters and constitutive promoters, and include tef1 promoter, alp promoter, amy promoter, and gpd promoter. Examples of the terminator include, but are not particularly limited to, alp terminator, amy terminator, and tef1 terminator.

In the DNA construct, an expression control sequence of the gene encoding the enzyme is not necessarily required when the DNA fragment containing the gene encoding the enzyme for insertion contains a sequence having an expression control function. In the case of performing transformation by a cotransformation method, the DNA construct may not have a marker gene.

The DNA construct can be tagged for purification. For example, a linker sequence is appropriately connected to upstream or downstream of the gene encoding the enzyme, and six or more codons of a nucleotide sequence encoding histidine can be connected thereto so that purification using a nickel column is attained.

The DNA construct may contain a homologous sequence necessary for marker recycling. For example, pyrG marker can be eliminated in a medium containing 5-fluoroorotic acid (5FOA) by adding a sequence homologous to a sequence upstream of an insertion site (5' homologous recombination region) to downstream of the pyrG marker, or adding a sequence homologous to a sequence downstream of an insertion site (3' homologous recombination region) to upstream of the pyrG marker. The length of the homologous sequence suitable for marker recycling is preferably 0.5 kb or larger.

One form of the DNA construct is, for example, a DNA construct containing tef1 gene promoter, the gene encoding the enzyme, alp gene terminator and pyrG marker gene linked to an in-fusion cloning site present in the multicloning site of pUC19.

In the case of inserting the gene by homologous recombination, one aspect of the DNA construct is a DNA construct containing a 5' homologous recombination sequence, tef1 gene promoter, the gene encoding the enzyme, alp gene terminator and pyrG marker gene, and a 3' homologous recombination sequence linked to each other.

In the case of inserting the gene by homologous recombination and recycling a marker, one form of the DNA construct is a DNA construct containing a 5' homologous recombination sequence, tef1 gene promoter, the gene encoding the enzyme, alp gene terminator, a homologous sequence for marker recycling, pyrG marker gene, and a 3' homologous recombination sequence linked to each other.

When the host organism is a filamentous fungus, a method known to those skilled in the art can be appropriately selected as a method for transforming the filamentous fungus. For example, a protoplast PEG method of preparing a protoplast of the host organism and then using polyethylene glycol and calcium chloride (see e.g., Mol. Gen. Genet. 218, 99-104, 1989 (supra); and Japanese Patent Laid-Open No. 2007-222055) can be used. An appropriate medium for regenerating the transformant is used according to the host organism used and the transformation marker gene. In the case of using, for example, A. *oryzae* or A. *sojae* as the host organism and pyrG gene as the transformation marker gene, the transformant can be regenerated in, for example, Czapek-Dox minimum medium (manufactured by Difco Laboratories Ltd.) containing 0.5% agar and 1.2 M sorbitol.

In order to obtain the transformant, for example, the promoter of the gene encoding the enzyme, originally carried by a chromosome of the host organism may be substituted by a high-expression promoter such as tef1 through the use of homologous recombination. In this respect, it is also preferred to insert a transformation marker gene such as pyrG, in addition to the high-expression promoter. For example, a cassette for transformation consisting of upstream region of the gene encoding the enzyme-transformation marker gene-high-expression promoter-whole or partial gene encoding the enzyme can be used for this purpose with reference to Examples described in Japanese Patent Laid-Open No. 2011-239681. In this case, the upstream region of the gene encoding the enzyme and the whole or partial gene encoding the enzyme are used for homologous recombination.

The whole or partial gene encoding the enzyme used can contain a sequence from a start codon to a midstream region. The length of the region suitable for homologous recombination is preferably 0.5 kb or larger.

The prepared transformant can be confirmed by culturing the transformant under conditions under which the enzymatic activity of the enzyme is observed, and subsequently detecting the product of interest in the cultures thus obtained by culture.

Alternatively, the prepared transformant may be confirmed by extracting chromosomal DNA from the transformant, and performing PCR with this chromosomal DNA as a template to confirm that a PCR product amplifiable by transformation is formed. In this case, the formation of a product having an expected length is confirmed by PCR using, for example, a forward primer against the nucleotide sequence of the promoter used and a reverse primer against the nucleotide sequence of the transformation marker gene in combination.

In the case of performing transformation by homologous recombination, it is preferred that the formation of a product having a length expected from homologous recombination be confirmed by PCR using a forward primer located upstream of the upstream homologous region used and a reverse primer located downstream of the downstream homologous region used in combination.

(Host organism)

The host organism is not particularly limited as long as the organism can produce the enzyme by transformation with a DNA construct containing the gene encoding the enzyme. Examples thereof include microbes and plants. Examples of the microbe include microbes of the genus *Aspergillus,* microbes of the genus *Escherichia,* microbes of the genus *Saccharomyces,* microbes of the genus *Pichia,* microbes of the genus *Schizosaccharomyces,* microbes of the genus *Zygosaccharomyces,* microbes of the genus *Trichoderma,* microbes of the genus *Penicillium,* microbes of the genus *Rhizopus,* microbes of the genus *Neurospora,* microbes of the genus *Mucor,* microbes of the genus *Acremonium,* microbes of the genus *Fusarium,* microbes of the genus *Neosartorya,* microbes of the genus *Byssochlamys,* microbes of the genus *Talaromyces,* microbes of the genus *Ajellomyces,* microbes of the genus *Paracoccidioides,* microbes of the genus *Uncinocarpus,* microbes of the genus *Coccidioides,* microbes of the genus *Arthroderma,* microbes of the genus *Trichophyton,* microbes of the genus *Exophiala,* microbes of the genus *Capronia,* microbes of the genus *Cladophialophora,* microbes of the genus *Macrophomina,* microbes of the genus *Leptosphaeria,* microbes of the genus *Bipolaris,* microbes of the genus *Dothistroma,* microbes of the genus *Pyrenophora,* microbes of the genus *Neofusicoccum,* microbes of the genus *Setosphaeria,* microbes of the genus *Baudoinia,* microbes of the genus *Gaeumannomyces,* microbes of the genus *Marssonina,* microbes of the genus *Sphaerulina,* microbes of the genus *Sclerotinia,* microbes of the genus *Magnaporthe,* microbes of the genus *Verticillium,* microbes of the genus *Pseudocercospora,* microbes of the genus *Colletotrichum,* microbes of the genus *Ophiostoma,* microbes of the genus *Metarhizium,* microbes of the genus *Sporothrix,* microbes of the genus *Sordaria,* and plants of the genus *Arabidopsis,* and microbes and plants are preferred. However, human is excluded from the host organism in every case.

Among the filamentous fungi, for example, microbes of the genus *Aspergillus* such as A. *oryzae, A. sojae, A. niger, A. tamarii, A. awamori, A. usamii, A. kawachii,* and A. *saitoi* are preferred in light of safety and easy culture.

In the present embodiment, the expression of the protein is not limited to embodiments using the host organism as described above. For example, an *in vitro* cell-free protein expression system can be suitably used, particularly, when large-scale production such as production in a commercial scale is not intended. The cell-free protein expression system does not require cell culture and also has the advantage that the protein can also be conveniently purified. In the cell-free protein expression system, a reaction liquid containing a gene corresponding to the desired protein and molecular mechanisms of transcription and translation such as a cell lysate is used.

(Specific example of gene encoding enzyme)

Examples of the gene encoding the enzyme derived from the genus *Sarocladium* include genes g4462 and g10122 having the nucleotide sequences as set forth in SEQ ID NOs: 1 and 3, respectively. The amino acid sequences of the pentosidine oxidase 1 protein (PenOX1) and the pentosidine oxidase 2 protein (PenOX2) are shown in SEQ ID NO: 2 and SEQ ID NO: 4, respectively.

The method for obtaining the gene encoding the enzyme from the genus *Sarocladium* or an organism other than the genus *Sarocladium* is not particularly limited. The gene can be obtained, for example, by searching the genomic DNA of the target organism by BLAST homology search on the basis of the nucleotide sequences of the genes g4462 and g10122 (SEQ ID NO: 1 and SEQ ID NO: 3), and identifying genes having nucleotide sequences having high sequence identity to the nucleotide sequences of the genes g4462 and g10122. Also, the gene can be obtained by identifying proteins having amino acid sequences having high sequence identity to the amino acid sequences of the pentosidine oxidase 1 and pentosidine oxidase 2 proteins (SEQ ID NO: 2 and SEQ ID NO: 4) on the basis of the total protein of the target organism, and identifying genes encoding the proteins.

The gene encoding the enzyme obtained from the genus *Sarocladium* or the gene encoding an enzyme having sequence identity to the enzyme can be transferred to arbitrary host cells of a host organism such as a microbe of the genus *Aspergillus* for transformation.

(Transformant)

One form of the transformant is a transformant having an insert of any one of the genes or a combination thereof in a host organism such as a microbe or a plant transformed so as to express the inserted gene.

Another form of the transformant is a transformant having an insert of a DNA construct designed to highly or low express a gene (also containing a promoter sequence, etc. in addition to the ORF) containing the whole or a portion of the gene g4462 or g10122, and a transcriptional factor that controls the transcription of the gene, in a host organism such as a microbe or a plant transformed so as to express the inserted gene.

When the host organism is an organism found to have the ability to produce pentosidine oxidase, such as the genus *Sarocladium,* it is desirable that the inserted gene should be forced to be constitutively expressed or more highly expressed than endogenous expression, or should be conditionally expressed at the late stage of culture after cell proliferation. Such a transformant is cultured or grown under conditions suitable for the host organism or the transformant and can thereby produce pentosidine oxidase that is not produced in the host organism or a more detectable level of pentosidine oxidase than that produced in the host organism, through the action of the transcriptional factor having a changed expression level.

The pentosidine oxidase can be produced by culturing the transformant described above under culture conditions suitable for the growth of the transformant using a medium suitable for the growth of the transformant. The culture method is not particularly limited. When the host organism is, for example, a filamentous fungus, examples thereof include a solid culture method and a liquid culture method which are performed under draft or non-draft conditions. Hereinafter, a production method using a filamentous fungus as a host organism or a wild-type organism will be mainly described. However, the present embodiment is not limited by the following description.

Any synthetic medium or natural medium can be used as long as the medium is a usual medium for the culture of a host organism or a wild-type organism (hereinafter, these organisms are also collectively referred to as a "host organism, etc."), i.e., contains a carbon source, a nitrogen source, an inorganic material, and other nutrients at an appropriate ratio. When the host organism, etc. is a microbe of the genus *Aspergillus,* YMG medium, PPY medium, or the like as described in Examples mentioned later can be used, though the medium is not particularly limited thereto.

Usual culture conditions for the host organism, etc. known to those skilled in the art can be adopted as culture conditions for the transformant. When the host organism, etc. is, for example, a filamentous fungus, the initial pH of the medium is adjusted to 5 to 10, and the culture temperature and the culture time can be appropriately set to 20 to 40°C and, for example, several hours to several days, preferably 1 to 7 days, more preferably 2 to 4 days, respectively. The culture approach is not particularly limited, and aeration-stirring submerged culture, shake culture, static culture, or the like can be adopted. The culture is preferably performed under conditions that attain sufficient dissolved air. For example, in the case of culturing a microbe of the genus *Aspergillus,* one example of the medium and the culture conditions includes shake culture at 160 rpm at 30°C for 3 to 5 days using YMG medium or PPY medium described in Examples mentioned later.

The method for extracting pentosidine oxidase from the cultures after the completion of culture is not particularly limited. For the extraction, a fungus body recovered by operation such as filtration or centrifugation from the cultures may be used as it is, or a fungus body dried after recovery or a further pulverized fungus body may be used. Examples of the method for drying the fungus body include, but are not particularly limited to, freeze drying, solar drying, hot-air drying, vacuum drying, through-flow drying, and drying under reduced pressure.

Instead of the treatments described above, fungus body disruption treatment may be adopted, for example, a method of destroying the fungus body using a destruction approach such as a sonicator, a French press, Dyno-Mill, or a mortar; a method of lysing the cell wall of the fungus body using a cell wall lytic enzyme such as yatalase; or a method of lysing the fungus body using a surfactant such as SDS or Triton X-100. These methods can be used singly or in combinations.

The obtained extracts can be subjected to a purification procedure such as centrifugation, filtration through a filter, ultrafiltration, gel filtration, separation based on difference in solubility, solvent extraction, chromatography (adsorption chromatography, hydrophobic chromatography, cation-exchange chromatography, anion-exchange chromatography, reverse-phase chromatography, etc.), crystallization, activated carbon treatment, or membrane treatment to purify the product of interest.

(Substrate specificity)

The protein having activity that oxidatively degrades pentosidine according to the present embodiment has high degrading activity (substrate specificity) against pentosidine and may have degrading activity against other amino acids. In one aspect, the relative activity of the protein having activity that oxidatively degrades pentosidine according to the present embodiment against one or more, two or more, three or more, four or more or five or more amino acids selected from arginine, leucine, methionine, phenylalanine, tryptophan and tyrosine, or all of these amino acids is 40% or higher, 50% or higher or 60% or higher when the activity against pentosidine is defined as 100%. In one aspect, the activity of the protein having activity that oxidatively degrades pentosidine according to the present embodiment against one or more or two or more amino acids selected from asparagine, glutamine and histidine, or all of these amino acids has 10% or higher or 20% or higher relative activity when the activity against pentosidine is defined as 100%.

The methods for measuring the relative activity and the substrate specificity can be carried out by use of an approach known to those skilled in the art using the same or similar approaches and conditions as in measurement for pentosidine. They can be measured, for example, using a reaction rate with reference to an approach described in Examples mentioned later.

(Measurement method)

The method for measuring pentosidine according to the present embodiment comprises the steps of:

degrading a specimen with an amino acid degrading enzyme;
contacting the specimen after the degradation step with a protein having activity that oxidatively degrades pentosidine; and
detecting change resulting from the contact.

As used herein, examples of the "specimen" include test subjects, for example, pentosidine solutions containing pentosidine to be quantified; and liquid components and solid components derived from organisms, such as blood, blood components (serum, plasma, blood cells, etc.), body fluids, and excrements. In one aspect, the specimen is derived from a subject having or suspected of having a disease associated with pentosidine. The specimen is preferably blood or a blood component, particularly preferably plasma. The specimen may not always contain pentosidine. If the specimen contains no pentosidine, the measurement method according to the present embodiment can be used in analysis on the presence or absence of contained pentosidine (qualitative analysis). When the specimen is derived from an organism, a specimen derived from an arbitrary organism such as a human, a mouse, a rat, or a monkey can be used. The specimen collected from the organism may be used as it is or may be used after arbitrary treatment.

(Amino acid degrading enzyme)

The measurement method of the present embodiment comprises the step of degrading the specimen with an amino acid degrading enzyme. The degradation with the amino acid degrading enzyme prior to contact with a protein having activity that oxidatively degrades pentosidine decreases measurement errors ascribable to the reaction of the protein having activity that oxidatively degrades pentosidine with another amino acid and allows more accurate measurement of pentosidine.

The amino acid degrading enzyme is an enzyme different from the protein having activity that oxidatively degrades pentosidine and is an enzyme that can preferentially degrade an amino acid other than an amino acid of pentosidine. Examples of the amino acid degrading enzyme include amino acid oxidase, amino acid dehydrogenase, amino acid aminotransferase, amino acid decarboxylase, amino acid ammonia lyase, amino acid oxygenase (hydroxylase), and amino acid hydrolase. An arbitrary enzyme can be used taking its substrate specificity into consideration. These amino acid degrading enzymes may be used singly or as a mixture or in combination of two or more thereof.

The amino acid degrading enzyme is not particularly limited, and a known enzyme can be used. A commercially available product may be used. For example, a reagent of a commercially available amino acid quantification kit may be used as the amino acid degrading enzyme. The production method therefor is not limited. For example, a transformant harboring a gene encoding the amino acid degrading enzyme (as one example, a gene encoding escapin (SEQ ID NO: 15: the amino acid sequence of a mature peptide of escapin derived from *Aplysia californica*) described in Example 11 mentioned later) is prepared by use of the same or similar approach as mentioned above about the production of pentosidine oxidase, and this transformation is cultured. The amino acid degrading enzyme may be obtained from the resulting medium.

For example, amino acid degrading enzymes shown in the following table can be used singly or in combinations taking substrate specificity into consideration.

[Table 1]

| **Large classification** | **Middle classification** | **Small classification** | **Manufacturer** | **Model** |
|---|---|---|---|---|
| Amino acid oxidase | L-Amino acid oxidase | *Crotalus atorx*-derived L-amino acid oxidase, Type I | Merck | A5147 |
| | | *Crotalus* adamanteus-derived L-amino acid oxidase, Type I | Merck | A9253 |
| | | *Crotalus* adamanteus-derived L-amino acid oxidase, Type IV | Merck | A9378 |
| | | *Aplysia* californica-derived amino acid oxidase, (Escapin) | - | - |
| | | *Trichoderma* viride-derived lysine oxidase | Merck | L6150 |
| Amino acid dehydrogenase | Glutamate dehydrogenase | Microbe-derived glutamate dehydrogenase | Toyobo Co., Ltd. | GTD-211 |
| | | *Bacillus* subtilis-derived glutamate dehydrogenase | - | - |
| | Leucine dehydrogenase | *Bacillus stearothermophilus-derived* leucine dehydrogenase (LeuDH) | NIPRO ENZYMES | - |
| | | *Bacillus* sp.-derived leucine dehydrogenase | Toyobo Co., Ltd. | LED-201 |
| | Alanine dehydrogenase | *Bacillus stearothermophilus-derived* alanine dehydrogenase (AlaDH) | NIPRO ENZYMES | - |
| | | *Streptomyces* griseus-derived alanine dehydrogenase | - | - |
| Amino acid aminotransferase | Alanine aminotransferase | Human liver-derived alanine aminotransferase (ALT/GPT) | LEE | 310-19 |
| | | *Oryza sativa*-derived alanine aminotransferase | - | - |
| | Aspartate aminotransferase | Human liver-derived aspartate aminotransferase (AST/GOT) | LEE | 306-10 |
| | | Swine heart-derived aspartate aminotransferase (AST/GOT) | LEE | 300-20 |

(continued)

| Large classification | Middle classification | Small classification | Manufacturer | Model |
|---|---|---|---|---|
| Amino acid decarboxylase | Glutamate decarboxylase | *Lactobacillus brevis* IFO 12005-derived glutamate decarboxylase | - | - |
| | | *Escherichia* coli-derived glutamate decarboxylase | - | - |
| | | *Solanum lycopersicum*-derived glutamate decarboxylase | - | - |
| | Aspartate decarboxylase | *Tetragenococcus halophilus*-derived aspartate decarboxylase | - | - |
| | | *Corynebacterium glutamicum*-derived aspartate decarboxylase | - | - |
| | Arginine decarboxylase | *Lactococcus lactis*-derived arginine decarboxylase | - | - |
| | | *Lactobacillus* sake-derived arginine decarboxylase | - | - |
| | | *Escherichia* coli-derived arginine decarboxylase | - | - |
| | Lysine decarboxylase | *Escherichia* coli-derived lysine decarboxylase | - | - |
| | | *Streptomyces* coelicolor-derived lysine decarboxylase | - | - |
| | Histidine decarboxylase | *Lactobacillus* buchneri-derived histidine decarboxylase | - | - |
| | | *Lactobacillus* 30a-derived histidine decarboxylase | - | - |
| | Phenylalanine decarboxylase | *Lactobacillus* buchneri-derived phenylalanine decarboxylase | - | - |
| | | *Pseudomonas* putida-derived phenylalanine decarboxylase | - | - |
| | Tyrosine decarboxylase | *Lactobacillus* buchneri-derived tyrosine decarboxylase | - | - |
| | | *Nicotiana* tabacum-derived tyrosine decarboxylase | - | - |
| | Tryptophan decarboxylase | *Oryza sativa*-derived tryptophan decarboxylase | - | - |
| | | *Nicotiana* tabacum-derived tryptophan decarboxylase | - | - |
| Amino acid ammonia lyase | Histidine ammonia lyase | *Pseudomonas* putida-derived histidine ammonia lyase | - | - |
| | | *Streptomyces* griseus-derived histidine ammonia lyase | - | - |

(continued)

| Large classification | Middle classification | Small classification | Manufacturer | Model |
|---|---|---|---|---|
| | Phenylalanine ammonia lyase | *Rhodotorula* rubra-derived phenylalanine ammonia lyase | - | - |
| | | *Rhodobacter capsulatus*-derived phenylalanine ammonia lyase | - | - |
| | Tyrosine ammonia lyase | *Rhodotorula glutinis*-derived tyrosine ammonia lyase | - | - |
| | | *Saccharothrix* espanaensis-derived tyrosine ammonia lyase | - | - |
| Amino acid oxygenase (hydroxylase) | Tryptophan dioxygenase | *Bacillus* megaterium-derived tryptophan dioxygenase | - | - |
| | Asparagine hydroxylase | *Streptomyces coelicolor* A3(2)-derived asparagine hydroxylase | - | - |
| Amino acid hydrolase | Arginase | *Saccharomyces cerevisiae*-derived arginase | - | - |
| | | *Solanum lycopersicum*-derived arginase | - | - |
| | Glutaminase | *Aspergillus* oryzae-derived glutaminase | - | - |
| | | *Micrococcus luteus*-derived glutaminase | - | - |
| | Asparaginase | *Aspergillus oryzae*-derived asparaginase | - | - |
| | | *Escherichia* coli-derived asparaginase | - | - |

The amino acid degrading enzyme is an enzyme capable of degrading the desired amino acid under conditions unreactive with pentosidine. In one aspect, the relative activity of the amino acid degrading enzyme against pentosidine is 30% or lower, preferably 20% or lower, more preferably 10% or lower, further preferably 5% or lower, when the activity against an amino acid against which the highest activity is exerted (which is most degraded) is defined as 100% under the same conditions thereas.

The amino acid degrading enzyme can be selected taking into consideration the type of an amino acid presumably contained in the specimen to be measured, or the substrate specificity of the protein having activity that oxidatively degrades pentosidine for use in measurement.

In one aspect, an amino acid degrading enzyme that degrades an amino acid against which the protein having activity that oxidatively degrades pentosidine has 5% or higher, 10% or higher, 20% or higher, 40% or higher or 60% or higher relative activity can be used, when the activity of the protein having activity that oxidatively degrades pentosidine against pentosidine is defined as 100%.

In one aspect, when the protein having activity that oxidatively degrades pentosidine is pentosidine oxidase (preferably pentosidine oxidase having the amino acid sequence of SEQ ID NO: 2 or SEQ ID NO: 4 or a variant thereof, more preferably pentosidine oxidase having the amino acid sequence of SEQ ID NO: 4 or a variant thereof), an amino acid degrading enzyme that degrades one or more, two or more, three or more, four or more or five or more amino acids selected from arginine, leucine, methionine, phenylalanine, tryptophan and tyrosine, or preferably all of these amino acids, can be used. For example, any of the following amino acid degrading enzymes can be used as such an amino acid degrading enzyme.

- An amino acid degrading enzyme having 30% or lower, preferably 20% or lower, more preferably 10% or lower, further preferably 5% or lower relative activity against pentosidine when the activity against one or more amino acids selected from arginine, leucine, methionine, phenylalanine, tryptophan and tyrosine is defined as 100% under the same conditions thereas.
- A combination of amino acid degrading enzymes, the combination having 30% or lower, preferably 20% or lower, more preferably 10% or lower, further preferably 5% or lower relative activity against pentosidine when the activity of the combined enzymes against arbitrary amino acid(s) selected from arginine, leucine, methionine, phenylalanine, tryptophan and tyrosine is defined as 100% under the same conditions thereas.

- A combination of escapin or a variant thereof that exhibits substrate specificity similar thereto, and *Crotalus* adamanteus-derived L-amino acid oxidase or a variant thereof that exhibits substrate specificity similar thereto.
- A combination of escapin or a variant thereof that exhibits substrate specificity similar thereto, *Crotalus* adamanteus-derived L-amino acid oxidase or a variant thereof that exhibits substrate specificity similar thereto, and further, one or more, two or more, three or more, four or more or all enzymes selected from histidine decarboxylase, asparaginase, aspartic acid decarboxylase, glutaminase and glutamic acid decarboxylase.

In one aspect, when the protein having activity that oxidatively degrades pentosidine is pentosidine oxidase (preferably pentosidine oxidase having the amino acid sequence of SEQ ID NO: 2 or SEQ ID NO: 4 or a variant thereof, more preferably pentosidine oxidase having the amino acid sequence of SEQ ID NO: 4 or a variant thereof), an amino acid degrading enzyme that degrades one or more or two or more amino acids selected from asparagine, glutamine and histidine, or all of these amino acids, in addition to arginine, leucine, methionine, phenylalanine, tryptophan and tyrosine, can be used.

The conditions under which the specimen is degraded with the amino acid degrading enzyme are not particularly limited as long as under the conditions, the amino acid degrading enzyme can degrade the desired amino acid and does not react with pentosidine. The conditions can be appropriately set according to the amino acid degrading enzyme used.

In the case of using, for example, amino acid oxidase as the amino acid degrading enzyme, its amount can be appropriately selected depending on the amount of the amino acid contained in the specimen, reaction conditions, etc., and is 0.001 to 50 U/ml, preferably 0.01 to 10 U/ml. The pH can be adjusted to, for example, pH 3 to 12, preferably pH 4 to 11, taking into consideration the range that allows the amino acid oxidase used to act. An arbitrary known pH adjuster and buffer solution can be used according to the specimen and the pH to be adjusted. For example, 15 to 65°C, preferably 20 to 60°C, can be adopted as a reaction temperature taking into consideration the optimum temperature range of the amino acid oxidase used. The reaction time can be a time sufficient for degrading the desired amino acid, and the reaction can be performed for, for example, 1 to 120 minutes, preferably 2 to 60 minutes.

After the degradation step, the specimen thus degraded with the amino acid degrading enzyme is contacted, either as it is or after appropriately undergoing, if necessary, a step such as heating, centrifugation, concentration, or dilution, with the protein having activity that oxidatively degrades pentosidine.

The conditions under which the specimen is contacted with the protein having activity that oxidatively degrades pentosidine are not particularly limited as long as under the conditions, pentosidine can be degraded.

In the case of using, for example, pentosidine oxidase as the protein having activity that oxidatively degrades pentosidine, its amount is appropriately selected depending on the amount of pentosidine that may be contained in the specimen, reaction conditions, etc., and is 0.001 to 50 U/ml, preferably 0.01 to 10 U/ml. The pH can be adjusted to, for example, pH 4 to 10, preferably pH 5.5 to 9, taking into consideration the range that allows the pentosidine oxidase used to act. An arbitrary known pH adjuster and buffer solution can be used according to the specimen and the pH to be adjusted. For example, 20 to 60°C, preferably 30 to 55°C, can be adopted as a reaction temperature taking into consideration the optimum temperature range of the pentosidine oxidase used. The reaction time can be a time sufficient for degrading the desired amino acid, and the reaction can be performed for, for example, 1 to 120 minutes, preferably 2 to 60 minutes.

Then, change resulting from the contact is detected. As used herein, the "change resulting from the contact" means the presence or absence of a starting material such as pentosidine contained in the specimen, a reaction product with the protein having activity that oxidatively degrades pentosidine or a material consumed through the reaction, etc., or time-dependent change in amount thereof.

In a more specific aspect, the method for measuring pentosidine may comprise the steps of:

(A) allowing pentosidine oxidase to act on a specimen in the presence of water and oxygen; and
(B) measuring an amount of at least one of a reaction product and a material consumed through the reaction through the action of the pentosidine oxidase.

Examples of the reaction product to be measured in the step (B) can include hydrogen peroxide, ammonia and pentosidine deamination products. The amount of the reaction product hydrogen peroxide can be measured, for example, through peroxidase reaction. The amount of the reaction product ammonia can be measured by, for example, a method using an indophenol method or Nessler's reagent, or a method of measuring the amount of NADH using an enzyme for ammonia as a substrate, such as glutamic acid dehydrogenase or NAD synthase. As used herein, the "deamination product" means, for example, a product having keto acid at at least one of the ends by the removal of one or both of the amino groups of lysine and arginine constituting pentosidine and the replacement thereof with oxygen. Figure 5 shows an example of such a deamination product. Examples of the material consumed through the reaction to be measured in the step (B) can include oxygen. The amount of oxygen decreased through enzyme reaction can be measured using, for example, an oxygen electrode. Alternatively, the amount may be colorimetrically determined by oxidizing a manganese ion with oxygen on the basis of the Winkler approach.

It has been reported that plasma has a pentosidine concentration higher by approximately 70% in, for example, schizophrenia patients (68.4 ng/mL) compared with healthy individuals (mean ± S.D.: 39.6 ± 7.8 ng/mL) (Arai et al., Psychiatria et Neurologia Japonica (2012), Vol. 114, No. 2, pp. 101-107; and Arai, M., et al. Arch Gen Psychiat, 67; 589-597, 2010). According to the measurement method of the present embodiment, the degradation with the amino acid degrading enzyme prior to contact with the protein having activity that oxidatively degrades pentosidine decreases measurement errors ascribable to the reaction of the protein having activity that oxidatively degrades pentosidine with another amino acid to less than 70%, preferably less than 60%, more preferably less than 50%, and allows more accurate measurement of pentosidine. Therefore, the measurement method of the present embodiment is also very useful in the diagnosis of a disease associated with pentosidine.

In another aspect, the present embodiment provides a kit for measuring pentosidine in a specimen, comprising: an amino acid degrading enzyme and a protein having activity that oxidatively degrades pentosidine. The kit according to the present embodiment can be used for detecting a reaction product of pentosidine and the protein having activity that oxidatively degrades pentosidine, or a material consumed through the reaction. The kit according to the present embodiment may further contain at least one of a buffer solution for reaction, a reagent for reaction product detection, for example, a reagent for hydrogen peroxide detection, a reagent for ammonia detection and a reagent for pentosidine deamination product detection, and a reagent for detection of a material consumed through the reaction, for example, a reagent for oxygen detection. The kit of the present embodiment may be used as an *ex vivo* diagnostic drug and can be suitably used, for example, in the diagnosis of a disease associated with pentosidine or a reaction product of pentosidine and pentosidine oxidase, for example, diabetes mellitus or nephropathy.

Examples of the reagent for hydrogen peroxide detection include 10-(carboxymethylaminocarbonyl)-3,7-bis(dimethylamino)-phenothiazine (DA-67) and N-(carboxymethylaminocarbonyl)-4,4'-bis(dimethylamino)-diphenylamine (DA-64), which can detect hydrogen peroxide with high sensitivity, as well as known chromogenic reagents such as Trinder's reagent. Examples of the reagent for ammonia detection include a combination of phenol-sodium nitroprusside and an oxidizing agent such as sodium hypochlorite (indophenol method), and Nessler's reagent. Examples of the reagent for oxygen detection include manganese ions and a combination of sodium hydroxide and sulfuric acid.

The detection of the reaction product using chromogenic reaction can be performed very conveniently and inexpensively as compared with an immunochemical method or an instrumental analytical approach. However, the detection of the reaction product or the material consumed through the reaction does not exclude other known quantitative or qualitative methods except for detection reagents, and any of the methods may be appropriately adopted. The detection may be performed using, for example, an apparatus such as an enzyme sensor equipped with a dedicated detection electrode, instead of the hydrogen peroxide or ammonia detection reagent.

The method for detecting the reaction product or the material consumed through the reaction may also be used in a method for detecting a disease associated directly or indirectly with pentosidine or each reaction product or material consumed through the reaction, and by extension, a method for diagnosing the disease.

The present embodiment further relates to a method for producing a reaction product of pentosidine derived from a specimen, comprising the steps of:

degrading the specimen with an amino acid degrading enzyme; and
contacting the specimen after the degradation step with a protein having activity that oxidatively degrades pentosidine, wherein

the amino acid degrading enzyme and the protein having activity that oxidatively degrades pentosidine are different from each other.

Each step can be carried out with reference to the description about the method for measuring pentosidine.

Hereinafter, the present embodiment will be described in more detail with reference to Examples. However, the present invention is not limited by these Examples. The present invention can assume various forms as long as the object of the present invention is attained.

Examples

(Example 1) Methods for culturing *Sarocladium* sp. and preparing enzyme liquid

- Medium used

MEA medium: Malt extract agar (manufactured by Oxoid Ltd.) was dissolved in distilled water into 50 g/L.

YMG medium: 0.4% yeast extract, 1% malt extract, and 0.4% glucose, pH 5.5

- Culture of fungus strain

*Sarocladium* sp. F10012 strain preserved at -80°C was applied to MEA medium and statically cultured at 24°C for 7 to 10 days until a sufficient amount of hypha was obtained. The obtained hypha was inoculated to 250 mL of YMG medium in a 1 L flask and shake-cultured at 30°C for 3 days.

- Preparation of crude enzyme liquid

The YMG medium containing the cultured fungus body was filtered using Miracloth (manufactured by Merck Millipore) for the removal of the fungus body to obtain a culture supernatant. The process of concentrating the culture supernatant using an ultrafiltration membrane (Vivaspin 20-3k, manufactured by GE Healthcare Japan Corp.), and diluting the concentrate with a 50 mM potassium phosphate buffer (pH 7.5) was repeated a plurality of times to replace the YMG medium with the potassium phosphate buffer while removing small molecules.

- Partial purification of enzyme of interest

The buffer-replaced crude enzyme liquid was fractionated using a column for ion-exchange chromatography (HiTrap Q Sepharose Fast Flow 1 mL, manufactured by GE Healthcare Japan Corp.). Specific procedures are as follows.
First, the crude enzyme liquid was loaded onto a column equilibrated with a 50 mM potassium phosphate buffer (pH 7.5) so that the enzyme was adsorbed onto the column. Then, the column was washed with 5 mL of a potassium phosphate buffer to elute unadsorbed proteins.
Then, 5 mL each of buffers containing 0.25 M, 0.5 M, 0.75 M, or 1.0 M sodium chloride dissolved in a potassium phosphate buffer was sequentially passed through the column to elute proteins adsorbed on the column.
A liquid eluted from the column upon loading of the crude enzyme liquid was designated as "Flow through"; a liquid eluted at the time of washing with the buffer was designated as "Start buffer"; and liquids eluted with the buffers containing sodium chloride were designated as "Elution 1", "Elution 2", "Elution 3" and "Elution 4", respectively. These liquids were separately recovered into different containers.

(Example 2) Method for measuring pentosidine oxidase activity

- Activity measurement of partially purified enzyme liquid

Each liquid eluted from the column for ion-exchange chromatography was used as a sample to measure activity. 50 μL of the sample was mixed with 25 μL of 4 mM pentosidine (in terms of a free form) (manufactured by Peptide Institute, Inc.; 3-trifluoroacetate (TFA) salt was used) dissolved in a 100 mM potassium phosphate buffer (pH 8.0) and 25 μL of an oxidase coloring reagent (4 U/mL peroxidase (manufactured by Toyobo Co., Ltd.), 1.8 mM 4-aminoantipyrine (manufactured by Fluka/Honeywell International Inc.), and 2 mM TOOS (manufactured by Dojindo Laboratories Co., Ltd.)), and the mixture was reacted at room temperature.
For the reaction, a 96-well microwell plate (manufactured by Nunc/Thermo Fisher Scientific, Inc.) was used. The blank used was supplemented with a 100 mM potassium phosphate buffer (pH 8.0) instead of a substrate solution. The absorbance at 555 nm of the reaction liquids and the blank solution was measured, and the strength of enzymatic activity was evaluated on the basis of difference in absorbance (ΔOD).

- Substrate concentration dependence test

The pentosidine oxidase activity of each partially purified enzyme liquid was measured using varying concentrations of a substrate to evaluate change in activity against the concentrations of the substrate. The concentrations of the substrate solutions used were 0.13 mM, 0.25 mM, 0.5 mM, 1.0 mM, 2.0 mM and 4.0 mM.

- Thermal deactivation test

Each partially purified enzyme liquid was heat-treated at 80°C for 1 hour for protein denaturation. The pentosidine oxidase activity of this heat-treated sample was measured in accordance with the method for measuring activity mentioned above, and compared with the activity of an unheated sample.

(Example 3) Pentosidine oxidase activity analysis of *Sarocladium* sp. enzyme liquid

Each sample of the culture supernatant of *Sarocladium* sp. fractionated with a column for ion-exchange chromatography was analyzed for its reactivity with pentosidine. As a result, strong activity was observed in Elution 1 obtained by elution with the potassium phosphate buffer containing 0.25 M sodium chloride, suggesting that pentosidine oxidase was contained therein. As a result of subjecting this Elution 1 to the substrate concentration dependence test (Figure 1) and the thermal deactivation test (Figure 2), the enzymatic activity was found to be elevated in a substrate concentration-dependent manner and completely deactivated by heat treatment. This indicated that the pentosidine oxidase activity observed in Elution 1 was derived from the enzyme.

(Example 4) Sequencing pentosidine oxidase derived from *Sarocladium* sp.

Two types of putative pentosidine oxidase genes (SEQ ID NO: 1 and SEQ ID NO: 3) and amino acid sequences thereof (SEQ ID NO: 2 and SEQ ID NO: 4) were identified on the basis of the results described above and sequence information on the whole genome of *Sarocladium* sp.

(Example 5) Heterologous recombinant expression of pentosidine oxidase derived from *Sarocladium* sp. in *Aspergillus sojae*

In order to analyze the enzymatic activity of two pentosidine oxidases identified as described above, heterologous recombinant expression was performed with *Aspergillus sojae* as a host.

- Preparation of expression vector

The codon-modified nucleotide sequences of SEQ ID NOs: 5 and 6 for expression in *Aspergillus* were each obtained by artificial gene synthesis on the basis of the amino acid sequences of SEQ ID NO: 2 and SEQ ID NO: 4.

For an expression cassette for expressing the pentosidine oxidase genes (penox1 and penox2) of SEQ ID NO: 5 and SEQ ID NO: 6, translation elongation factor gene tef1 promoter sequence Ptef (748-bp upstream region of the tef1 gene; SEQ ID NO: 7) was used as a promoter, and alkaline protease gene alp terminator sequence Talp (800-bp downstream region of the alp gene; SEQ ID NO: 8) was used as a terminator.

The selective marker used was transcription marker gene pyrG3 (1,487 bp including a 56-bp upstream region, an 896-bp coding region and a 535-bp downstream region; SEQ ID NO: 9) which complements uracil/uridine auxotrophy and allows multicopy transfer of a gene (see Japanese Patent Laid-Open No. 2018-068292). These Ptef, Talp, and pyrG3 sequences were obtained through PCR reaction with the genomic DNA of *Aspergillus sojae* NBRC4239 strain as a template.

Next, In-Fusion HD Cloning Kit (manufactured by Clontech Laboratories, Inc.) was used for linking these DNAs. For example, in the case of linking Ptef, the penox1 gene and Talp, DNA fragments were amplified through PCR reaction using the reverse primer of SEQ ID NO: 10 for Ptef and the forward primer of SEQ ID NO: 11 for Talp. In this respect, a 15-bp sequence complementary to the 5' end of the penox1 gene (SEQ ID NO: 5) was added to the 5' end of the reverse primer for Ptef amplification of SEQ ID NO: 10. A 15-bp sequence homologous to the 3' end of the penox1 gene (SEQ ID NO: 5) was added to the 5' end of the forward primer for Talp amplification of SEQ ID NO: 11. Therefore, Ptef, the penox1 gene and Talp can be linked through in-fusion reaction. In this way, expression vectors p19-pG3-penox1 and p19-pG3-penox2 were prepared in which Ptef-penoxl-Talp-pyrG3 or Ptef-penox2-Talp-pyrG3 consisting of Ptef, the penox1 gene or the penox2 gene, Talp and pyrG3 linked in this order was inserted in the multicloning site of a pUC19 plasmid.

- Preparation and culture of expressing *Aspergillus* strain

A pyrG gene disruptant (strain deficient in a 48-bp upstream region, an 896-bp coding region and a 240-bp downstream region of the pyrG gene) of *Aspergillus sojae* was transformed by the protoplast PEG method using the plasmid p19-pG3-penox1 or p19-pG3-penox2 for transformation obtained as described above to obtain nine As-penox1 strain and six As-penox2 strain as *Aspergillus sojae* transformants having multicopy inserts of the expression cassette of penox1 or penox2.

Each of the obtained *Aspergillus sojae* transformants (As-penoxl strain and As-penox2 strain) was inoculated to 15 mL of PPY liquid medium (2% (w/v) Pinedex, 1% (w/v) Polypeptone, 0.5% (w/v) yeast extracts, 0.5% (w/v) monopotassium dihydrogen phosphate, and 0.05% (w/v) magnesium sulfate heptahydrate) contained in a 50 mL Erlenmeyer flask, and shake-cultured at 30°C for 4 to 5 days.

- Preparation of hypha extract

The culture liquid of each of the As-penox1 strain and the As-penox2 strain was filtered using Miracloth (manufactured by Merck Millipore) for the removal of the culture supernatant to obtain a fungus body. The fungus body was resuspended in 15 mL of a 10 mM potassium phosphate buffer (pH 7.5) and then disrupted using Micro Smash MS-100R (manufactured by Tomy Seiko Co., Ltd.). The fungus body homogenates were centrifuged at 15,000 rpm for 15 minutes to recover a supernatant as a crude enzyme liquid.

- Measurement of L-arginine oxidizing activity of hypha extract

200 μL of each crude enzyme liquid was mixed with 380 μL of a solution of 7.1 U/mL peroxidase, 0.70 mM 4-aminoantipyrine, and 0.79 mM TOOS dissolved in a 150 mM potassium phosphate buffer (pH 7.0), and the mixture was incubated at 37°C for 5 minutes. Then, 20 μL of a 60 mM L-arginine solution was added thereto, and the mixture was stirred and reacted at 37°C for 5 minutes. Time-dependent change in $A_{555}$ during the reaction was measured in a spectrophotometer (U-3900, manufactured by Hitachi High-Tech Science Corp.). A control experiment was carried out by adding 20 μL of ion-exchange water instead of 20 μL of the 60 mM L-arginine solution. The amount of the enzyme that produced 1 μmol of hydrogen peroxide per minute at 37°C was defined as 1 unit (U). The activity was calculated according to the following expression.

$$\text{Activity (U/mL)} = \{(\Delta As - \Delta A0) \times 0.6 \times df\} / (39.2 \times 0.5 \times 0.2)$$

ΔAs: Amount of change in $A_{555}$ per minute of the reaction liquid
ΔA0: Amount of change in $A_{555}$ per minute of the control experiment
39.2: Millimolar extinction coefficient ($mM^{-1}\cdot cm^{-1}$) of a quinonimine dye produced through reaction
0.5: Molar number of a quinonimine dye produced with 1 mol of hydrogen peroxide
0.6: Total volume (mL) of the reaction liquid
df: Dilution coefficient
0.2: Volume (mL) of the enzyme liquid

The L-arginine oxidizing activity of the crude enzyme liquids of the As-penox1 strain and the As-penox2 strain was 0.009 U/mL (As-penox1-15 strain) and 5.1 U/mL (As-penox2-16 strain), respectively, at maximum.

(Example 6) Purification of recombinant penox2 extracted from hypha

The crude enzyme liquid of the As-penox2-16 strain was buffer-replaced with a 10 mM potassium phosphate buffer (pH 7.5) and then fractionated using an anion-exchange chromatography column (HiScreen Capto Q, manufactured by GE Healthcare Japan Corp.). First, the crude enzyme liquid was loaded onto a column equilibrated with a 10 mM potassium phosphate buffer (pH 7.5) so that the enzyme was adsorbed onto the column. Then, the column was washed with a 10 mM potassium phosphate buffer (pH 7.5) to elute unadsorbed proteins. Then, proteins adsorbed on the column were eluted while the concentration of sodium chloride contained in a 10 mM potassium phosphate buffer (pH 7.5) was linearly elevated from 0 mM to 40 mM. Fractions that exhibited L-arginine oxidizing activity were analyzed by SDS-PAGE to recover a fraction free from foreign proteins as purified PenOX2. The recovered purified PenOX2 solution was concentrated using an ultrafiltration membrane (Amicon Ultra 15-30k, manufactured by Merck KGaA) until the L-arginine oxidizing activity reached 24 U/mL, and used in a pentosidine quantification test.

(Example 7) Pentosidine quantification test

The following reagents were prepared, and pentosidine was measured using Bio Majesty JCA-BM1650 (manufactured by JEOL Ltd.).

(Sample: pentosidine solution)

0.2 μM, 0.4 μM, 0.6 μM, 1.0 μM, 2.0 μM or 4.0 μM pentosidine solution (prepared using the same pentosidine as in Example 2)

(First reagent: leuco dye, peroxidase solution)

120 mM potassium phosphate buffer (pH 7.0)
0.2 mM DA-67 (10-(carboxymethylaminocarbonyl)-3,7-bis(dimethylamino)phenothiazine, sodium salt) (manufactured by FUJIFILM Wako Pure Chemical Corp.)
3.0 U/mL peroxidase

(Second reagent: PenOX2 solution)

120 mM potassium phosphate buffer (pH 7.0)
24 U/mL PenOX2

25 μL of each sample was added to 50 μL of the first reagent, and the mixture was incubated at 37°C for 5 minutes. Then, 25 μL of the second reagent was added thereto, and pentosidine oxidation reaction mediated by PenOX2 and reaction for the detection of hydrogen peroxide produced through the reaction were allowed to proceed at 37°C for 5 minutes.

In the reaction for the detection of hydrogen peroxide, DA-67 was oxidized, with concomitant consumption of peroxidase, into methylene blue which in turn developed color while absorbance ($A_{658}$) was elevated. Figure 3 shows, as one example, the relationship between the time elapsed from the mixing of the sample (4.0 μM pentosidine solution) with the first reagent and the absorbance ($A_{658}$). Elevation in $A_{658}$ was able to be confirmed immediately after addition of the second reagent containing PenOX2.

Subsequently, the amount of elevation in $A_{658}$ ($\Delta$A) caused by the oxidation of pentosidine was calculated according to the following expression.

$$\Delta A = \text{(Absorbance 5 minutes after addition of the second reagent)} - \text{(Absorbance immediately before addition of the second reagent} \times 0.75)$$

(Since the concentration of the composition in the reaction liquid was changed 0.75-fold (75/100-fold) by the addition of the second reagent,
the value obtained by multiplying the absorbance immediately before addition of the second reagent by 0.75 was regarded as absorbance immediately after addition of the second reagent.)

Correlation held true between the final pentosidine concentration and ΔA (Figure 4). This indicated that PenOX2 exhibits pentosidine oxidizing activity and can be used in the quantification of pentosidine. Likewise, PenOX1 exhibited pentosidine oxidizing activity, though the results are not shown.

(Example 8) Purification of recombinant penox2 secreted from hypha

The hypha culture liquid of the As-penox2 strain was filtered using Miracloth (manufactured by Merck Millipore) to recover a hypha culture supernatant. 75 mL of the obtained hypha culture supernatant was filtered through a syringe filter having a pore size of 0.2 μm and then concentrated using an ultrafiltration membrane (Amicon Ultra 15-30k, manufactured by Merck KGaA). Ammonium sulfate was gradually added to the concentrate so as to attain 70% saturation. The mixture was left at 4°C for 2 hours and then centrifuged (15,000 rpm, 4°C, 5 min) to recover a supernatant while precipitating redundant proteins. The recovered supernatant was concentrated using an ultrafiltration membrane (Amicon Ultra 0.5-30k, manufactured by Merck KGaA).

A 50 mM potassium phosphate buffer (pH 7.5) containing 2 M ammonium sulfate was added to this concentrate, and the mixture was then fractionated using a column for hydrophobic interaction chromatography (HiTrap Butyl Fast Flow 1 mL, manufactured by GE Healthcare Japan Corp.). Specific procedures are as follows.

First, the crude enzyme liquid was loaded onto a column equilibrated with a 50 mM potassium phosphate buffer (pH 7.5) containing 2 M ammonium sulfate so that the enzyme was adsorbed onto the column. Then, the column was washed with 10 mL of a 50 mM potassium phosphate buffer (pH 7.5) containing 2 M ammonium sulfate to elute unadsorbed proteins.

Then, 5 mL each of 50 mM potassium phosphate buffers (pH 7.5) containing 1.5 M, 1.3 M, or 1.15 M ammonium sulfate, 10 mL of a 50 mM potassium phosphate buffer (pH 7.5) containing 1 M ammonium sulfate, and 5 mL of a 50 mM potassium phosphate buffer (pH 7.5) free from ammonium sulfate were sequentially passed through the column to elute proteins adsorbed on the column.

A liquid eluted from the column upon loading of the crude enzyme liquid was designated as "Flow through 1"; a liquid eluted at the time of washing with the buffer containing 2 M ammonium sulfate was designated as "Elution 1"; liquids eluted with the buffers containing 1.5 M, 1.3 M, 1.15 M, or 1 M ammonium sulfate were designated as "Elution 2", "Elution 3", "Elution 4" and "Elution 5", respectively; and a liquid eluted with the buffer free from ammonium sulfate was designated as "Elution 6". These liquids were separately recovered into different containers.

Each fractionated sample was analyzed for its reactivity with pentosidine. As a result, strong activity was observed in Elution 5 obtained by elution with the potassium phosphate buffer containing 1 M ammonium sulfate, suggesting that pentosidine oxidase (PenOX2) was contained therein. This Elution 5 was concentrated using an ultrafiltration membrane (Amicon Ultra 15-30k, manufactured by Merck KGaA), and the concentrate was buffer-replaced with a 50 mM potassium phosphate buffer (pH 7.5) free from ammonium sulfate and then concentrated again using an ultrafiltration membrane (Amicon Ultra 15-30k, manufactured by Merck KGaA). This concentrate was fractionated using a column for ion-exchange chromatography (HiTrap Q Sepharose Fast Flow 1 mL, manufactured by GE Healthcare Japan Corp.). Specific procedures are as follows.

First, the crude enzyme liquid was loaded onto a column equilibrated with a 50 mM potassium phosphate buffer (pH 7.5) so that the enzyme was adsorbed onto the column. Then, the column was washed with 5 mL of a 50 mM potassium phosphate buffer (pH 7.5) to elute unadsorbed proteins.

Then, 1 mL (which was passed five times) of a liquid of 0.1 M sodium chloride dissolved in a 50 mM potassium phosphate buffer (pH 7.5), 1 mL (which was passed five times) of a liquid of 0.175 M sodium chloride dissolved in the buffer, and 5 mL (which was passed once) of a liquid of 1 M sodium chloride dissolved in the buffer were sequentially passed through the column to elute proteins adsorbed on the column.

A liquid eluted from the column upon loading of the crude enzyme liquid was designated as "Flow through 2"; a liquid eluted at the time of washing with the buffer was designated as "Elution 7"; and liquids eluted with the buffers containing sodium chloride were designated as "Elution 8-1", "Elution 8-2", "Elution 8-3", "Elution 8-4", "Elution 8-5", "Elution 9-1", "Elution 9-2", "Elution 9-3", "Elution 9-4", "Elution 9-5" and "Elution 10", respectively. These liquids were separately recovered into different containers.

Each fractionated sample was analyzed for its reactivity with pentosidine. As a result, strong activity was observed in Elution 9-1 and Elution 9-2 obtained by elution with the potassium phosphate buffer containing 0.175 M sodium chloride, suggesting that pentosidine oxidase was contained therein. As a result of analyzing a mixture of the active fractions Elution 9-1 and Elution 9-2 in equal amounts by SDS-PAGE, substantially a single band was obtained (molecular weight: approximately 80,000). The active fractions thus obtained were used for determining the following physicochemical properties.

(Example 9) Physicochemical property of PenOX2 produced from *Aspergillus sojae* transformant As-penox2 strain

In order to determine the physicochemical properties of PenOX2, the following method for measuring enzymatic activity was used.

600 μL of an arbitrary buffer, 400 μL of a solution of 3.99 U/mL peroxidase, 1.8 mM 4-aminoantipyrine, and 2 mM TOOS dissolved in deionized water, and 150 μL of deionized water were incubated at an arbitrary temperature for 10 minutes. Then, 50 μL of the enzyme liquid preserved on ice and 400 μL of a solution of 2 mM pentosidine dissolved in a 100 mM potassium phosphate buffer (pH 8.0) incubated at an arbitrary temperature for 10 minutes were added thereto, and the mixture was stirred and reacted at an arbitrary temperature for 3 minutes. Time-dependent change in $A_{555}$ during the reaction was measured in a spectrophotometer (U-3900, manufactured by Hitachi High-Tech Science Corp.). Time elapsed after the start of measurement - amount of change in $A_{555}$ from 20 seconds to 60 seconds was regarded as an activity value.

The amount of the enzyme that produced 1 μmol of hydrogen peroxide per minute at 37°C was defined as 1 unit (U). The activity was calculated according to the following expression.

$$\text{Activity (U/mL)} = \{(\Delta As - \Delta A0) \times 1.6 \times df\} / (39.2 \times 0.5 \times 0.05)$$

ΔAs: Amount of change in $A_{555}$ per minute of the reaction liquid
ΔA0: Amount of change in $A_{555}$ per minute of the control experiment
1.6: Total volume (mL) of the reaction liquid
df: Dilution coefficient
39.2: Millimolar extinction coefficient ($mM^{-1} \cdot cm^{-1}$) of a quinonimine dye produced through reaction

0.5: Molar number of a quinonimine dye produced with 1 mol of hydrogen peroxide
0.05: Volume (mL) of the enzyme liquid

The physicochemical properties of penox2 were as follows.

(a) Range of optimum pH

Each buffer was prepared as a 50 mM (final concentration) citric acid-100 mM potassium phosphate buffer (pH 4.0 to 7.5), a 100 mM (final concentration) potassium phosphate buffer (pH 6.5 to 8.0), and a 100 mM (final concentration) glycine buffer (pH 8.0 to 11.0). Enzyme reaction was performed at each pH at a temperature of 37°C using these buffers. The results are shown in Figure 7. PenOX2 exhibited the highest activity at pH 7.5. Also, the activity exhibited at pH 6.5 to 8.0 was 70% or more of the activity value around pH 7.5 of the potassium phosphate buffer. It was therefore concluded that the optimum pH of PenOX2 is pH 6.5 to 8.0 and is most preferably pH 7.5.

(b) Range of optimum temperature

The activity of PenOX2 was measured at varying temperatures using a 50 mM (final concentration) potassium phosphate buffer (pH 7.5). The results are shown in Figure 8. The temperature range in which 80% or more activity was exhibited, relative to the activity at a temperature around 50°C at which the highest activity was exhibited, was from 37°C to 50°C. It was thus concluded that the range of the optimum temperature of PenOX2 is from 37°C to 50°C.

(c) Heat stability

The enzyme liquid treated at each temperature for 10 minutes was evaluated for residual activity by performing the activity measurement described above at a temperature of 37°C using a 100 mM (final concentration) potassium phosphate buffer (final pH at the time of activity measurement: 7.5). The results about heat stability are as shown in Figure 9, and PenOX2 was stable up to around 30°C.

(d) Range of stable pH

Treatment at each pH at 25°C for 20 hours was performed using a 100 mM citric acid-200 mM potassium phosphate buffer (pH 3.0 to 6.5), a 200 mM potassium phosphate buffer (pH 6.5 to 8.0), or a 200 mM glycine buffer (pH 8.0 to 10.0) as a buffer solution, followed by the measurement of the residual activity of PenOX2. The results are shown in Figure 10. The pH range in which 90% or more activity was exhibited, relative to the activity of PenOX2 preserved at 4°C, was from pH 4.5 to 7.5, and the pH range in which 60% or more activity was exhibited was from pH 4.0 to 9.0.

(e) Activity value against pentosidine

In the method for measuring activity described above, the activity was measured at 37°C using a 50 mM (final concentration) potassium phosphate buffer (final pH at the time of activity measurement: 7.5), and an activity value (U/mL) was determined according to the calculation expression described above. The activity value was found to be 7.8 U/mL with specific activity of 29.1 U/mg (Bradford method).

(f) Km value for pentosidine

In the method for measuring activity described above, the activity was measured at varying concentrations of the substrate pentosidine at 37°C using a 50 mM (final concentration) potassium phosphate buffer (pH 7.5), and a Michaelis constant (Km) was determined from a Lineweaver-Burk plot. The results are shown in Figure 11. The Km value for pentosidine (free form) was found to be 0.070 mM.

(g) Molecular weight

A molecular weight was determined by SDS-PAGE performed according to the method of Laemmli. The electrophoresis gel used was Mini-PROTEAN TGX Stain-Free Precast Gels 4-20% (manufactured by Bio-Rad Laboratories, Inc.), and the molecular weight marker used was Precision Plus Protein All Blue Prestained Protein Standards. The results are shown in Figure 12. The molecular weight of PenOX2 was approximately 80,000.

(Example 10) Pentosidine oxidase activity measurement of enzyme having sequence homology to PenOX1 or PenOX2

As mentioned above, both PenOX1 and PenOX2 had pentosidine oxidase activity. When the percent match between their amino acid sequences was examined using the BLAST program, the amino acid sequence homology therebetween was 38.2%. Subsequently, three enzymes given below were purchased and examined for their pentosidine oxidase activity by the method for measuring activity described above at 37°C using a 100 mM (final concentration) potassium phosphate buffer (pH 7.5). The amino acid sequence homology to PenOX1 and PenOX2 and pentosidine oxidase activity of each enzyme were as follows.

(a) *Crotalus adamanteus*-derived amino acid oxidase Type VI (manufactured by Merck KGaA) (SEQ ID NO: 12)
Molecular weight: 130,000
The amino acid sequence homology of this enzyme to PenOX1 and PenOX2 was 26.8% and 23.5%, respectively. The enzyme was diluted to a concentration of 1 mg/mL (Biuret method) with deionized water and used in activity measurement. The pentosidine oxidase activity of this enzyme was 0.555 (U/mL) with specific activity of 0.555 (U/mg).
(b) *Crotalus atrox*-derived amino acid oxidase Type I (manufactured by Merck KGaA) (SEQ ID NO: 13)

Molecular weight: 59,000 (calculation value based on the amino acid sequence)

The amino acid sequence homology of this enzyme to PenOX1 and PenOX2 was 26.3% and 23.4%, respectively. 1 mg of the enzyme powder was dissolved in 1 mL of deionized water and used in activity measurement. The pentosidine oxidase activity of this enzyme was 0.022 (U/mL) with specific activity of 0.022 (U/mg) as a reference value.

(c) *Trichoderma viride*-derived lysine oxidase (manufactured by Merck KGaA) (SEQ ID NO: 14) molecular weight: 116,000

The amino acid sequence homology of this enzyme to PenOX1 and PenOX2 was 24.0% and 23.3%, respectively. 1 mg of the enzyme powder was dissolved in 1 mL of deionized water and used in activity measurement. The pentosidine oxidase activity of this enzyme was 0.063 (U/mL) with specific activity of 0.063 (U/mg) as a reference value. The sequence homology among the enzymes used in this Example is shown in the following table.

[Table 2]

| | penox1 | penox2 | (a)Cad_LAAO | (b)Cat_LAAO | (c)Tvi_LysO |
|---|---|---|---|---|---|
| penox1 | | 38.2% | 26.8% | 26.3% | 24.0% |
| penox2 | | | 23.5% | 23.4% | 23.3% |
| (a)Cad_LAAO | | | | 98.6% | 25.1% |
| (b)Cat_LAAO | | | | | 24.6% |
| (c)Tvi_LysO | | | | | |

(Example 11) Heterologous recombinant expression of escapin in *Aspergillus sojae*

The heterologous recombinant expression of a gene encoding mature escapin with a 5'-terminally added gene encoding a signal peptide of the genus *Aspergillus* was performed with *Aspergillus sojae* as a host.

- Preparation of expression vector

The gene sequence of mature escapin used was codon-modified 1,554 base pairs for expression in *Aspergillus* (SEQ ID NO: 18) based on the amino acid sequence of SEQ ID NO: 15 (nucleotide sequence: SEQ ID NO: 17) derived from *Aplysia californica* described in the literature (Yang et al., J Exp Biol, 208 (18): 3609-22, 2005).
The gene encoding the signal peptide used was 69-base pair AoCDHss (exon region of a gene encoding the signal peptide of *Aspergillus oryzae*-derived cellulose dehydrogenase; SEQ ID NO: 16).
In order to integrate the gene encoding mature escapin with the 5'-terminally linked gene encoding a signal peptide of the genus *Aspergillus* (SEQ ID NO: 16 linked to the 5' end of SEQ ID NO: 18; hereinafter, referred to as gene sequence A) into a plasmid, gene sequence A with a 5'-terminally added 12-base pair gene sequence (SEQ ID NO: 20) and a 3'-terminally added 12-base pair gene sequence (SEQ ID NO: 21) was obtained (hereinafter, the resulting sequence is referred to as gene sequence A') by artificial gene synthesis.
For an expression cassette for expressing the gene sequence A, translation elongation factor gene tef1 promoter sequence Ptef (748-bp upstream region of the tef1 gene; SEQ ID NO: 7) was used as a promoter, and alkaline protease gene alp terminator sequence Talp (800-bp downstream region of the alp gene; SEQ ID NO: 8) was used as a terminator.

The selective marker used was transcription marker gene pyrG3 (1,487 bp including a 56-bp upstream region, an 896-bp coding region and a 535-bp downstream region; SEQ ID NO: 9) which complements uracil/uridine auxotrophy and allows multicopy transfer of a gene (see Japanese Patent Laid-Open No. 2018-068292). These Ptef, Talp, and pyrG3 sequences were obtained through PCR reaction with the genomic DNA of *Aspergillus sojae* NBRC4239 strain as a template.

Next, In-Fusion HD Cloning Kit (manufactured by Clontech Laboratories, Inc.) was used for linking these DNAs. For example, in the case of linking Ptef, the gene sequence A and Talp, DNA fragments were amplified through PCR reaction using the reverse primer of SEQ ID NO: 22 for Ptef and the forward primer of SEQ ID NO: 23 for Talp. In this respect, the 5' end of the gene sequence A' had a 15-bp sequence (CAT sequence complementary to the start codon ATG of AoCDHss, and SEQ ID NO: 20) complementary to the 5' end of the reverse primer for Ptef amplification (SEQ ID NO: 22). The 3' end of the gene sequence A' had a 15-bp sequence (the stop codon TGA sequence of escapin and SEQ ID NO: 21) complementary to the 5' end of the forward primer for Talp amplification (SEQ ID NO: 23). Therefore, Ptef, the gene sequence A and Talp can be linked through in-fusion reaction. In this way, expression vector p19-pG3-AoCDHss-Escapin was prepared in which Ptef-AoCDHss-Escapin-Talp-pyrG3 consisting of Ptef, AoCDHss, mature escapin, Talp and pyrG3 linked in this order was inserted in the multicloning site of a pUC19 plasmid.

- Preparation and culture of expressing *Aspergillus* strain

A pyrG gene disruptant (strain deficient in a 48-bp upstream region, an 896-bp coding region and a 240-bp downstream region of the pyrG gene) of *Aspergillus sojae* was transformed by the protoplast PEG method using the plasmid p19-pG3-AoCDHss-Escapin for transformation obtained as described above to obtain two AoCDHss-Escapin strain as *Aspergillus sojae* transformants having multicopy inserts of the expression cassette of AoCDHss-Escapin.

Each of the obtained *Aspergillus sojae* transformants (AoCDHss-Escapin strain) was inoculated to 15 mL of PPY liquid medium (2% (w/v) Pinedex, 1% (w/v) Polypeptone, 0.5% (w/v) yeast extracts, 0.5% (w/v) monopotassium dihydrogen phosphate, and 0.05% (w/v) magnesium sulfate heptahydrate) contained in a 50 mL Erlenmeyer flask, and shake-cultured at 30°C for 4 to 5 days.

(Example 12) Measurement of pentosidine in combination with amino acid degrading enzyme

- Preparation of solution containing amino acid degrading enzyme 1 liquid (escapin; SEQ ID NO: 15)

The culture supernatant of each transformant obtained in Example 11 was concentrated using an ultrafiltration membrane (Amicon Ultra 15-30k, manufactured by Merck KGaA).

A saturated aqueous solution of ammonium sulfate cooled in ice was added to the concentrate cooled in ice so as to attain 70% saturation of ammonium sulfate. The mixture was left at 4°C for 2 hours and then centrifuged (15,000 rpm, 4°C, 15 min) to recover a supernatant, which was then redissolved in a 0.1 M potassium phosphate buffer (pH 6.8).

- Preparation of amino acid degrading enzyme 2 liquid (solution containing *Crotalus adamanteus-derived* L-amino acid oxidase (SEQ ID NO: 19))

*Crotalus adamanteus*-derived L-amino acid oxidase Type I (L-Amino Acid Oxidase from *Crotalus adamanteus,* Type I, manufactured by Merck KGaA) was dissolved at 1 mg/ml in a 0.1 M potassium phosphate buffer (pH 6.8). This solution was concentrated using an ultrafiltration membrane (Amicon Ultra 15-30k, manufactured by Merck KGaA) .

- Preparation of enzyme liquid for pentosidine measurement

The *A. sojae* recombinant strain As-penox2 obtained in the sections "Preparation of expression vector" and "Preparation and culture of expressing *Aspergillus* strain" in Example 5 was shake-cultured at 180 rpm at 30°C for 5 days in sterilized PPY medium. The obtained culture supernatant was concentrated using an ultrafiltration membrane (Amicon Ultra 15-30k, manufactured by Merck KGaA). Ammonium sulfate was gradually added to the concentrate so as to attain 70% saturation. The mixture was left at 4°C for 2 hours and then centrifuged (15,000 rpm, 4°C, 15 min) to recover a supernatant. The recovered supernatant was concentrated using an ultrafiltration membrane (Amicon Ultra 0.5-30k, manufactured by Merck KGaA) and buffer-replaced with a 0.1 M potassium phosphate buffer (pH 6.8).

- Foreign substance elimination test in pentosidine measurement

A model system involving various amino acids artificially added as foreign substances to a solution to be measured was used as a pentosidine measurement system using pentosidine oxidase to verify the effect of the measurement method of the present invention on pentosidine measurement.

(1) Preparation of pentosidine solution
Pentosidine (in terms of a free form) (manufactured by Peptide Institute, Inc.; 3TFA salt was used) was dissolved at 0.1 mM, 0.2 mM, 0.3 mM, 0.4 mM or 0.5 mM in deionized water.
(2) Preparation of foreign substance solution
L-Alanine (290 μM), L-cysteine (48 μM), L-aspartic acid (4 μM), L-glutamic acid (57 μM), L-phenylalanine (40 μM), glycine (245 μM), L-isoleucine (54 μM), L-lysine (128 μM), L-leucine (92 μM), L-methionine (22 μM), L-proline (184 μM), L-arginine (60 μM), L-serine (94 μM), L-threonine (116 μM), L-valine (155 μM), L-tryptophan (39 μM) and L-tyrosine (45 μM) were each dissolved in a 0.1 M (final concentration) potassium phosphate buffer (pH 6.8). The amino acid concentrations were established with reference to each data (values of half the upper limit values) on the amounts of the amino acids in the blood of 18-old-year or older people in the values of the literature (Mayo Clinic Laboratories Neurology Catalog, "Plasma Amino Acid Reference Values" (https://neurology.testcatalog.org/show/AAQP, date of access: October 10, 2018; https://www.mayomedicallaboratories.com/test-catalog/Clinical+and+Interpretive/9265, date of access: June 24, 2020).
(3) Preparation of reagent
Reagents for use in measurement were prepared as follows.

3A. Coloring reagent
Peroxidase (manufactured by Toyobo Co., Ltd.) (3.99 U/ml), 4-aminoantipyrine (manufactured by Fluka/Honeywell International Inc.) (1.8 mM), and TOOS (manufactured by Dojindo Laboratories Co., Ltd.) (2 mM) were dissolved in deionized water.
3B. Foreign substance elimination reagent
The amino acid degrading enzyme 1 liquid (1.63 U/ml) and the amino acid degrading enzyme 2 liquid (2.40 U/ml) were mixed at a ratio of 5:3 in terms of the amounts of the liquids.
3C. Reagent for pentosidine measurement
The enzyme liquid for pentosidine measurement (3.31 U/ml) was used.

(4) Measurement

Every measurement was performed at room temperature unless otherwise specified. A 96-well microwell plate (manufactured by Nunc/Thermo Fisher Scientific, Inc.) was used in reaction. 20 μl of the foreign substance solution of (2), 25 μl of the coloring reagent of (3A), and 50 μl of the foreign substance elimination reagent of (3B) were added in order to 5 μl of the pentosidine solution of (1). Ten minutes later, absorbance at 555 nm was measured and designated as data 1.

Subsequently, 25 μl of the reagent for pentosidine measurement of (3C) was added thereto. Ten minutes later, absorbance at 555 nm was measured and designated as data 2. A value (ΔOD) obtained by subtracting the data 1 from the data 2 was used as a measurement value. In Comparative Example 1, measurement was also performed using a solution free from foreign substances, i.e., a solution containing a 0.1 M potassium phosphate buffer (pH 6.8) replaced for the foreign substance solution of (2) .

In Comparative Example 2, measurement was also performed using a system involving foreign substances without foreign substance elimination, i.e., a solution containing a 0.1 M potassium phosphate buffer (pH 6.8) replaced for the foreign substance elimination reagent of (3B) .

Table 3 given below shows an average value from measurement performed three times. Table 4 shows relative values of Comparative Example 2 and Example wherein the measurement value of Comparative Example 1 was defined as 100%.

[Table 3]

| | Comparative Example 1 | Comparative Example 2 | Example 12 |
|---|---|---|---|
| Presence or absence of foreign substance addition | Absent | Present | Present |
| Presence or absence of foreign substance elimination reagent addition | Present | Absent | Present |

(continued)

| | | Comparative Example 1 | Comparative Example 2 | Example 12 |
|---|---|---|---|---|
| Pentosidine concentration | **5nM** | 0.067 | 0.203 | 0.089 |
| | **10nM** | 0.120 | 0.239 | 0.128 |
| | **15nM** | 0.115 | 0.280 | 0.138 |
| | **20nM** | 0.160 | 0.304 | 0.175 |
| | **25nM** | 0.191 | 0.329 | 0.186 |

[Table 4]

| | | Comparative Example 1 | Comparative Example 2 | Example 12 |
|---|---|---|---|---|
| Presence or absence of foreign substance addition | | Absent | Present | Present |
| Presence or absence of foreign substance elimination reagent addition | | Present | Absent | Present |
| Pentosidine concentration | **5nM** | 100% | 303% | 133% |
| | **10nM** | 100% | 199% | 107% |
| | **15nM** | 100% | 243% | 120% |
| | **20nM** | 100% | 190% | 109% |
| | **25nM** | 100% | 172% | 103% |

As shown in the tables, in pentosidine measurement without foreign substance elimination (Comparative Example 2), much higher pentosidine concentrations which reflected the addition of foreign substances were measured than those in measurement without foreign substance addition (Comparative Example 1), and accurate values were not obtained.

By contrast, in measurement comprising the elimination step using the foreign substance elimination reagent, measurement values ascribable to foreign substances were reduced, and values close to the pentosidine concentrations obtained in the foreign substance-free system of Comparative Example 1 were obtained. Thus, accurate measurement was achieved by reducing the influence of foreign substances leading to measurement errors.

(Example 13) Substrate specificity analysis of amino acid degrading enzyme 1

The amino acid eliminating enzyme 1 was analyzed for its substrate specificity for various amino acids and pentosidine.

(1) Preparation of enzyme liquid

The amino acid eliminating enzyme 1 liquid obtained in Example 12 was diluted to 0.134 U/ml with a 0.1 M potassium phosphate buffer (pH 6.8).

(2) Preparation of various amino acid and pentosidine solutions

Pentosidine (which was the same as in Example 12) was dissolved at 2 mM in deionized water. Various amino acids were each dissolved at 4 mM in deionized water.

(3) Preparation of coloring reagent

Peroxidase (manufactured by Toyobo Co., Ltd.) (3.99 U/ml), 4-aminoantipyrine (manufactured by Fluka/Honeywell International Inc.) (1.8 mM), and TOOS (manufactured by Dojindo Laboratories Co., Ltd.) (2 mM) were dissolved in deionized water.

(4) Measurement

Every measurement was performed at room temperature unless otherwise specified. A 96-well microwell plate (manufactured by Nunc/Thermo Fisher Scientific, Inc.) was used in reaction.

25 μl of each amino acid solution or the pentosidine solution of (2) and 25 μl of the coloring reagent of (3) were added in order to 50 μl of the enzyme liquid of (1). At the start and ten minutes later, absorbance at 555 nm was measured. A slope of elevation in absorbance was regarded as a reaction rate for the target substrate.

Figure 13 shows reaction rates for various amino acids and pentosidine wherein the reaction rate for arginine which was the substrate having the highest reaction rate was defined as 100, i.e., substrate specificity.

(Example 14) Substrate specificity analysis of amino acid eliminating enzyme 2

The amino acid eliminating enzyme 2 was analyzed for its substrate specificity for various amino acids and pentosidine.

(1) Preparation of enzyme liquid

The amino acid eliminating enzyme 2 liquid obtained in Example 12 was diluted to 0.12 U/ml with a 0.1 M potassium phosphate buffer (pH 6.8).

Various amino acid and pentosidine solutions (2) and a coloring reagent (3) were prepared in the same way as in Example 13.

(4) Measurement

Every measurement was performed at room temperature unless otherwise specified. A 96-well microwell plate (manufactured by Nunc/Thermo Fisher Scientific, Inc.) was used in reaction. 25 μl of each amino acid solution or the pentosidine solution of (2) and 25 μl of the coloring reagent of (3) were added in order to 50 μl of the enzyme liquid of (1). At the start and ten minutes later, absorbance at 555 nm was measured. A slope of elevation in absorbance was regarded as a reaction rate for the target substrate. Figure 14 shows reaction rates for various amino acids and pentosidine wherein the reaction rate for leucine which was the substrate having the highest reaction rate was defined as 100, i.e., substrate specificity.

(Example 15) Substrate specificity analysis of enzyme for pentosidine measurement

The enzyme for pentosidine measurement was analyzed for its substrate specificity for various amino acids and pentosidine.

(1) Preparation of enzyme liquid

The enzyme liquid for pentosidine measurement obtained in Example 12 was diluted to 0.083 U/ml with a 0.1 M potassium phosphate buffer (pH 6.8).

Various amino acid and pentosidine solutions (2) and a coloring reagent (3) were prepared in the same way as in Example 13.

(4) Measurement

Every measurement was performed at room temperature unless otherwise specified. A 96-well microwell plate (manufactured by Nunc/Thermo Fisher Scientific, Inc.) was used in reaction. 25 μl of each amino acid solution or the pentosidine solution of (2) and 25 μl of the coloring reagent of (3) were added in order to 50 μl of the enzyme liquid of (1). At the start and ten minutes later, absorbance at 555 nm was measured. A slope of elevation in absorbance was regarded as a reaction rate for the target substrate.

Figure 15 shows reaction rates, for various amino acids wherein the reaction rate for pentosidine was defined as 100 i.e., substrate specificity.

The results of Examples 13 to 15 suggested that in Example 12, amino acids highly reactive with the enzyme for pentosidine measurement were eliminated by the amino acid degrading enzyme 1 liquid and the amino acid degrading enzyme 2 liquid, and the accurate measurement of pentosidine was achieved by reducing the influence of foreign substances leading to measurement errors.

## Claims

1. A method for measuring pentosidine in a specimen, the measurement method comprising the steps of:

   degrading the specimen with an amino acid degrading enzyme;
   contacting the specimen after the degradation step with a protein having activity that oxidatively degrades pentosidine; and
   detecting change resulting from the contact, wherein the amino acid degrading enzyme and the protein having activity that oxidatively degrades pentosidine are different from each other.

2. The measurement method according to claim 1, wherein in the detection step, change in an amount of oxygen, hydrogen peroxide or ammonia is detected.

3. The measurement method according to claim 1 or 2, wherein the protein having activity that oxidatively degrades pentosidine has the following physicochemical properties:

   (1) action: activity that oxidatively degrades pentosidine; and
   (2) molecular weight based on SDS-PAGE: 75,000 to 85,000.

4. The measurement method according to any one of claims 1 to 3, wherein the protein having activity that oxidatively degrades pentosidine is any protein selected from the group consisting of the following (a) to (f):

(a) a protein consisting of the amino acid sequence as set forth in SEQ ID NO: 2 or SEQ ID NO: 4;
(b) a protein encoded by a gene consisting of the nucleotide sequence as set forth in SEQ ID NO: 1, SEQ ID NO: 3, SEQ ID NO: 5 or SEQ ID NO: 6;
(c) a protein consisting of an amino acid sequence having 75% or higher identity to the amino acid sequence as set forth in SEQ ID NO: 2 or SEQ ID NO: 4;
(d) a protein encoded by a gene consisting of a nucleotide sequence having 75% or higher identity to the nucleotide sequence as set forth in SEQ ID NO: 1, SEQ ID NO: 3, SEQ ID NO: 5 or SEQ ID NO: 6;
(e) a protein consisting of an amino acid sequence derived from the amino acid sequence as set forth in SEQ ID NO: 2 or SEQ ID NO: 4 by the deletion, substitution and/or addition of one or more amino acids; and
(f) a protein encoded by a nucleotide sequence that hybridizes under stringent conditions to the nucleotide sequence as set forth in SEQ ID NO: 1, SEQ ID NO: 3, SEQ ID NO: 5 or SEQ ID NO: 6.

5. The measurement method according to any one of claims 1 to 4, wherein the protein having activity that oxidatively degrades pentosidine is derived from a filamentous fungus.

6. The measurement method according to any one of claims 1 to 5, wherein

the protein having activity that oxidatively degrades pentosidine is pentosidine oxidase, and
the amino acid degrading enzyme degrades an amino acid contained in the specimen, wherein the amino acid is selected from arginine, leucine, methionine, phenylalanine, tryptophan and tyrosine.

7. The measurement method according to any one of claims 1 to 6, wherein the amino acid to be degraded by the amino acid degrading enzyme is an amino acid against which the protein having activity that oxidatively degrades pentosidine has 40% or higher relative activity when the activity of the protein having activity that oxidatively degrades pentosidine against pentosidine is defined as 100%.

8. The measurement method according to any one of claims 1 to 7, wherein the amino acid degrading enzyme is selected from the group consisting of amino acid oxidase, amino acid dehydrogenase, amino acid aminotransferase, amino acid decarboxylase, amino acid ammonia lyase, amino acid oxygenase and amino acid hydrolase.

9. A kit for measuring pentosidine in a specimen, comprising:

(i) an amino acid degrading enzyme; and
(ii) a protein having activity that oxidatively degrades pentosidine.

10. The kit according to claim 9, wherein the protein having activity that oxidatively degrades pentosidine is any protein selected from the group consisting of the following (a) to (f):

(a) a protein consisting of the amino acid sequence as set forth in SEQ ID NO: 2 or SEQ ID NO: 4;
(b) a protein encoded by a gene consisting of the nucleotide sequence as set forth in SEQ ID NO: 1, SEQ ID NO: 3, SEQ ID NO: 5 or SEQ ID NO: 6;
(c) a protein consisting of an amino acid sequence having 75% or higher identity to the amino acid sequence as set forth in SEQ ID NO: 2 or SEQ ID NO: 4;
(d) a protein encoded by a gene consisting of a nucleotide sequence having 75% or higher identity to the nucleotide sequence as set forth in SEQ ID NO: 1, SEQ ID NO: 3, SEQ ID NO: 5 or SEQ ID NO: 6;
(e) a protein consisting of an amino acid sequence derived from the amino acid sequence as set forth in SEQ ID NO: 2 or SEQ ID NO: 4 by the deletion, substitution and/or addition of one or more amino acids; and
(f) a protein encoded by a nucleotide sequence that hybridizes under stringent conditions to the nucleotide sequence as set forth in SEQ ID NO: 1, SEQ ID NO: 3, SEQ ID NO: 5 or SEQ ID NO: 6.

11. The kit according to claim 9 or 10, wherein the amino acid degrading enzyme is an enzyme that degrades an amino acid selected from arginine, leucine, methionine, phenylalanine, tryptophan and tyrosine.

12. A method for producing a reaction product of pentosidine derived from a specimen, the method comprising the steps of:

degrading the specimen with an amino acid degrading enzyme; and
contacting the specimen after the degradation step with a protein having activity that oxidatively degrades pentosidine, wherein

the amino acid degrading enzyme and the protein having activity that oxidatively degrades pentosidine are different from each other.

Fig.1

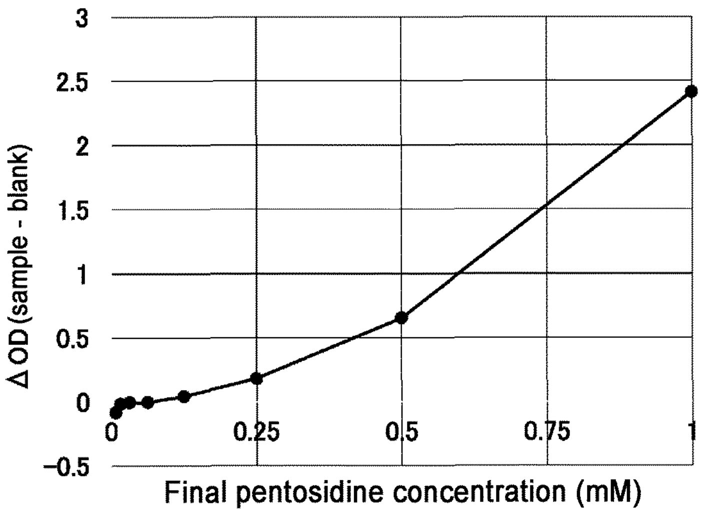


Fig.2

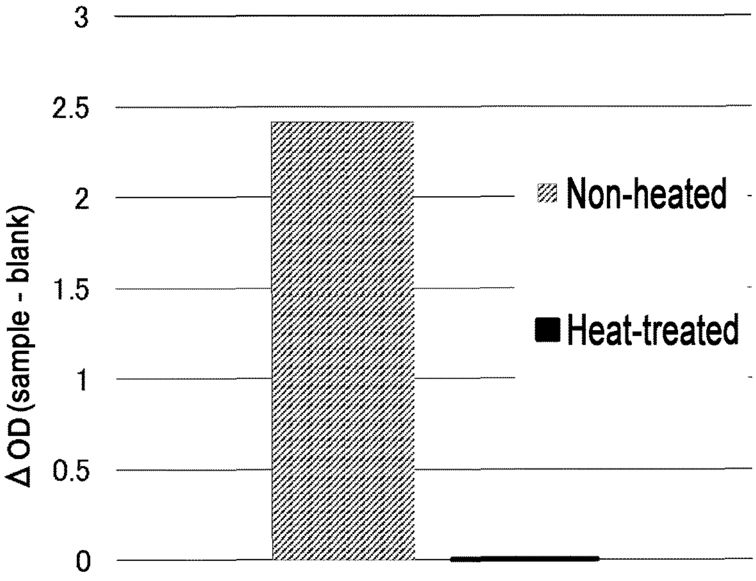


Fig.3

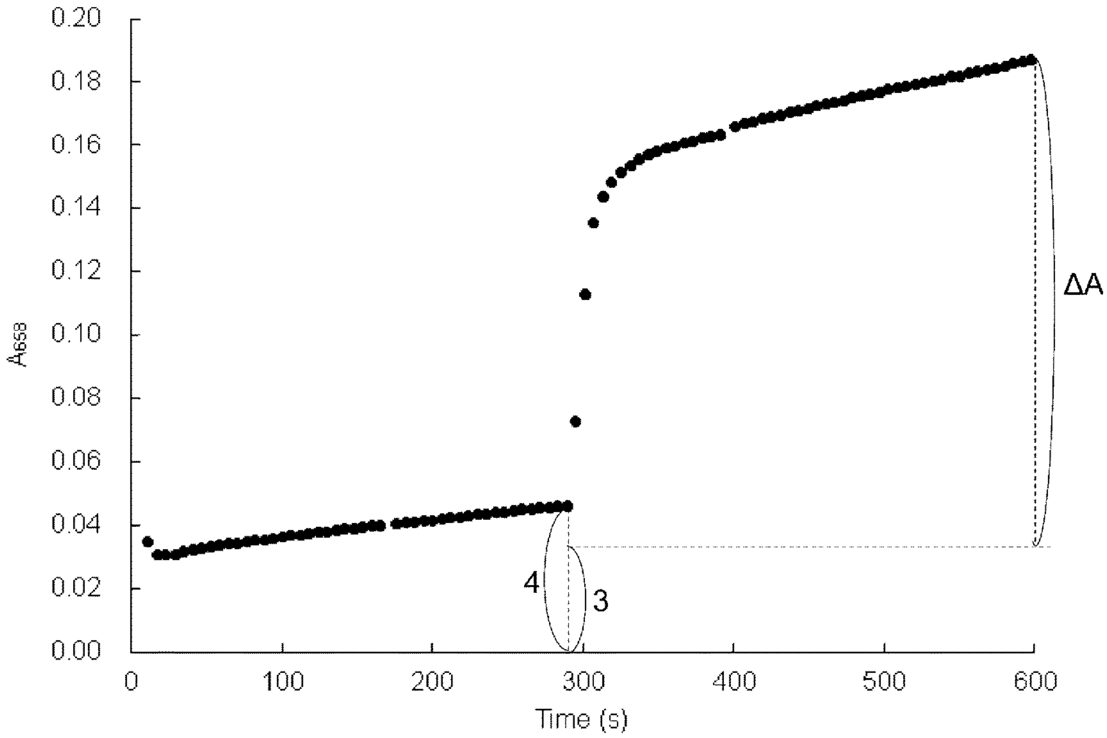
0.20
0.18
0.16
0.14
0.12
0.10
0.08
0.06
0.04
0.02
0.00
$A_{653}$
0
100
200
300
400
500
600
Time (s)
ΔA
4
3

Fig.4

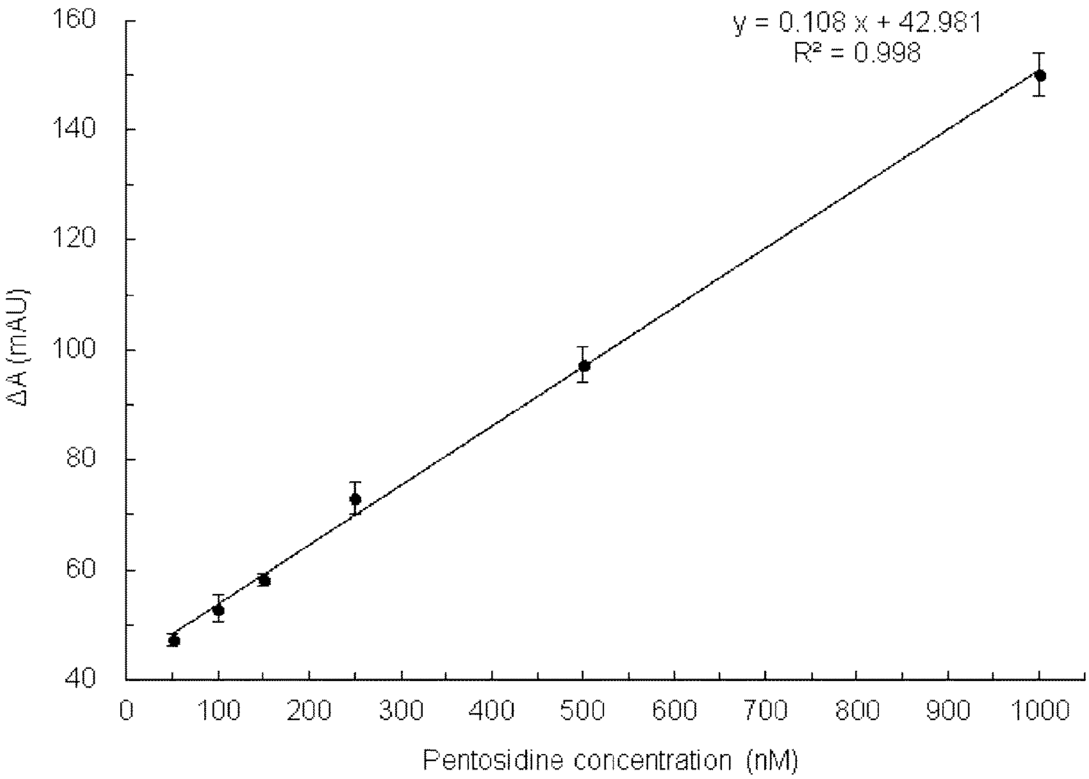
y = 0.108 x + 42.981
R² = 0.998
160
140
120
100
80
60
40
ΔA (mAU)
0
100
200
300
400
500
600
700
800
900
1000
Pentosidine concentration (nM)

Fig.5

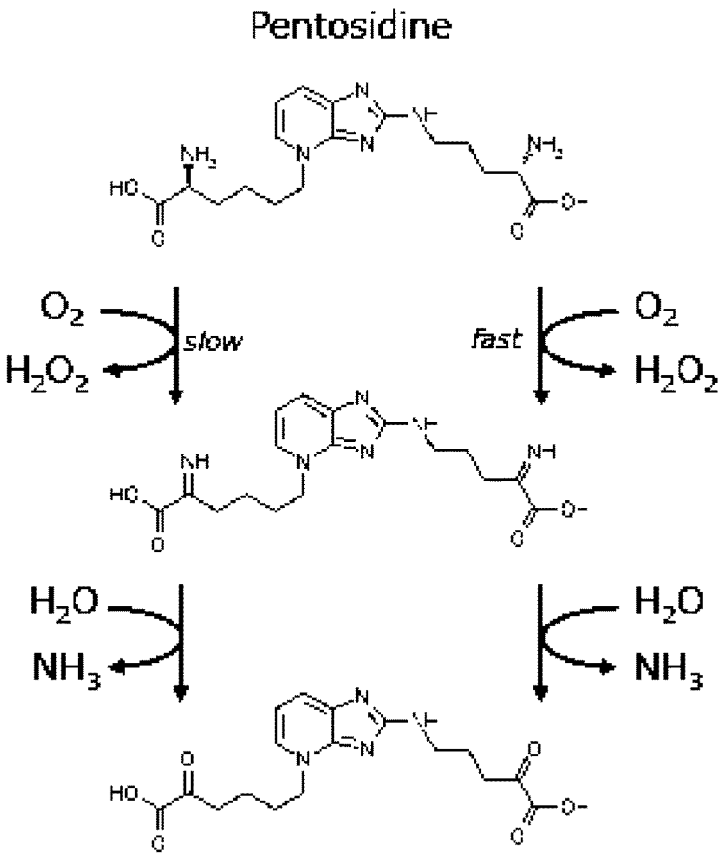


Fig6A

**SEQ ID NO: 1**

**g4462_DNA sequence (containing introns)**

ATGAAGTCTCCCAGTCTGGCCGTGGCCGGCCTTCTTCTTGGCTCGAGTTCCTTAAGCCATGCTACCCAGCTTCGTATTGA
GACGAGGAAATCGCTCAACTCTCGTATCGCCAACGTCCACATTGATGTTGACGCTCCAGTCGCTCACCAAGTTGTCTTCA
CATATGGCCCTTGTGATTCGGAGAGCCAAGAGAACGCTCACCATGTCATTGCTCAAAGCCAAAAGCTAGAGGGCAGGAAA
CCCCATCGATTGATCTGGACTATGCCCAAGGATCTTCACCCGGATGACTGCATCTCTGCGTGGGGTGAATCAGGAGACCT
CCTCGGTCGCAGTGTCCCTCAAAAGGTCGCGCACAAGGAGATGAGGAGACGCAAGAGAGATGATAGCGACTACTCCATCC
CTATGAACAGCTCGAGTGGCATCGACGTTTATGGCCCTTGGTTCGATGGTGTAGCTCTCCTCGAGAAGAGCGACAATCAC
AATGTGGACGTCGAAGCCGCCAAGGCCAAGGAGATTGCTATCGTCGGGGCTGGGATGGCTGGTCTGACCACGTACTTCAT
CCTCAGTGAAGCTGGACTCAGCAACCTGACGATCCTGGAGGCAAGTGGTCGTCTTGGAGGCCGCGTACGCACCGAATATC
TCTCCGGAGGACCCAGGGACCATTCCTATGCCGAGATGGGCCCTATGCGGATCCCATACCAGGCTCGTTTTGGAGACAAG
GCATACAACATCTCGGATCAAGCAATATTCTTCCGACTTGTGGAGAAGGTGAACGAGCGGAACAAGAAGCTTGGAAACAC
CAAGGACCTCATCAACCTGATCCCGTTCATCCAGTCCAGTCCGAATGGGCTCGCTTATTATCAAGGTAACAAGCTGGAGA
ATGGGCTGCCGCCAACGCAGGCAGATGTTGCTGCAGATCCAGCACTGGGGAATGAATCCCCTGAGATCCCTGAATCTGCA
CAAGAATTGGCTGCTCAGCTTCAGAGAGCATTGCCTAACGCTGAATTCCTTGAGCTAATGGCCACAAACTTCTGGCAAGC
CCATGCCGAATTCCTCGGTATGTTGCTTCAGCTCACCACCCCGTATAGCCAGGGCTCTCAAACCCTCATAGAGGATGCCA
CTAAAATGAAATGCGAATCTAGAGAATCAGGGTCCAGCTGGCCTGCCTGGCGATCAGTGGTCTGAGTTTGCTTTCCTGGT
GAACTACCTCAACGCGACCGTCTTCGATGCCAACGCAGTCACTGGTGGTTATGACTGGCACAGCAGCCTTGACAGGGTAC
GTGACGCTGAAGAAGATCTCGTGGTTGCATCATTAATCCCACGGTCTCCCTAGTTGTACTACACTATGCTCTTCGGAGGC
GGGGGCAGTTTCAAGACGATTGATGGAGGCAAGTCAAAATGAGCGTTGCGGGCACCTGGACGAGAACTGACAGAGCTCGC
TCGTAGGCATGAACTTGCTCCCCAACGCTTTCCATCCCCTAGTTGACGATATCACGAAGTTCAATGCAAAAGTAGAGAAG
GTTCAGCTCGATGAGAAGACTTCCCGCCTGAAGCTGCATTGGCGCGCGAACTACACTGATCCGGAGCTCGAGTCGCAGTC
ATTTGACTACGCCATTCTGTCACCTACCATGCCAGCAGTACAGAAGTTGCGCCTTCCAGGTAGGTCCTGCGCAATCTCTC
GTTGCTTTGCGGACCATACCTTTCAATGTCACAGGTCTACCCTTCGCTATGCGCAACGCCGTCGACTCCATGCCTTACGC
CTCTGCGTGCAAAGTCGCCCTCGAATACCGCACTCGGTTCTGGGAAAAGTTCGACAACCCCATCTACGGCTCCTGTTCCA
CCAGCACCGACATTCCCGGTATTGGATCCGTGTGCTACCCATCTTCCAACATCAACGGCAGCGGCCCAGCTTCCCTCCTT
GCGAGCTACGAGATTGGCAGGCCTTACGGCGCAGAGTGGGCTGGCATTCCTGAGGAGCAGCATGTGCAGTATGTGATTGA
CGCAATGATTGATATCCACGGCGAGGTTGCACGACGAGAGTTTACCGGGAAATGCAAGAGAAAGTGTTGGGCTCTGGACG
AGTTCTCAAACGGGGGTTGGGCTTCACCGACCGTGGGTAATCATGAGACGTATCTGCCATCGTTTTTCGAGACCCACAGC
CATGTGAGTGCTCCGACATTTGACCTCTTGTGCCAGGAATGAGGGACTTGTTGACCTTGTTCCAGATGATATTCGTGGGT
GAGCATACCTCCTATACACACGCATGGATCGCCTCTGCGATCGAATCTGCAGTCCGAGGCAGCGTGCAGCTGCTCTTGGG
TATGTTTCGCGTTCTTGCAAATCGTGCAGATGTGCTTCTGACATTACTTTGCCACAGAGCTTGGCCTTATAGACGAGGCG
AAGGATGTCGTAAACACGTGGATGGCACGATGGATCAGTGTTGTAAGTGACCTAAGCAAAGCCGTGGTGTCTGGAGCGTC
AGCGTAG

**(NOTE: Intronic regions are highlighted in yellow)**

**SEQ ID NO: 2**

**g4462_Putative amino acid sequence**

MKSPSLAVAGLLLGSSSLSHATQLRIETRKSLNSRIANVHIDVDAPVAHQVVFTYGPCDSESQENAHHVIAQSQKLEGRK
PHRLIWTMPKDLHPDDCISAWGESGDLLGRSVPQKVAHKEMRRRKRDDSDYSIPMNSSSGIDVYGPWFDGVALLEKSDNH
NVDVEAAKAKEIAIVGAGMAGLTTYFILSEAGLSNLTILEASGRLGGRVRTEYLSGGPRDHSYAEMGPMRIPYQARFGDK
AYNISDQAIFFRLVEKVNERNKKLGNTKDLINLIPFIQSSPNGLAYYQGNKLENGLPPTQADVAADPALGNESPEIPESA
QELAAQLQRALPNAEFLELMATNFWQAHAEFLENQGPAGLPGDQWSEFAFLVNYLNATVFDANAVTGGYDWHSSLDRSSL
VGMNLLPNAFHPLVDDITKFNAKVEKVQLDEKTSRLKLHWRANYTDPELESQSFDYAILSPTMPAVQKLRLPGLPFAMRN
AVDSMPYASACKVALEYRTRFWEKFDNPIYGSCSTSTDIPGIGSVCYPSSNINGSGPASLLASYEIGRPYGAEWAGIPEE
QHVQYVIDAMIDIHGEVARREFTGKCKRKCWALDEFSNGGWASPTVGNHETYLPSFFETHSHMIFVGEHTSYTHAWIASA
IESAVRGSVQLLLELGLIDEAKDVVNTWMARWISVVSDLSKAVVSGASA

Fig.6B

**SEQ ID NO: 3**

**g10122_ DNA sequence (containing introns)**

ATGTTCACTCCCAAAGCTTGGATCCCTCTGTTGGCCTTGAGCCGCGAGGTCTTCTCCAATCCTACTTCTGCATCCCATTC
CATCTCTTTCCACGGTCTCCTTGGCGTCTCTTCGGAGTCAGTCCACAACATCCACCTCACTTATGGCGATGCCTTCCCAC
ATGGAGACTTCCGTGTTGTCTTTGGAGACTGCGGTATGACCAGTGAGGATGAACTTCATCACGAGGTTGCATCTCTCTCC
ACGAAGATGGAGTCTGCTCCTGATCGTTTGGTCTGGCTTGTGCCAAAGGATGTCCGCGAGAATGGTTGTCTTCACGCTTT
CTCGGAGGGCGTCCTCCTCGGTCGGTCTGAGCCTGTCGCTTTCACAGAGCCACTCCGGAAGCGCGAGTCTCTCTCTGAGA
TTGGTGACTCCGATGGCCTCTGGTTCAGCGGAATCCGCTATCTGCAGTCAAGCAACCTGACATCCGTCAAGGCCGCCGAA
GCTAAGGAAAAGAAGATTGGCATCGTTGGCGGTGGCATTTCCGGTCTGATGACGAGTCTGCTCCTGACTTCCGTCGGCAT
GACCAACTGGCACATCATTGAGGCGACTGAGCGTGTCGGAGGCCGCATCCGCACCAAGTATATGAATGGAACTAGTCCCG
ATGACTACCAGTACCAGGAGATGGGGCCCATGAGGTTCCCCGTTGAGGTCAAATACAACGACACCAACGAGACGCTGCCC
ATTCAGGATCACAAGATGGTCTTCCAGCTGGCTGAAGTCTTGAACGAGATGAACGGCAACGACACAGATCTCGCTGTCAA
GTTCATTCCCTGGACCCAGAACAATCCGAACACTCCCGCCAACTCTCGAGGATACCGCCTCCCTGATGGCCGCATCCCTA
CCGCGGCCCAAATCGCCCAGAACCCCAGCATCTTGCCGGCAGCTGCCAACGCGTCAGACCCCCAAGATGCGGAACTTGGC
AAGGAGTCCTTGGAGCGTCTCAGTGACCTTACCCCCGAGCGGATGCGCAACATCTCCGCCAACATCTTCAAGGCGCACCG
AGATGCCATTGACCGCGGCTTGTTTCACTGGTCTGAGGCAGCCTATCTCCGGTACCAGCTGGGCCTTGACGACGATACTG
TGGACTTCGTGGCTGCCTCCGACAACTATCCCATGTTCCCTGACTGGTGGCACGCGGTCTACTTCGCGGCCACGAAGTGG
CTCACGATCGACAAGGGCCTCGATTCGCTGTCCAGGGCTTTTGTACCCCACGTCAAGGACAAGATCACGTACGGTCGCAA
GATTGAAGCCATGCAATGGAATGAGTCAACATCCAAGATCTCACTGTCATGGAGGGAGAGCCCTCTTGCGGCGGCGAAGT
CCGATGAGTACGACTATGCTGTCGTCGCGGTGCCGTTCTCCAAGGTTCGTCTGTGGAAGCGGCCGGCTTACTCCAACCTC
TTGACGAGGGCTATTGGCAAGCTGAACTACGAGCAGGCCTGCAAAGTGAGCACCCCCTCATCCGCCCGAAGAGTGACTAG
AATACTAATCCCTCCAAAAGGTTGCTCTGCTCTACGAGACACGCTTCTGGGAGCACCAGGAGATCCCCATCTTTGGAGGC
TGTGGCTCGGTTGATATCCCGGGTATTGGTGGCGTGTGCTACCCATCATACGAGATCAACTCCACGAGGCCTGGCGTGAT
TCTCTCTTCCTACATTACCGGCACAGAAGCCAGATCCGTGGTAGCCCTGAGCGAGGAAGATCACGTCGCCATGGTGCAGC
GTGCCATGGTCGAAGTCCACGGCCCTATGGCGGATGAGCAGTGGACCGGGATTTACGACCGTCTGTGCTGGGAGGTGGAT
GAGAACGCGGCCGGAGGCTGGGCTTCGCCGACAGTTGGCCAGCAGGAGCTGTTCATCCCGGCGTACCACAAGACGGAGCT
CAACACCATCTTCATCGGAGAGCACACGAGCATCACGCACGGGTGGATCTTCTCGGCCCTGGAATCGTCGGTCAGGGGCA
CGACGCAGTTGCTGCTCGATCTTGGTCTAGTGGATGAGGCGAAGCAGATTGTTGAGACTTGGATGGCGAGGTGGATCACC
GTTTGA

**(NOTE: The gene for 'g10122' is located on the minus strand. The given sequence is the reverse complement which translates the final protein product.)**

**SEQ ID NO: 4**

**g10122_Putative amino acid sequence**

MFTPKAWIPLLALSREVFSNPTSASHSISFHGLLGVSSESVHNIHLTYGDAFPHGDFRVVFGDCGMTSEDELHHEVASLS
TKMESAPDRLVWLVPKDVRENGCLHAFSEGVLLGRSEPVAFTEPLRKRESLSEIGDSDGLWFSGIRYLQSSNLTSVKAAE
AKEKKIGIVGGGISGLMTSLLLTSVGMTNWHIIEATERVGGRIRTKYMNGTSPDDYQYQEMGPMRFPVEVKYNDTNETLP
IQDHKMVFQLAEVLNEMNGNDTDLAVKFIPWTQNNPNTPANSRGYRLPDGRIPTAAQIAQNPSILPAAANASDPQDAELG
KESLERLSDLTPERMRNISANIFKAHRDAIDRGLFHWSEAAYLRYQLGLDDDTVDFVAASDNYPMFPDWWHAVYFAATKW
LTIDKGLDSLSRAFVPHVKDKITYGRKIEAMQWNESTSKISLSWRESPLAAAKSDEYDYAVVAVPFSKVRLWKRPAYSNL
LTRAIGKLNYEQACKVALLYETRFWEHQEIPIFGGCGSVDIPGIGGVCYPSYEINSTRPGVILSSYITGTEARSVVALSE
EDHVAMVQRAMVEVHGPMADEQWTGIYDRLCWEVDENAAGGWASPTVGQQELFIPAYHKTELNTIFIGEHTSITHGWIFS
ALESSVRGTTQLLLDLGLVDEAKQIVETWMARWITV

Fig.6C

**SEQ ID NO: 5**
**Codon-modified g4462 (penox1)**
ATGAAGTCTCCATCTCTGGCTGTGGCTGGTCTCCTGCTTGGATCCTCTAGCCTCTCGCATGCCACCCAGCTGCGCATCGA
AACTCGTAAGTCGCTCAACTCCCGCATCGCTAATGTGCATATCGATGTTGACGCCCCTGTCGCTCACCAGGTCGTGTTCA
CCTACGGTCCCTGCGATTCCGAGTCTCAGGAAAACGCCCATCACGTTATCGCTCAGTCGCAGAAGCTCGAGGGACGGAAG
CCTCATCGCCTGATCTGGACTATGCCTAAGGATCTTCACCCCGATGACTGTATCTCTGCCTGGGGAGAATCTGGTGACTT
GCTCGGCCGGTCCGTGCCACAGAAGGTTGCTCATAAGGAGATGCGCCGTCGGAAGCGCGATGACTCTGACTACAGCATCC
CAATGAACTCGTCCTCTGGAATCGATGTGTATGGCCCGTGGTTCGACGGTGTTGCCCTGCTTGAGAAGTCCGATAACCAC
AATGTTGACGTCGAAGCCGCTAAGGCCAAGGAGATCGCTATCGTCGGAGCCGGCATGGCTGGCCTTACCACTTATTTCAT
CTTGTCGGAAGCCGGTCTTTCCAACTTGACCATCCTCGAAGCTTCTGGACGCCTGGGCGGTCGTGTGCGCACTGAATACC
TTTCTGGAGGCCCCCGTGATCACAGCTATGCCGAGATGGGCCCTATGCGTATCCCCTACCAGGCCCGGTTCGGTGATAAG
GCTTATAATATCAGCGACCAGGCTATCTTCTTCCGCCTCGTCGAGAAGGTTAACGAGCGCAATAAGAAGCTTGGAAACAC
CAAGGACCTGATCAATCTTATCCCTTTCATCCAGAGCTCGCCCAACGGACTGGCCTACTATCAGGGCAACAAGTTGGAAA
ATGGTCTCCCTCCCACTCAAGCTGATGTGGCTGCTGACCCAGCTCTCGGTAATGAGTCGCCAGAAATTCCTGAGTCTGCT
CAAGAATTGGCTGCTCAGCTTCAGCGTGCTTTGCCGAACGCTGAATTCCTTGAGTTGATGGCCACCAATTTCTGGCAGGC
CCATGCTGAATTCTTGGAGAACCAGGGTCCAGCTGGACTCCCGGGCGATCAGTGGTCTGAGTTCGCCTTCCTCGTCAACT
ACCTGAATGCTACCGTTTTCGACGCCAATGCTGTCACTGGTGGATATGATTGGCATTCCTCTCTTGACCGCAGCTCGTTG
GTTGGCATGAACTTGCTCCCAAATGCCTTCCACCCGTTGGTCGATGACATCACCAAGTTCAACGCTAAGGTCGAAAAGGT
GCAGCTCGATGAGAAGACCTCTCGCCTCAAGCTGCACTGGCGTGCCAATTACACTGATCCAGAGTTGGAAAGCCAGTCGT
TCGACTATGCCATCCTCAGCCCTACCATGCCCGCTGTCCAGAAGCTTCGTTTGCCAGGCTTGCCGTTCGCCATGCGGAAC
GCTGTCGACTCTATGCCTTACGCCAGCGCTTGCAAGGTGGCTCTTGAATATCGTACTCGGTTCTGGGAGAAGTTCGATAA
TCCCATCTACGGTTCCTGCTCTACCAGCACTGACATCCCAGGTATCGGATCCGTTTGTTATCCGTCCTCTAACATCAATG
GCTCCGGTCCGGCCTCTCTGCTTGCTAGCTACGAAATTGGACGTCCTTATGGTGCTGAGTGGGCTGGCATCCCCGAGGAA
CAGCATGTTCAGTACGTCATCGATGCCATGATCGACATCCACGGAGAAGTCGCTCGCCGTGAGTTCACCGGCAAGTGCAA
GCGTAAGTGTTGGGCCCTGGATGAGTTCTCTAACGGCGGTTGGGCCTCTCCAACCGTGGGAAATCATGAAACTTACCTTC
CGTCGTTCTTCGAGACTCATTCCCACATGATCTTCGTTGGCGAACATACCTCGTATACTCACGCCTGGATCGCCTCGGCT
ATCGAGTCCGCTGTCCGTGGTTCCGTGCAGTTGCTCCTGGAACTCGGACTGATCGATGAGGCCAAGGACGTTGTCAACAC
CTGGATGGCTCGGTGGATCTCGGTGGTTTCCGATCTGTCTAAGGCCGTCGTGAGCGGTGCCTCGGCTTGA

**SEQ ID NO: 6**
**Codon-modified g10122 (penox2)**
ATGTTCACTCCTAAGGCTTGGATTCCACTCCTGGCTCTGAGCCGCGAGGTCTTCTCGAACCCTACCTCTGCCTCTCATAG
CATCTCGTTCCACGGACTTTTGGGCGTCTCCTCTGAATCTGTGCATAATATCCACCTTACCTACGGTGACGCTTTCCCCC
ATGGAGATTTCCGTGTCGTGTTCGGTGACTGCGGAATGACTTCTGAGGATGAACTGCATCACGAGGTCGCCTCCCTTTCT
ACCAAGATGGAAAGCGCTCCAGACCGCCTCGTGTGGCTGGTTCCGAAGGATGTGCGTGAGAACGGATGTTTGCACGCCTT
CTCTGAAGGCGTTCTCCTGGGTCGGAGCGAGCCTGTCGCTTTCACTGAACCCTTGCGCAAGCGTGAGAGCCTCTCGGAAA
TCGGAGACTCTGATGGCCTGTGGTTCAGCGGCATCCGCTATCTGCAGAGCTCGAATCTTACCTCTGTCAAGGCCGCTGAG
GCCAAGGAAAAGAAGATCGGTATCGTGGGCGGTGGAATCTCCGGACTTATGACCTCTCTTTTGCTCACTAGCGTGGGTAT
GACCAACTGGCATATCATCGAGGCTACCGAACGCGTTGGCGGTCGGATCCGCACTAAGTACATGAATGGAACCAGCCCTG
ATGACTACCAGTATCAGGAGATGGGCCCTATGCGTTTCCCCGTCGAGGTGAAGTATAACGACACCAATGAAACTCTCCCC
ATCCAGGATCACAAGATGGTGTTCCAGTTGGCCGAGGTTCTCAACGAAATGAACGGCAATGACACTGATTTGGCTGTCAA
GTTCATCCCATGGACTCAGAACAATCCTAACACCCCTGCCAATAGCCGCGGCTACCGTCTCCCAGACGGTCGTATCCCGA
CCGCCGCTCAGATCGCTCAGAACCCATCTATTTTGCCTGCTGCTGCTAATGCTTCTGACCCACAGGATGCTGAGCTTGGC
AAGGAGTCGCTTGAACGGTTGTCCGATCTCACTCCGGAACGTATGCGGAACATCTCGGCCAATATCTTCAAGGCCCATCG
TGACGCTATCGATCGGGGTCTGTTCCACTGGTCCGAGGCTGCCTACCTTCGCTATCAGCTCGGACTGGATGACGATACCG
TCGACTTCGTGGCTGCCTCCGATAACTACCCAATGTTCCCGGACTGGTGGCATGCTGTGTATTTCGCTGCCACCAAGTGG
CTGACTATCGACAAGGGCCTGGATTCCCTTTCTCGGGCCTTCGTTCCACACGTCAAGGATAAGATCACTTACGGTCGCAA
GATCGAGGCTATGCAGTGGAATGAAAGCACCTCGAAGATCTCCCTTTCCTGGCGTGAGTCGCCGCTCGCTGCTGCTAAGT
CCGACGAATACGATTATGCCGTTGTCGCTGTTCCATTCTCTAAGGTCCGTCTGTGGAAGCGTCCTGCCTATTCCAACCTG
CTTACTCGCGCTATCGGCAAGCTTAATTACGAGCAGGCCTGCAAGGTGGCTTTGCTCTATGAAACCCGTTTCTGGGAGCA
TCAGGAAATCCCAATCTTCGGAGGCTGCGGATCGGTGGATATCCCTGGCATCGGTGGAGTTTGTTACCCCTCTTATGAGA
TCAACAGCACCCGTCCGGGAGTTATCTTGTCCTCTTATATCACCGGCACTGAAGCCCGGTCGGTGGTTGCTCTCTCCGAG
GAAGACCATGTGGCCATGGTTCAGCGGGCTATGGTTGAGGTCCACGGTCCCATGGCCGATGAACAGTGGACTGGAATCTA
CGACCGCCTCTGTTGGGAAGTCGATGAAAATGCTGCTGGCGGTTGGGCTTCTCCTACTGTTGGACAGCAGGAGTTGTTCA
TCCCCGCTTATCACAAGACCGAGCTCAATACTATCTTCATCGGCGAACATACCTCGATCACTCACGGTTGGATCTTCTCC
GCCCTGGAGAGCTCGGTTCGTGGCACCACTCAGCTGCTTTTGGACCTTGGTTTGGTCGATGAGGCCAAGCAGATCGTGGA
AACTTGGATGGCTCGGTGGATCACCGTCTGA

Fig.6D

**SEQ ID NO: 7**
**Ptef**
TGTGGACCAGACAGGCGCCACTCGGCCGGGCCACAACTGCTTGGGTTTTGACCGGGAGCGGACCAATTAAGGACTCGAAC
GACCGCGGGGTTCAAATGCAAACAAGTACAACACGCAGCAAACGAAGCAGCCCACCACTGCGTTGATGCCCAGTTTGTCT
GTCCGAAATCCACCGGAAAGGTGGAAACATACTATGTAACAATCAGAGGGAAGAAAAATTTTTTATCGACGAGGCAGGAT
AGTGACTGATGGTGGGGTCATGGTCGGGTCTCCGAGCGAAAGAGAACCAAGGAAACAAGATCAACGAGGTTGGTGTACCC
AAAAGGCCGCAGCAACAAGAGTCATCGCCCAAAAGTCAACAGTCTGGAAGAGACTCCGCCGTGCAGATTCTGCGTCGGTC
CCGCACATGCGTGGTGGGGGCATTACCCCTCCATGTCCAATGATAAGGGCGGCGGTCGAGGGCTTAAGCCCGCCCACTAA
TTCGCCTTCTCGCTTGCCCCTCCATATAAGGATTCCCCTCCTTCCCCTCCCACAACTTTTTTCCTCTTTCTCTCTTCGTC
CGCATCAGTACGTATATCTTTCCCCCCTACCTCTTTCTCACTCTTCCTCGATTCATTCCACTCTTCTCCTTACTGACATC
TGTTTTGCTCAGTACCTCTACGCGATCAGCCGTAGTATCTGAGCAAGCTTTTTTACAGAATCTTTCTAGTATCTTACAAA
GAACTACAAAGTTCGCACCACCTTCAAA

**SEQ ID NO: 8**
**Talp**
GTACCAGGAGTACATTGGAGAGTTCTACCATTGTTGCTGGAATACAATGATGATTAGAAACCGAAGAGTGTTATGATTCG
GACGGATATACGCATGGCACGCATACAGCGTGATACATAGGCTGTTTGCTCAAGAATTAGGATTTTATCTGAATCCATGT
ACAGAGTTTACTTATGTTAGTAGTCAATGAAATCTTGGCTTTCTAATTTTGTCCGATCTACAAGGGGTAGTCGATCACAG
AACGAACTAGATGTGCAGGGAACGATGATCACCCGCTCTTAGCAAGACCTCTAGTAGTTTTCGACCATAGCTTTAACGCG
AATCATGACCCTACTATTTTCTAGATTGCAGACCAAGTCACATGACAATGTCCTCTTTGAAGTAGGATCAGTAGCTGATT
AGATTCCGGGAAATGAATTAGGGCTGGCGTTCCAACTACTGGGGAGTGCCGATGTTGCTGTATGAAAGATAGTAAGATTA
CTAGTGCACAGCTGTAGTAATTATTTACTCTAGATTATATATTCCAAATAATAAGTAATCTAAGATAGTAGACAGTCCTA
TGATATAGCTCCGGGTTCGAAGTCGGCAAAAGATATGCAATCACCTGTCGGGATGATATATGTATATCTGAAATACCGAC
ATCAACCATCCAGTCGGATCAGCTAAACGAAGTATCACTTCTTTCGCCACTGCCAATCACTACTTCTATTAAAGTTCATG
TTACAGTATAAGCCACAAGACTTATCTCCAGAACTAACTTGTGCATAGGAGCTCTGCCGATAGCCGGGTGGTTGGATCGG

**SEQ ID NO: 9**
**pyrG3**
TAAGTACTCATTTATACAATAGTTGCAGAACCCCGCGCTACCCCTCCATTGCCAACATGTCTTCCAAGTCGCAATTGACC
TACAGCGCACGCGCTAGCAAGCACCCCAATGCGCTCGTGAAGAAGCTCTTCGAGGTTGCCGAGGCCAAGAAAACCAATGT
CACCGTTTCCGCCGACGTGACAACCACCAAAGAGCTGCTGGATTTGGCTGACCGTATGCGCACCGGGGATGCCACTTACA
TATGATCTAGTAATGGTTAATGGTGGAATATATAACAGGACTCGGTCCGTACATTGCCGTGATCAAAACTCACATCGATA
TCCTCTCCGATTTCAGCGAAGAGACCATCATCGGTCTGAAGGCCCTTGCAGAGAAGCACAATTTCCTCATCTTCGAAGAT
CGCAAGTTCATCGATATCGGAAACACAGTCCAAAAGCAGTACCATGGCGGCACTCTGCGCATCTCTGAGTGGGCCCACAT
CATCAACTGCAGTATTCTGCCCGGTGAGGGTATCGTCGAGGCTCTGGCCCAGACTGCTTCGGCCGAGGACTTCCCCTATG
GCTCTGAGAGGGGCCTTTTGATCCTTGCGGAGATGACATCCAAGGGATCTTTGGCTACCGGTCAATATACTACTTCTTCT
GTTGACTATGCCCGGAAGTATAAGAAGTTTGTGATGGGATTCGTCTCGACGCGTCACCTGGGCGAGGTTCAGTCTGAAGT
TAGCTCGCCTTCGGAGGAGGAGGATTTCGTCGTCTTCACGACAGGTGTCAACCTCTCCTCGAAGGGAGACAAACTGGGAC
AGCAATACCAGACTCCTGAGTCTGCTGTTGGACGCGGTGCCGACTTTATCATTGCTGGTCGTGGAATTTATGCTGCTCCT
GATCCCGTGGAGGCAGCGAAGCGGTACCAGAAAGAGGGATGGGATGCATACCAGAAGCGTGTTGGTGCGCAATAAGTAGT
GGTGAATACGTGCTCTTTTTATGGCAGTATATCGCAAGTATGATGCGATTCATAAATTCAGCAGTCGAATTCTACGAGAG
AACGATGCTAAGAGATACCCTCTCTATATGAATAATATGCCTGCCTCGAGATATGGACATATTCAAGATCAGAGTTAAGG
GTCATGTTTCAAAATCACACCAATCTCCAACATAGACGAGAATTTTTACCGGATTGTCTGAAGGTGCAGCTGGAGATTGG
TCTATTTTCTAAGAGTGGGGTATCACTAATGTACAGTCGGTCACTATCGTACAAACAATCACAATTATATACAAGATTTC
CCATCACCCCTTACTCTAACATGGCACTTTTATCCATCGAGTCCGAGCCTAGCCACCATTTGGTGCTTTCGTAGAGACCA
AAGTATAACCCTGATCCGACAGCGGCCATAAACGTGTTGATAGCACACCCTCGGAATAGTCCTCTCGGGCCATCTGTTCG
TATAATCTCCCGTACGGTATTGATCATCCTTTTCTTCTGAGGTGCGG

**SEQ ID NO: 10**
**Ptef-Rv**
AGATGGAGACTTCATtttgaaggtggtgcgaactttgtag

**SEQ ID NO: 11**
**Talp-Fw**
GGTGCCTCGGCTTGAgtaccaggagtacattggagagt

Fig.6E

**SEQ ID NO: 12**
AHDRNPLEECFRETDYEEFLEIAKNGLTATSNPKRVVIVGAGMAGLSAAYVLAGAGHQVTVLEASERVGGRVRTYRKKDW
YANLGPMRLPTKHRIVREYIKKFDLKLNEFSQENENAWYFIKNIRKRVREVKNNPGLLEYPVKPSEEGKSAAQLYVESLR
KVVEELRSTNCKYILDKYDTYSTKEYLLKEGNLSPGAVDMIGDLLNEDSGYYVSFIESLKHDDIFGYEKRFDEIVGGMDQ
LPTSMYEAIKEKVQVHFNARVIEIQQNDREATVTYQTSANEMSSVTADYVIVCTTSRAARRIKFEPPLPPKKAHALRSVH
YRSGTKIFLTCTKKFWEDDGIHGGKSTTDLPSRFIYYPNHNFTSGVGVIIAYGIGDDANFFQALDFKDCADIVINDLSLI
HELPKEDIQTFCHPSMIQRWSLDKYAMGGITTFTPYQFQHFSEALTAPFKRIYFAGEYTAQFHGWIDSTIKSGLTAARDV
NRASENPSGIHLSNDNEF

**SEQ ID NO: 13**
AHDRNPLEECFRETDYEEFLEIAKNGLTATSNPKRVVIVGAGMAGLSAAYVLAGAGHQVTVLEASERVGGRVRTYRKKDW
YANLGPMRLPTKHRIVREYIKKFDLKLNEFSQENENAWYFIKNIRKRVREVKNNPGLLEYPVKPSEEGKSAAQLYVESLR
KVVKELKRTNCKYILDKYDTYSTKEYLLKEGNLSPGAVDMIGDLLNEDSGYYVSFIESLKHDDIFGYEKRFDEIVGGMDQ
LPTSMYEAIKEKVQVHFNARVIEIQQNDREATVTYQTSANEMSSVTADYVIVCTTSRAARRIKFEPPLPPKKAHALRSVH
YRSGTKIFLTCKKKFWEDDGIRGGKSTTDLPSRFIYYPNHNFTSGVGVIIAYGIGDDANFFQALDFKDCADIVINDLSLI
HQLPKEDIQTFCRPSMIQRWSLDKYAMGGITTFTPYQFQHFSEALTAPFKRIYFAGEYTAQFHGWIDSTIKSGLTAARDV
NRASENPSGIHLSNDNEF

**SEQ ID NO: 14**
MDNVDFAESVRTRWARRLIREKVAKELNILTERLGEVPGIPPPREGRFLGGGYSHDNLPSDPLYSSIKPALLKEAPRAEE
ELPPRKVCIVGAGVSGLYIAMILDDLKIPNLTYDIFESSSRTGGRLYTHHFTDAKHDYYDIGAMRYPDIPSMKRTFNLFK
RTGMPLIKYYLDGENTPQLYNNHFFAKGVVDPYMVSVANGGTVPDDVVDSVGEKLQQAFGYYKEKLAEDFDKGFDELMLV
DDMTTREYLKRGGPKGEAPKYDFFAIQWMETQNTGTNLFDQAFSESVIDSFDFDNPTKPEWYCIEGGTSLLVDAMKETLV
HKVQNNKRVEAISIDLDAPDDGNMSVKIGGKDYSGYSTVFNTTALGCLDRMDLRGLNLHPTQADAIRCLHYDNSTKVALK
FSYPWWIKDCGITCGGAASTDLPLRTCVYPSYNLGDTGEAVLLASYTWSQDATRIGSLVKDAPPQPPKEDELVELILQNL
ARLHAEHMTYEKIKEAYTGVYHAYCWANDPNVGGAFALFGPGQFSNLYPYLMRPAAGGKFHIVGEASSVHHAWIIGSLES
AYTAVYQFLYKYKMWDYLRLLLERWQYGLQELETGKHGTAHLQFILGSLPKEYQVKIV

Fig.7

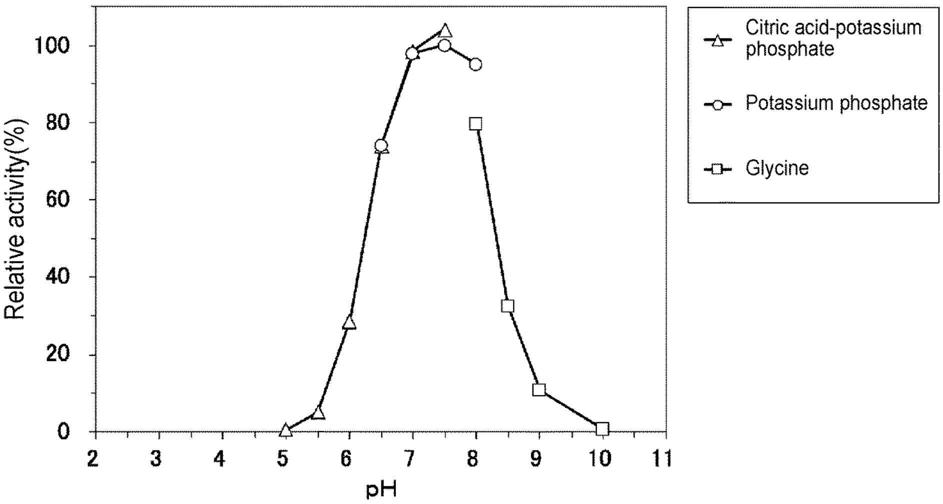


Fig.8

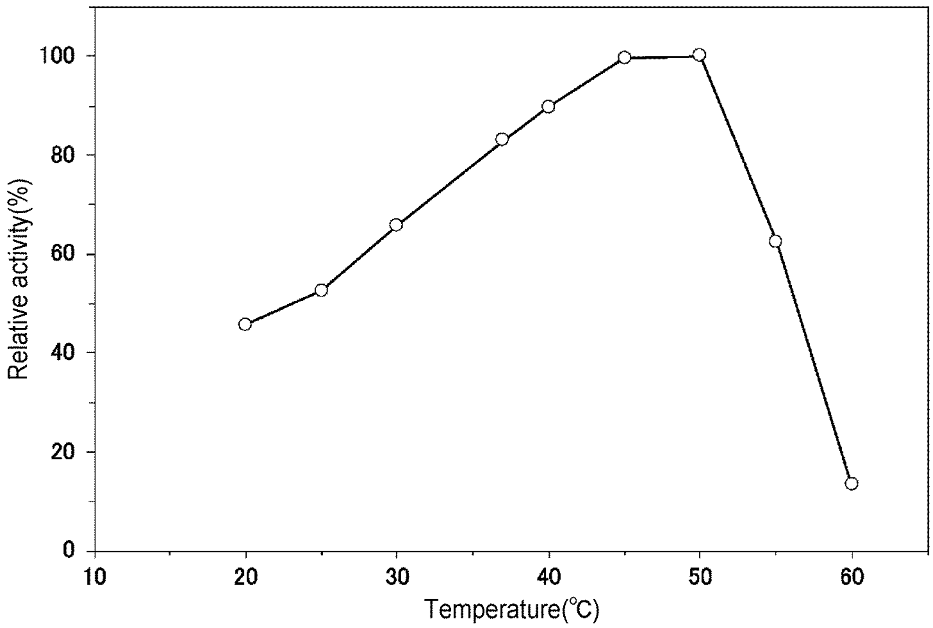
100
80
60
40
20
0
Relative activity(%)
10
20
30
40
50
60
Temperature(℃)

Fig.9

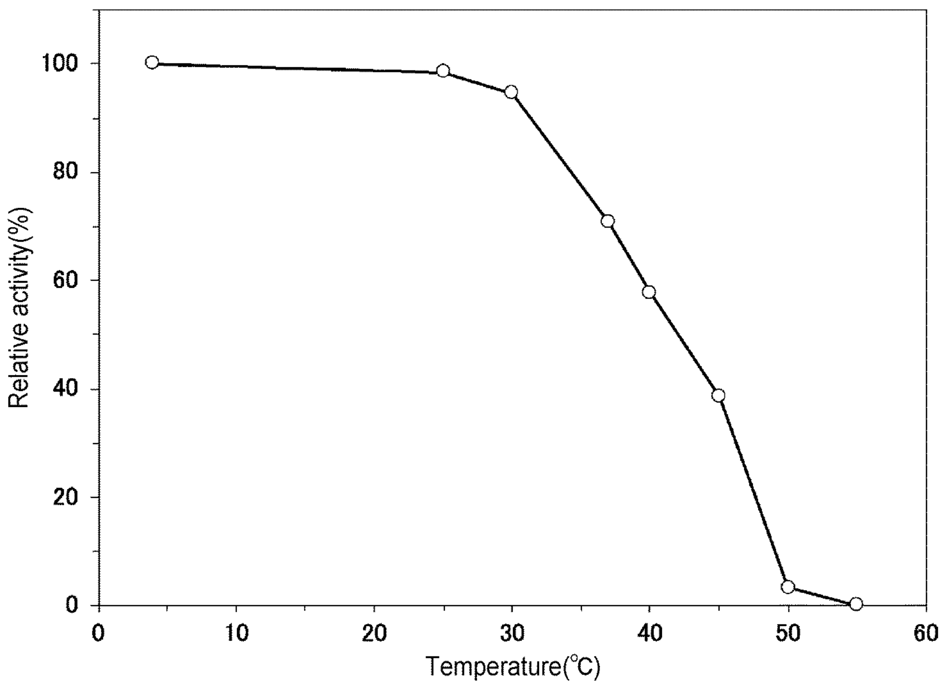
100
80
60
40
20
0
Relative activity(%)
0
10
20
30
40
50
60
Temperature(℃)

Fig.10

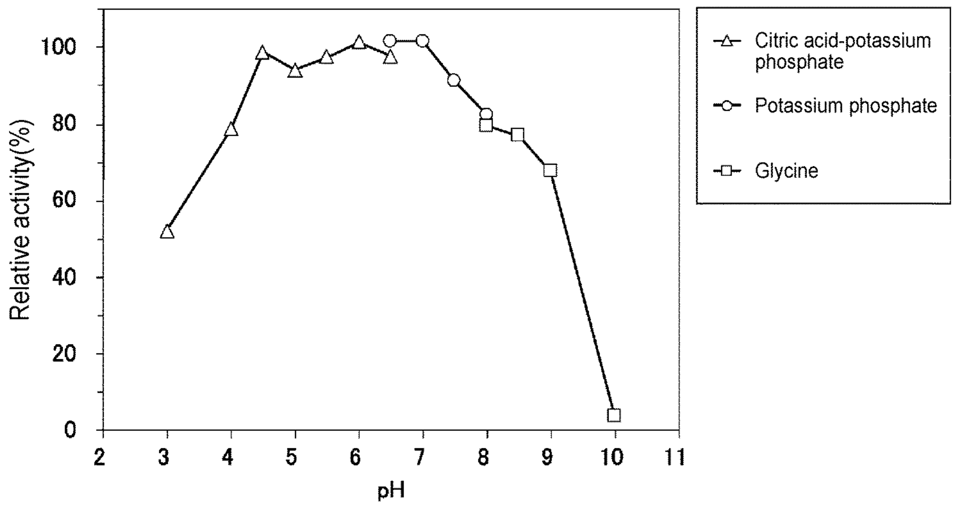
Relative activity(%)
100
80
60
40
20
0
2
3
4
5
6
7
8
9
10
11
pH
Citric acid-potassium phosphate
Potassium phosphate
Glycine

Fig.11

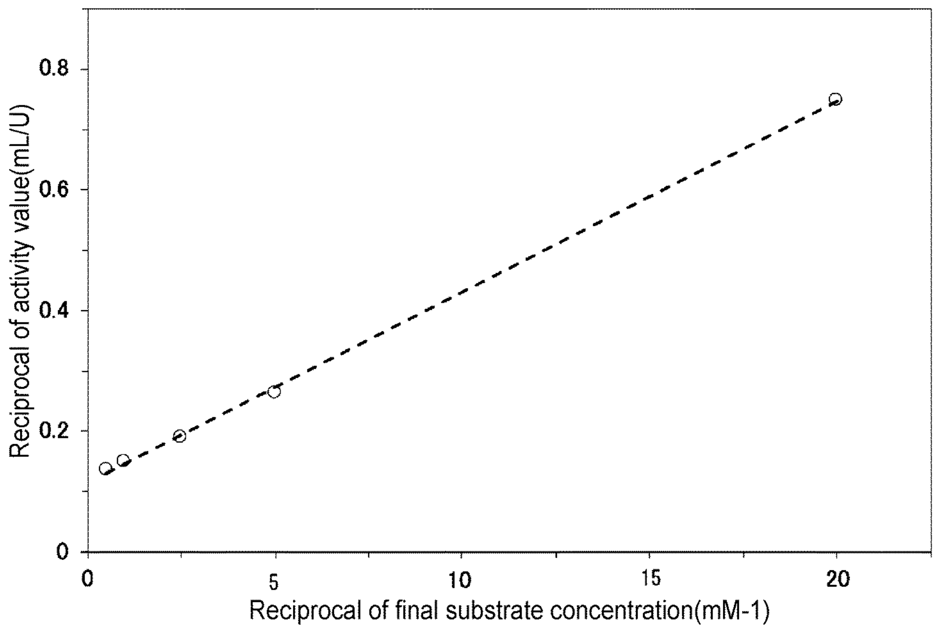
Reciprocal of activity value(mL/U)
0.8
0.6
0.4
0.2
0
0
5
10
15
20
Reciprocal of final substrate concentration(mM-1)

Fig.12

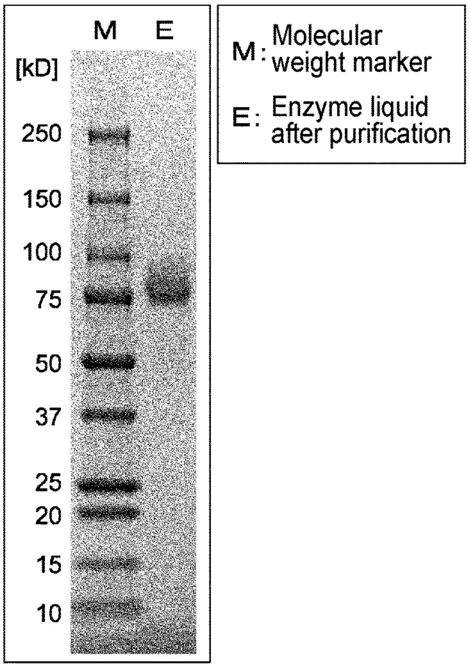
M
E
[kD]
250
150
100
75
50
37
25
20
15
10
M: Molecular weight marker
E: Enzyme liquid after purification

Fig.13

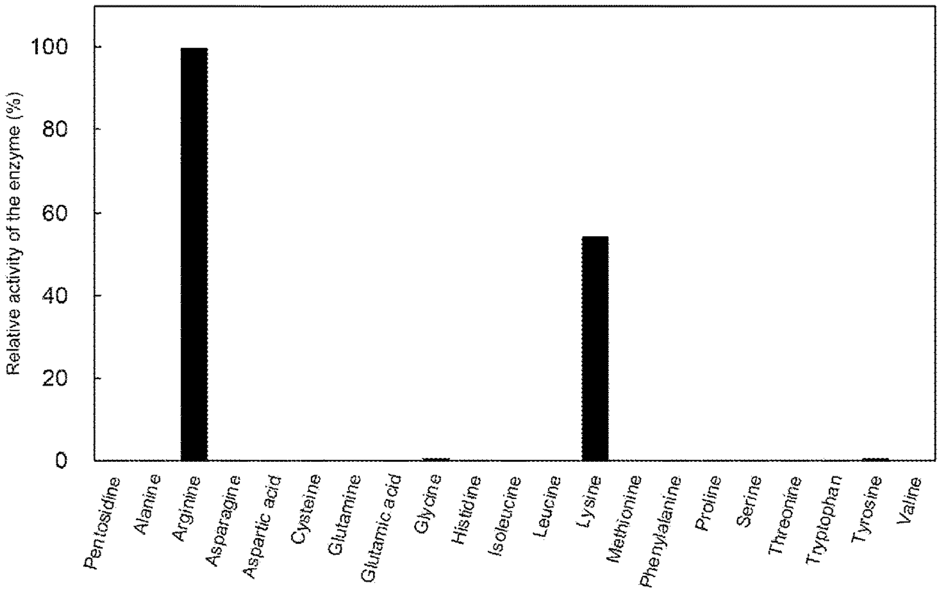
Relative activity of the enzyme (%)
100
80
60
40
20
0
Pentosidine
Alanine
Arginine
Asparagine
Aspartic acid
Cysteine
Glutamine
Glutamic acid
Glycine
Histidine
Isoleucine
Leucine
Lysine
Methionine
Phenylalanine
Proline
Serine
Threonine
Tryptophan
Tyrosine
Valine

Fig.14

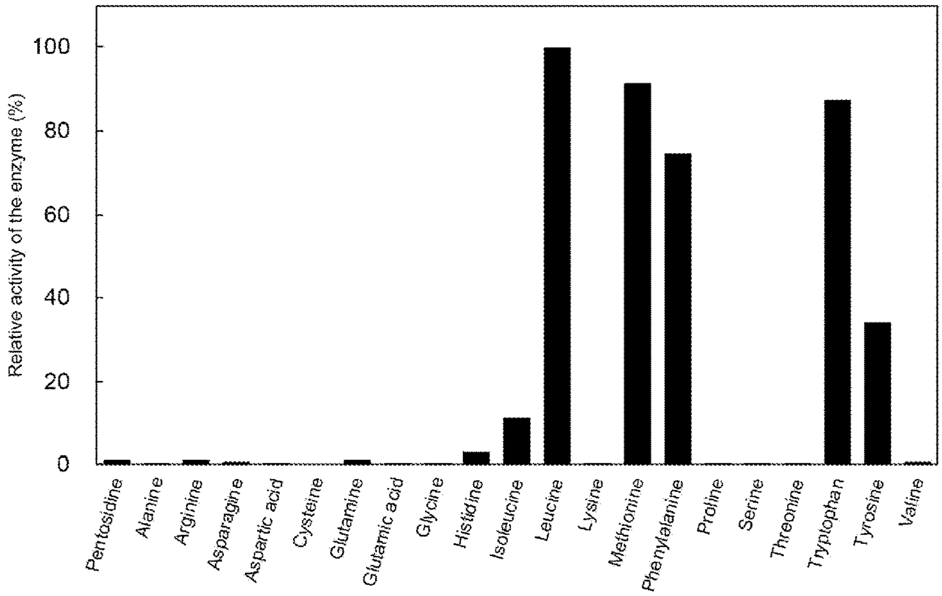
Relative activity of the enzyme (%)
100
80
60
40
20
0
Pentosidine
Alanine
Arginine
Asparagine
Aspartic acid
Cysteine
Glutamine
Glutamic acid
Glycine
Histidine
Isoleucine
Leucine
Lysine
Methionine
Phenylalanine
Proline
Serine
Threonine
Tryptophan
Tyrosine
Valine

Fig.15

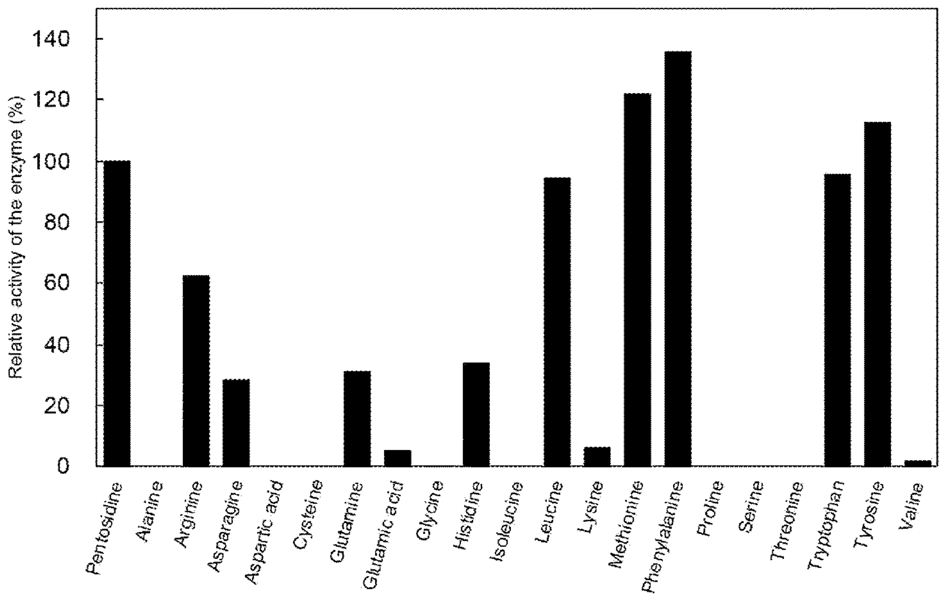
Relative activity of the enzyme (%)
140
120
100
80
60
40
20
0
Pentosidine
Alanine
Arginine
Asparagine
Aspartic acid
Cysteine
Glutamine
Glutamic acid
Glycine
Histidine
Isoleucine
Leucine
Lysine
Methionine
Phenylalanine
Proline
Serine
Threonine
Tryptophan
Tyrosine
Valine

## INTERNATIONAL SEARCH REPORT

International application No. PCT/JP2020/031553

**A. CLASSIFICATION OF SUBJECT MATTER**

C12N 15/53(2006.01)i; C12Q 1/26(2006.01)i; C12N 9/04(2006.01)i
FI: C12N15/53; C12N9/04 Z; C12Q1/26 ZNA

According to International Patent Classification (IPC) or to both national classification and IPC

**B. FIELDS SEARCHED**

Minimum documentation searched (classification system followed by classification symbols)
C12N15/53; C12Q1/26; C12N9/04

Documentation searched other than minimum documentation to the extent that such documents are included in the fields searched

| | |
|---|---|
| Published examined utility model applications of Japan | 1922-1996 |
| Published unexamined utility model applications of Japan | 1971-2020 |
| Registered utility model specifications of Japan | 1996-2020 |
| Published registered utility model applications of Japan | 1994-2020 |

Electronic data base consulted during the international search (name of data base and, where practicable, search terms used)
JSTPlus/JMEDPlus/JST7580 (JDreamIII); CAplus/MEDLINE/EMBASE/BIOSIS/WPIDS (STN); GenBank/EMBL/DDBJ/GeneSeq; UniProt/GeneSeq

**C. DOCUMENTS CONSIDERED TO BE RELEVANT**

| Category* | Citation of document, with indication, where appropriate, of the relevant passages | Relevant to claim No. |
|---|---|---|
| P, A | WO 2019/168062 A1 (KIKKOMAN CORPORATION) 06 September 2019 (2019-09-06) claims, examples | 1-12 |
| A | JP 2001-21559 A (FUSHIMI PHARMACEUTICAL CO., LTD.) 26 January 2001 (2001-01-26) claims, paragraphs [0002]-[0006], examples | 1-12 |
| A | RAIBEKAS, Andrei A. and MASSEY, Vincent, "Primary Structure of the Snake Venom L-Amino Acid Oxidase Shows High Homology with the Mouse B Cell Interleukin 4-Induced Fig1 Protein", BIOCHEMICAL AND BIOPHYSICAL RESEARCH COMMUNICATIONS, 1998, vol. 248, pp. 476-478, in particular, abstract, fig. 2 | 1-12 |
| A | AMANO, Marie, et al., "Recombinant expression, molecular characterization and crystal structure of antitumor enzyme, L-lysine α-oxidase from Trichodermaviride", The Journal of Biochemistry, 2015, vol. 157, no. 6, pp. 549-559, in particular, abstract, fig. 2 | 1-12 |
| A | JP 2013-212109 A (TOKAI UNIVERSITY, et al.) 17 October 2013 (2013-10-17) examples, paragraph [0055] | 1-12 |

☐ Further documents are listed in the continuation of Box C. ☒ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

Date of the actual completion of the international search
12 October 2020 (12.10.2020)

Date of mailing of the international search report
27 October 2020 (27.10.2020)

Name and mailing address of the ISA/
Japan Patent Office
3-4-3, Kasumigaseki, Chiyoda-ku,
Tokyo 100-8915, Japan

Authorized officer

Telephone No.

Form PCT/ISA/210 (second sheet) (January 2015)

**INTERNATIONAL SEARCH REPORT**
Information on patent family members

International application no.
PCT/JP2020/031553

| Patent Documents referred in the Report | Publication Date | Patent Family | Publication Date |
|---|---|---|---|
| WO 2019/168062 A1 | 06 Sep. 2019 | (Family: none) | |
| JP 2001-21559 A | 26 Jan. 2001 | (Family: none) | |
| JP 2013-212109 A | 17 Oct. 2013 | US 2011/0028470 A1 examples, paragraph [0152]<br>EP 2189537 A1 | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- JP 5738346 B
- JP 2007222055 A
- JP 2011239681 A
- JP 2018068292 A

### Non-patent literature cited in the description


- Molecular Cloning: A laboratory Manual. Cold Spring Harbor Laboratory, 1989
- Current Protocols in Molecular Biology. John Wiley & Sons, 1987
- **KARLIN ; ALTSCHUL.** *Proc. Natl. Acad. Sci. USA,* 1990, vol. 87, 2264-2268
- *Proc. Natl. Acad. Sci. USA,* 1993, vol. 90, 5873-5877
- **ALTSCHUL et al.** *J. Mol. Biol.,* 1990, vol. 215, 403-410
- *Nucleic Acids Res.,* 1997, vol. 25, 3389-3402
- *Science,* 1985, vol. 227, 1435-1441
- *Methods in Enzymology,* 1983, vol. 101, 20-78
- *Mol. Gen. Genet.,* 1989, vol. 218, 99-104
- **OZEKI et al.** *Biosci. Biotechnol. Biochem.,* 1995, vol. 59, 1133
- **ARAI et al.** *Psychiatria et Neurologia Japonica,* 2012, vol. 114 (2), 101-107
- **ARAI, M. et al.** *Arch Gen Psychiat,* 2010, vol. 67, 589-597
- **YANG et al.** *J Exp Biol,* 2005, vol. 208 (18), 3609-22
- Plasma Amino Acid Reference Values. *ayo Clinic Laboratories Neurology Catalog,* 10 October 2018, https://neurology.testcatalog.org/show/AAQP